(19) Europäisches Patentamt
European Patent Office
Office européen des brevets

(11) **EP 4 335 861 A1**

(12) **EUROPEAN PATENT APPLICATION**
published in accordance with Art. 153(4) EPC


(43) Date of publication:
**13.03.2024 Bulletin 2024/11**

(21) Application number: **22799017.3**

(22) Date of filing: **14.04.2022**

(51) International Patent Classification (IPC):
***C07K 7/08*** *(2006.01)* ***A01N 37/46*** *(2006.01)*

(52) Cooperative Patent Classification (CPC):
**A01N 37/46; C07K 7/08;** Y02A 50/30

(86) International application number:
**PCT/KR2022/005387**

(87) International publication number:
**WO 2022/234972 (10.11.2022 Gazette 2022/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.05.2021 KR 20210058480**
**11.04.2022 KR 20220044371**



(71) Applicant: **Gwangju Institute of Science and Technology**
**Gwangju 61005 (KR)**

(72) Inventors:
- **SEO, Ji Won**
  **Gwangju 61005 (KR)**
- **SHIN, Dong Min**
  **Gwangju 61005 (KR)**
- **CHOI, Ji Eun**
  **Gwangju 61005 (KR)**
- **KIM, Min Sang**
  **Gwangju 61005 (KR)**

(74) Representative: **Gulde & Partner**
**Patent- und Rechtsanwaltskanzlei mbB**
**Wallstraße 58/59**
**10179 Berlin (DE)**


(54) **ANTIMICROBIAL PEPTOID HAVING IMPROVED SELECTIVITY AND ANTIMICROBIAL COMPOSITION COMPRISING SAME**


(57) The present invention relates to an antimicrobial peptoid having improved selectivity and an antimicrobial composition comprising same. The antimicrobial peptoid according to the present invention exhibits excellent antimicrobial activity against bacteria and low cytotoxicity to animal cells by comprising an indole ring, introducing a cationic monomer thereinto, or comprising *N*His as a submonomer containing imidazole on a side branch. Therefore, the antimicrobial peptoid of the present invention has improved selectivity to bacteria and thus can be advantageously used for an antimicrobial composition.


FIG. 5

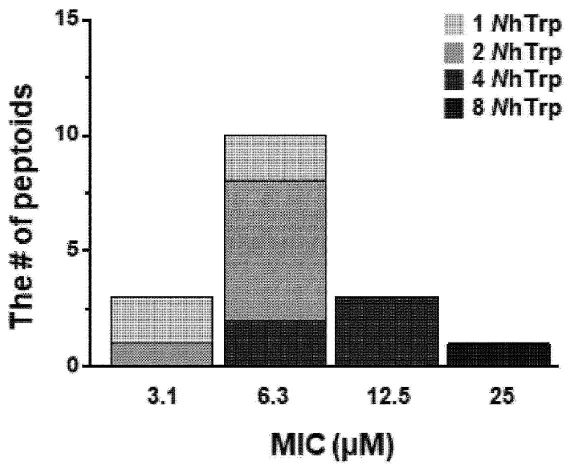


EP 4 335 861 A1

## Description

[Technical Field]

The present invention relates to an antimicrobial peptoid, and more particularly, to an antimicrobial peptoid which has high antimicrobial activity and reduced toxicity to animal cells.

[Background Art]

In 1928, Alexander Fleming (the Nobel Prize in Physiology or Medicine, 1945) discovered penicillin in the green mold (*Penicillium rubens*), and since penicillin has been used as a miracle antibiotic from 1942, the mortality caused by pathogenic bacterial infections in humans has dramatically reduced. Afterward, various types of antibiotics such as sulfa drugs, tetracyclines, quinolones, aminoglycosides, vancomycin, rifamycins, daptomycin, and carbapenems have been developed to treat various bacterial infections. However, because of the abuse of antibiotics in hospitals or livestock farms and the neglect of the development of new antibiotic drugs by pharmaceutical companies over the past 30 years, the number of patients infected with antibiotic-resistant bacteria that cannot be treated with any existing antibiotics (pan-resistant bacteria) has increased.

Multi drug-resistant bacteria (MDR), also called super bacteria, refer to bacteria that have acquired resistance to multiple antibiotics simultaneously through natural selection or mutation. The major pathogens that cause a problem with antibiotic resistance are so-called 'ESKAPE' strains, including *Enterococcus*, *Staphylococcus aureus, Klebsiella pneumoniae, Acinetobacter baumannii, Pseudomonas aeruginosa,* and *Enterobacter.* The increase in multidrug-resistant bacteria having resistance to various antibiotics has become a global problem that threatens human health in the 21$^{st}$ century, and no new treatment is being developed, which is a serious problem. Therefore, there is an urgent need for development of antibiotics to treat bacteria with multi-drug resistance and differentiated antimicrobial mechanisms.

Antimicrobial peptides (AMPs) are, as part of the innate defense system in most organisms, small-sized peptides consisting of 10 to 50 amino acids, and natural materials acting as primary defense materials when an organism is infected by a pathogen. The antimicrobial peptides have excellent antimicrobial activity even against bacteria resistant to existing antibiotics, and are attracting attention as a next-generation therapeutic agent that can address the problem of antibiotic resistance.

Antimicrobial peptoids structurally mimicking antimicrobial peptides are promising antimicrobial agents compensating for the limitation of antimicrobial peptides. Peptoids are artificially-synthesized peptidomimetics, and have a backbone in which tertiary amides in which a substituent is attached to an amine are repeated, that is, a structure in the form of an oligomer of N-alkylated glycine. Such peptoids have very excellent stability in the body due to high resistance to a protease unlike a peptide, and are able to be simply synthesized by solid-phase synthesis. Accordingly, antimicrobial peptoids may more stably exhibit antimicrobial activity than natural antimicrobial peptides as *in vivo* hydrolysis by an enzyme is delayed. However, antimicrobial peptoids have a disadvantage of showing cytotoxicity against mammalian cells such as NIH 3T3 mouse fibroblasts. Accordingly, there is a need for research on antimicrobial peptoids with reduced toxicity to animal cells and increased selectivity.

[Disclosure]

[Technical Problem]

The present invention is directed to providing an The term "peptoid" used herein,, which exhibits antimicrobial activity against bacteria and low cytotoxicity, and an antimicrobial composition including the same.

The present invention is also directed to providing a method of preparing an antimicrobial composition, which includes the above-described antimicrobial peptoid.

The technical objects of the present invention are not limited to the technical objects mentioned above, and other technical objects not mentioned herein will be clearly understood by those of ordinary skill in the art from the following description.

[Technical Solution]

To achieve the technical objects described above, the present invention may provide an antimicrobial peptoid represented by Formula 1 below.

[Formula 1] $H\text{-}X_1\text{-}Y_1\text{-}Z_1\text{-}X_2\text{-}Y_2\text{-}Z_2\text{-}X_3\text{-}Y_3\text{-}Z_3\text{-}X_4\text{-}Y_4\text{-}Z_4\text{-}NH_2$

In Formula 1, $X_1$ to $Z_1$, $X_2$ to $Z_2$, $X_3$ to $Z_3$, and $X_4$ to $Z_4$ are each independently *N*spe, *N*Lys, *N*hTrp, *N*His, *N*pm, or *N*4hb.

Specifically, the antimicrobial peptoid may be any one or more selected from Formulas 2 to 5 below.

[Formula 2] H-*N*Lys-*N*spe-*N*spe-*N*Lys-*N*spe-*N*spe-*N*Lys-*N*Lys-*N*spe-*N*Lys-*N*hTrp-*N*hTrp-$NH_2$;

[Formula 3] H-*N*Lys-*N*hTrp-*N*hTrp-*N*Lys-*N*spe-*N*spe-*N*Lys-*N*spe-*N*spe-*N*Lys-*N*Lys-*N*spe-$NH_2$;

[Formula 4] H-*N*Lys-*N*hTrp-*N*hTrp-*N*His-*N*spe-*N*spe-*N*Lys-*N*spe-*N*spe-*N*Lys-*N*Lys-*N*spe-$NH_2$; and

[Formula 5] H-*N*Lys-*N*spe-*N*spe-*N*Lys-*N*spe-*N*spe-*N*Lys-*N*His-*N*spe-*N*Lys-*N*hTrp-*N*hTrp-$NH_2$.

In addition, the present invention for achieving the above-described technical objects may provide an antimicrobial composition which contains any one or more of the above-described antimicrobial peptoids as an active ingredient.

The antimicrobial peptoid may exhibit antimicrobial activity against gram-positive or gram-negative bacteria.

The gram-positive bacteria may be bacteria of *Staphylococcus sp., Bacillus sp., Streptococcus sp.,* or *Enterococcus sp.*.

The gram-positive bacteria may be *Staphylococcus aureus, methicillin-resistant Staphylococcus aureus* (MRSA), *Quinolone-resistant Staphylococcus aureus* (QRSA), *vancomycin-resistant enterococcus* (VRE), *vancomycin intermediate-resistant Staphylococcus aureus* (VISA), *Bacillus subtilis, Bacillus cereus, Streptococcus pneumoniae, Enterococcus faecalis, Enterococcus faecium,* or *Staphylococcus epidermidis.*

The gram-negative bacteria may be bacteria of *Salmonella sp., Acinetobacter sp., Escherichia sp., Pseudomonas sp., Enterobacter sp.,* or *Klebsiella sp.*.

The gram-negative bacteria may be *Salmonella typhimurium, Acinetobacter calcoaceticus, Acinetobacter baumannii, Escherichia coli, Pseudomonas aeruginosa, Klebsiella pneumoniae,* or *Klebsiella aerogenes.*

The present invention for achieving the above-described technical objects may include preparing deprotected polymer resin beads (S 100); preparing a bromoacetylate peptoid by adding the deprotected polymer resin beads, bromoacetic acid, diisopropylcarbodiimide (DIC), and an organic solvent and performing a bromoacetylation reaction (S200); adding a submonomer to the bromoacetylate peptoid and performing an amine substitution reaction (S300); obtaining an antimicrobial peptoid having the desired sequence by repeating S200 to S300 (S400); and separating a polymer resin from the antimicrobial peptoid using a cleavage solution (S500), wherein the submonomer is *N*spe, *N*Lys, *N*hTrp, *N*His, *N*pm, or *N*4hb.

After S500, a process of performing a counter ion substitution reaction by removing trifluoroacetate (TFA) ions included in the composition using a carbonate ion exchange resin and adding an acidic solution (S600) may be further included.

The acidic solution may be acetic acid or hydrochloric acid.

[Advantageous Effects]

According to the present invention, as an antimicrobial peptoid of the present invention includes an indole ring, introduces a cationic monomer, or includes NHis as a submonomer including imidazole at a side chain, excellent antimicrobial activity against bacteria and low cytotoxicity in animal cells are exhibited. Accordingly, due to improved selectivity, the antimicrobial peptoid of the present invention can be effectively used as an antimicrobial composition.

[Description of Drawings]

FIG. 1 is a schematic diagram illustrating the process of solid-phase peptoid synthesis (SPPS) for preparing a peptoid of the present invention.

FIGS. 2 to 4 are graphs showing circular dichroism spectra in the range of 190 nm to 260 nm of an antimicrobial peptoid according to one embodiment of the present invention.

FIGS. 5 and 6 are graphs analyzing the antimicrobial activity against *E. coli* according to the substitution number and position of *N*hTrp of an antimicrobial peptoid according to one embodiment of the present invention.

FIGS. 7 and 8 are graphs analyzing the relationships between the HPLC elution and the antimicrobial activity and the red blood cell toxicity of an antimicrobial peptoid according to one embodiment of the present invention.

FIGS. 9 to 74 are the HPLC analysis results for antimicrobial Peptoids 1 to 66 according to one embodiment of the present invention.

FIG. 75 is a graph showing the $^{19}$F-NMR quantitative analysis for Peptoid 1 to measure a remaining fluorine amount resulting from TFA ions_after a counter ion substitution reaction.

FIGS. 76 to 83 are the HPLC analysis results after a counter ion substitution reaction for Peptoids 1, 10, 11, 14, 15, 29, 32 and 37 of the present invention using (a) HCl and (b) AcOH.

FIG. 84 is a graph showing the rate of change in the $LC_{50}$ value of a counter ion-substituted peptoid and showing the results for MRC-5 cells according to one embodiment of the present invention.

FIG. 85 is a graph showing the rate of change in the LC50 value of a counter ion-substituted peptoid and showing the results for HaCaT cells according to one embodiment of the present invention.

FIGS. 86 to 100 are the HPLC analysis results for antimicrobial Peptoids 67 to 81 according to one embodiment of the present invention.

[Modes of the Invention]

While the invention is susceptible to various modifications and alternative forms, specific embodiments thereof are shown by way of example in the drawings and will herein be described in detail. It should be understood, however, that there is no intent to limit the invention to the particular forms disclosed, but on the contrary, the invention is to cover all modifications, equivalents, and alternatives falling within the spirit and scope of the invention. To explain each drawing, like numerals denote like elements.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, the present invention will be described in detail.

Antimicrobial peptoid

The term "peptoid" used herein, as a structural isomer of a peptide, may refer to a peptide derivative which has a similar monomer sequence but the structure of oligo-*N*-substituted glycine in which a side chain is attached to nitrogen, not carbon, of the backbone. Since the peptoid has a substituent (R) attached to the nitrogen (N) atom of an amide group, it has a configuration without a chiral center and amide hydrogen, and has the sequence specificity of natural biopolymers, such as a protein, a nucleic acid, etc. Monomers with basic information serve as components to form a precisely defined sequence, having a structure with a specific function through folding or assembly. Since such a peptoid is unnatural unlike a peptide, and is a non-natural biopolymer or biomimetic polymer, it can overcome the disadvantage of peptides being easily degraded *in vivo* because of high resistance to proteases and very excellent *in vivo* stability. In addition, since the helical structure of a peptide is maintained by hydrogen bonds, structural transformation occurs at 40 °C or more. However, a peptoid may have thermal and chemical stability in a relatively wide range, such as not only maintaining structural stability even at 70 °C or more, but also maintaining a stable helical structure even with a solution containing a salt or having a low pH.

However, the type of monomer constituting a peptoid and the peptoid sequence may play a pivotal role in determining the peptoid function. As described above, a peptoid has a structure in which an amino acid side chain is attached to the nitrogen of an amide bond. Accordingly, the peptoid backbone has no asymmetry of monomer molecules, and the peptoid amide bond facilitates the conversion between cis-trans amide isomers. In addition, since there is no hydrogen bond formed by the backbone amide-NH, which serves to strongly fix a peptide secondary structure, a peptoid may have a relatively flexible structure than a peptide. Due to the structural characteristics of such a peptoid backbone, it is difficult to form secondary structures such as helical and/or sheet structures by hydrogen bonds. Accordingly, research is being conducted to induce the sequence and structure of a peptoid having various characteristics using monomers with various functional groups and chemical structures, and since a protein and a peptide have different functions depending on the degree of a helical content, it is important to design the peptoid sequence-structure relationship for mimicking with further detailed characterization.

The term "antimicrobial peptoid" used herein may refer to a peptoid having antimicrobial activity, and may also be referred to as a peptoid.

In addition, the antimicrobial peptoid may be easily synthesized using solid-phase synthesis like a peptide. A peptoid having a desired sequence may be obtained by repeating a two-step reaction including a bromoacetylation

reaction and an amine substitution reaction several times using polymer beads with an amine group as a starting material.

The antimicrobial peptoid may include at least one or more submonomers selected from *N*spe, *N*Lys, *N*hTrp, *N*His, *N*pm, and *N*4hb.

The antimicrobial peptoid of the present invention may be formed by mimicking Omiganan, which is a derivative of the natural antimicrobial peptide, indolicidin. The Omiganan is a cationic antimicrobial peptide consisting of 12 amino acids, and may include an amino acid having an indole ring, that is, tryptophan, and/or an analogue thereof. As a peptoid mimic of the Omiganan, the antimicrobial peptoid of the present invention may have an excellent interaction with a membrane interface, and thus may have the characteristic of strong cell membrane disrupting activity.

The antimicrobial peptoid of the present invention may be represented by Formula 1 below.

[Formula 1] $H\text{-}X_1\text{-}Y_1\text{-}Z_1\text{-}X_2\text{-}Y_2\text{-}Z_2\text{-}X_3\text{-}Y_3\text{-}Z_3\text{-}X_4\text{-}Y_4\text{-}Z_4\text{-}NH_2$

In Formula 1, $X_1$ to $Z_1$, $X_2$ to $Z_2$, $X_3$ to $Z_3$, and $X_4$ to $Z_4$ may each be independently *N*spe, *N*Lys, *N*hTrp, *N*His, *N*pm, or *N*4hb.

Specifically, the antimicrobial peptoid may be any one or more selected from Peptoids 1 to 81 shown in Tables 1 to 4 below.

[Table 1]

| Classification | Structure |
|---|---|
| Peptoid 1 | H-*N*Lys-*N*spe-*N*spe-*N*Lys-*N*spe-*N*spe-*N*Lys-*N*spe-*N*spe-*N*Lys-*N*spe-*N*spe-$NH_2$ |
| Peptoid 2 | H-*N*Lys-*N*pm-*N*pm-*N*Lys-*N*pm-*N*pm-*N*Lys-*N*pm-*N*pm-*N*Lys-*N*pm-*N*pm-$NH_2$ |
| Peptoid 3 | H-*N*hTrp-*N*Lys-*N*spe-*N*spe-*N*Lys-*N*spe-*N*spe-*N*Lys-*N*spe-*N*spe-*N*Lys-*N*spe-*N*spe-$NH_2$ |
| Peptoid 4 | H-*N*Lys-*N*spe-*N*spe-*N*Lys-*N*spe-*N*spe-*N*Lys-*N*spe-*N*spe-*N*Lys-*N*spe-*N*spe-*N*hTrp-$NH_2$ |
| Peptoid 5 | H-*N*hTrp-*N*Lys-*N*spe-*N*spe-*N*Lys-*N*spe-*N*spe-*N*Lys-*N*spe-*N*spe-*N*Lys-*N*spe-*N*spe-*N*hTrp-$NH_2$ |
| Peptoid 6 | H-*N*hTrp-*N*hTrp-*N*Lys-*N*spe-*N*spe-*N*Lys-*N*spe-*N*spe-*N*Lys-*N*spe-*N*spe-*N*Lys-*N*spe-*N*spe-$NH_2$ |
| Peptoid 7 | H-*N*Lys-*N*spe-*N*spe-*N*Lys-*N*spe-*N*spe-*N*Lys-*N*spe-*N*spe-*N*Lys-*N*spe-*N*hTrp-$NH_2$ |
| Peptoid 8 | H-*N*Lys-*N*spe-*N*spe-*N*Lys-*N*spe-*N*spe-*N*Lys-*N*spe-*N*hTrp-*N*Lys-*N*spe-*N*spe-$NH_2$ |
| Peptoid 9 | H-*N*Lys-*N*spe-*N*spe-*N*Lys-*N*spe-*N*hTrp-*N*Lys-*N*spe-*N*spe-*N*Lys-*N*spe-*N*spe-$NH_2$ |
| Peptoid 10 | H-*N*Lys-*N*spe-*N*hTrp-*N*Lys-*N*spe-*N*spe-*N*Lys-*N*spe-*N*spe-*N*Lys-*N*spe-*N*spe-$NH_2$ |
| Peptoid 11 | H-*N*Lys-*N*spe-*N*spe-*N*Lys-*N*spe-*N*spe-*N*Lys-*N*spe-*N*spe-*N*Lys-*N*hTrp-*N*hTrp-$NH_2$ |
| Peptoid 12 | H-*N*Lys-*N*spe-*N*spe-*N*Lys-*N*spe-*N*spe-*N*Lys-*N*hTrp-*N*hTrp-*N*Lys-*N*spe-*N*spe-$NH_2$ |
| Peptoid 13 | H-*N*Lys-*N*spe-*N*spe-*N*Lys-*N*hTrp-*N*hTrp-*N*Lys-*N*spe-*N*spe-*N*Lys-*N*spe-*N*spe-$NH_2$ |
| Peptoid 14 | H-*N*Lys-*N*hTrp-*N*hTrp-*N*Lys-*N*spe-*N*spe-*N*Lys-*N*spe-*N*spe-*N*Lys-*N*spe-*N*spe-$NH_2$ |
| Peptoid 15 | H-*N*Lys-*N*spe-*N*spe-*N*Lys-*N*spe-*N*hTrp-*N*Lys-*N*hTrp-*N*spe-*N*Lys-*N*spe-*N*spe-$NH_2$ |
| Peptoid 16 | H-*N*Lys-*N*spe-*N*hTrp-*N*Lys-*N*spe-*N*spe-*N*Lys-*N*spe-*N*spe-*N*Lys-*N*spe-*N*hTrp-$NH_2$ |
| Peptoid 17 | H-*N*Lys-*N*hTrp-*N*spe-*N*Lys-*N*spe-*N*spe-*N*Lys-*N*spe-*N*spe-*N*Lys-*N*spe-*N*hTrp-$NH_2$ |
| Peptoid 18 | H-*N*lys-*N*hTrp-*N*hTrp-*N*Lys-*N*spe-*N*spe-*N*Lys-*N*spe-*N*spe-*N*Lys-*N*hTrp-*N*hTrp-$NH_2$ |
| Peptoid 19 | H-*N*Lys-*N*spe-*N*spe-*N*Lys-*N*spe-*N*spe-*N*Lys-*N*hTrp-*N*hTrp-*N*Lys-*N*hTrp-*N*hTrp-$NH_2$ |
| Peptoid 20 | H-*N*Lys-*N*spe-*N*spe-*N*Lys-*N*hTrp-*N*hTrp-*N*Lys-*N*hTrp-*N*hTrp-*N*Lys-*N*spe-*N*spe-$NH_2$ |
| Peptoid 21 | H-*N*Lys-*N*hTrp-*N*hTrp-*N*Lys-*N*hTrp-*N*hTrp-*N*Lys-*N*spe-*N*spe-*N*Lys-*N*spe-*N*spe-$NH_2$ |
| Peptoid 22 | H-*N*Lys-*N*spe-*N*hTrp-*N*Lys-*N*spe-*N*hTrp-*N*Lys-*N*spe-*N*hTrp-*N*Lys-*N*spe-*N*hTrp-$NH_2$ |
| Peptoid 23 | H-*N*lys-*N*hTrp-*N*hTrp-*N*lys-*N*hTrp-*N*hTrp-*N*lys-*N*hTrp-*N*hTrp-*N*lys-*N*hTrp-*N*hTrp-$NH_2$ |

[Table 2]

| Classification | Structure |
|---|---|
| Peptoid 24 | H-*N*Lys-*N*spe-*N*spe-*N*Lys-*N*spe-*N*hTrp-*N*Lys-*N*spe-*N*spe-*N*Lys-*N*Lys-*N*spe-NH$_2$ |
| Peptoid 25 | H-*N*Lys-*N*spe-*N*spe-*N*Lys-*N*spe-*N*hTrp-*N*Lys-*N*Lys-*N*spe-*N*Lys-*N*spe-*N*spe-NH$_2$ |
| Peptoid 26 | H-*N*Lys-*N*spe-*N*spe-*N*Lys-*N*Lys-*N*hTrp-*N*Lys-*N*spe-*N*spe-*N*Lys-*N*spe-*N*spe-NH$_2$ |
| Peptoid 27 | H-*N*Lys-*N*Lys-*N*spe-*N*Lys-*N*spe-*N*hTrp-*N*Lys-*N*spe-*N*spe-*N*Lys-*N*spe-*N*spe-NH$_2$ |
| Peptoid 28 | H-*N*Lys-*N*spe-*N*spe-*N*Lys-*N*spe-*N*spe-*N*Lys-*N*spe-*N*Lys-*N*Lys-*N*hTrp-*N*hTrp-NH$_2$ |
| Peptoid 29 | H-*N*Lys-*N*spe-*N*spe-*N*Lys-*N*spe-*N*spe-*N*Lys-*N*Lys-*N*spe-*N*Lys-*N*hTrp-*N*hTrp-NH$_2$ |
| Peptoid 30 | H-*N*Lys-*N*spe-*N*spe-*N*Lys-*N*Lys-*N*spe-*N*Lys-*N*spe-*N*spe-*N*Lys-*N*hTrp-*N*hTrp-NH$_2$ |
| Peptoid 31 | H-*N*Lys-*N*Lys-*N*spe-*N*Lys-*N*spe-*N*spe-*N*Lys-*N*spe-*N*spe-*N*Lys-*N*hTrp-*N*hTrp-NH$_2$ |
| Peptoid 32 | H-*N*Lys-*N*hTrp-*N*hTrp-*N*Lys-*N*spe-*N*spe-*N*Lys-*N*spe-*N*spe-*N*Lys-*N*Lys-*N*spe-NH$_2$ |
| Peptoid 33 | H-*N*Lys-*N*hTrp-*N*hTrp-*N*Lys-*N*spe-*N*spe-*N*Lys-*N*Lys-*N*spe-*N*Lys-*N*spe-*N*spe-NH$_2$ |
| Peptoid 34 | H-*N*Lys-*N*hTrp-*N*hTrp-*N*Lys-*N*Lys-*N*spe-*N*Lys-*N*spe-*N*spe-*N*Lys-*N*spe-*N*spe-*N*H$_2$ |
| Peptoid 35 | H-*N*hTrp-*N*hTrp-*N*Lys-*N*spe-*N*spe-*N*Lys-*N*spe-*N*spe-*N*Lys-*N*Lys-*N*spe-*N*Lys-*N*spe-*N*spe-NH$_2$ |
| Peptoid 36 | H-*N*hTrp-*N*hTrp-*N*Lys-*N*Lys-*N*spe-*N*Lys-*N*spe-*N*spe-*N*Lys-*N*spe-*N*spe-*N*Lys-*N*spe-*N*spe-*N*H$_2$ |
| Peptoid 37 | H-*N*hTrp-*N*hTrp-*N*Lys-*N*spe-*N*spe-*N*Lys-*N*spe-*N*spe-*N*Lys-*N*spe-*N*spe-*N*Lys-*N*H$_2$ |
| Peptoid 38 | H-*N*hTrp-*N*hTrp-*N*Lys-*N*spe-*N*Lys-*N*Lys-*N*spe-*N*spe-*N*Lys-*N*spe-*N*spe-*N*Lys-*N*H$_2$ |
| Peptoid 39 | H-*N*Lys-*N*spe-*N*spe-*N*Lys-*N*spe-*N*spe-*N*Lys-*N*Lys-*N*pm-*N*Lys-*N*hTrp-*N*hTrp-*N*H$_2$ |
| Peptoid 40 | H-*N*Lys-*N*spe-*N*spe-*N*Lys-*N*pm-*N*pm-*N*Lys-*N*Lys-*N*spe-*N*Lys-*N*hTrp-*N*hTrp-*N*H$_2$ |
| Peptoid 41 | H-*N*Lys-*N*pm-*N*pm-*N*Lys-*N*spe-*N*spe-*N*Lys-*N*Lys-*N*spe-*N*Lys-*N*hTrp-*N*hTrp-*N*H$_2$ |
| Peptoid 42 | H-*N*pm-*N*Lys-*N*spe-*N*spe-*N*Lys-*N*spe-*N*spe-*N*Lys-*N*spe-*N*spe-*N*Lys-*N*spe-*N*spe-*N*pm-*N*H$_2$ |
| Peptoid 43 | H-*N*pm-*N*pm-*N*Lys-*N*spe-*N*spe-*N*Lys-*N*spe-*N*spe-*N*Lys-*N*spe-*N*spe-*N*Lys-*N*spe-*N*spe-*N*H$_2$ |
| Peptoid 44 | H-*N*Lys-*N*spe-*N*spe-*N*Lys-*N*spe-*N*spe-*N*Lys-*N*spe-*N*spe-*N*Lys-*N*pm-*N*pm-*N*H$_2$ |
| Peptoid 45 | H-*N*Lys-*N*spe-*N*spe-*N*Lys-*N*spe-*N*spe-*N*Lys-*N*Lys-*N*spe-*N*Lys-*N*pm-*N*pm-*N*H$_2$ |
| Peptoid 46 | H-*N*Lys-*N*pm-*N*pm-*N*Lys-*N*spe-*N*spe-*N*Lys-*N*spe-*N*spe-*N*Lys-*N*spe-*N*spe-*N*H$_2$ |
| Peptoid 47 | H-*N*Lys-*N*pm-*N*pm-*N*Lys-*N*spe-*N*spe-*N*Lys-*N*spe-*N*spe-*N*Lys-*N*Lys-*N*spe-*N*H$_2$ |
| Peptoid 48 | H-*N*spe-*N*Lys-*N*spe-*N*spe-*N*Lys-*N*spe-*N*spe-*N*Lys-*N*spe-*N*spe-*N*Lys-*N*spe-*N*H$_2$ |

[Table 3]

| Classification | Structure |
|---|---|
| Peptoid 49 | H-*N*Lys-*N*spe-*N*spe-*N*Lys-*N*spe-*N*spe-*N*Lys-*N*spe-*N*spe-*N*Lys-*N*Lys-*N*H$_2$ |
| Peptoid 50 | H-*N*Lys-*N*spe-*N*spe-*N*Lys-*N*spe-*N*spe-*N*Lys-*N*Lys-*N*spe-*N*Lys-*N*spe-*N*H$_2$ |
| Peptoid 51 | H-*N*Lys-*N*spe-*N*spe-*N*Lys-*N*Lys-*N*spe-*N*Lys-*N*spe-*N*spe-*N*Lys-*N*spe-*N*H$_2$ |
| Peptoid 52 | H-*N*Lys-*N*Lys-*N*spe-*N*Lys-*N*spe-*N*spe-*N*Lys-*N*spe-*N*spe-*N*Lys-*N*spe-*N*H$_2$ |
| Peptoid 53 | H-*N*spe-*N*Lys-*N*spe-*N*spe-*N*Lys-*N*spe-*N*spe-*N*Lys-*N*spe-*N*spe-*N*Lys-*N*Lys-*N*H$_2$ |
| Peptoid 54 | H-*N*spe-*N*Lys-*N*spe-*N*spe-*N*Lys-*N*spe-*N*spe-*N*Lys-*N*Lys-*N*spe-*N*Lys-*N*spe-*N*H$_2$ |

(continued)

| Classification | Structure |
|---|---|
| Peptoid 55 | H-*N*spe-*N*Lys-*N*spe-*N*spe-*N*Lys-*N*Lys-*N*spe-*N*Lys-*N*spe-*N*spe-*N*Lys-*N*spe-*N*$H_2$ |
| Peptoid 56 | H-*N*spe-*N*Lys-*N*Lys-*N*spe-*N*Lys-*N*spe-*N*spe-*N*Lys-*N*spe-*N*spe-*N*Lys-*N*spe-*N*$H_2$ |
| Peptoid 57 | H-*N*hTrp-*N*Lys-*N*spe-*N*spe-*N*Lys-*N*spe-*N*spe-*N*Lys-*N*spe-*N*spe-*N*Lys-*N*Lys-*N*$H_2$ |
| Peptoid 58 | H-*N*hTrp-*N*Lys-*N*spe-*N*spe-*N*Lys-*N*spe-*N*spe-*N*Lys-*N*Lys-*N*spe-*N*Lys-*N*spe-*N*$H_2$ |
| Peptoid 59 | H-*N*hTrp-*N*Lys-*N*spe-*N*spe-*N*Lys-*N*Lys-*N*spe-*N*Lys-*N*spe-*N*spe-*N*Lys-*N*spe-*N*$H_2$ |
| Peptoid 60 | H-*N*hTrp-*N*Lys-*N*Lys-*N*spe-*N*Lys-*N*spe-*N*spe-*N*Lys-*N*spe-*N*spe-*N*Lys-*N*spe-*N*$H_2$ |
| Peptoid 61 | H-*N*hTrp-*N*spe-*N*Lys-*N*spe-*N*spe-*N*Lys-*N*spe-*N*spe-*N*Lys-*N*spe-*N*spe-*N*Lys-*N*Lys-*N*$H_2$ |
| Peptoid 62 | H-*N*hTrp-*N*spe-*N*Lys-*N*spe-*N*spe-*N*Lys-*N*spe-*N*spe-*N*Lys-*N*Lys-*N*spe-*N*Lys-*N*spe-*N*$H_2$ |
| Peptoid 63 | H-*N*hTrp-*N*spe-*N*Lys-*N*spe-*N*spe-*N*Lys-*N*Lys-*N*spe-*N*Lys-*N*spe-*N*spe-*N*Lys-*N*spe-*N*$H_2$ |
| Peptoid 64 | H-*N*hTrp-*N*spe-*N*Lys-*N*Lys-*N*spe-*N*Lys-*N*spe-*N*spe-*N*Lys-*N*spe-*N*spe-*N*Lys-*N*spe-*N*$H_2$ |
| Peptoid 65 | H-*N*4hb-*N*hTrp-*N*hTrp-*N*4hb-*N*hTrp-*N*hTrp-*N*4hb-*N*hTrp-*N*hTrp-*N*4hb-*N*hTrp-*N*hTrp-*N*$H_2$ |
| Peptoid 66 | H-*N*4hb-*N*spe-*N*spe-*N*4hb-*N*spe-*N*spe-*N*4hb-*N*4hb-*N*spe-*N*4hb-*N*hTrp-*N*hTrp-*N*$H_2$ |

[Table 4]

| Classification | Structure |
|---|---|
| Peptoid 67 | H-*N*Lys-*N*hTrp-*N*hTrp-*N*Lys-*N*His-*N*spe-*N*Lys-*N*spe-*N*spe-*N*Lys-*N*Lys-*N*spe-$NH_2$ |
| Peptoid 68 | H-*N*Lys-*N*hTrp-*N*hTrp-*N*Lys-*N*spe-*N*His-*N*Lys-*N*spe-*N*spe-*N*Lys-*N*Lys-*N*spe-$NH_2$ |
| Peptoid 69 | H-*N*Lys-*N*hTrp-*N*hTrp-*N*Lys-*N*spe-*N*spe-*N*Lys-*N*His-*N*spe-*N*Lys-*N*Lys-*N*spe-$NH_2$ |
| Peptoid 70 | H-*N*Lys-*N*hTrp-*N*hTrp-*N*Lys-*N*spe-*N*spe-*N*Lys-*N*spe-*N*His-*N*Lys-*N*Lys-*N*spe-$NH_2$ |
| Peptoid 71 | H-*N*Lys-*N*hTrp-*N*hTrp-*N*Lys-*N*spe-*N*spe-*N*Lys-*N*spe-*N*spe-*N*Lys-*N*Lys-*N*His-$NH_2$ |
| Peptoid 72 | H-*N*His-*N*hTrp-*N*hTrp-*N*Lys-*N*spe-*N*spe-*N*Lys-*N*spe-*N*spe-*N*Lys-*N*Lys-*N*spe-$NH_2$ |
| Peptoid 73 | H-*N*Lys-*N*hTrp-*N*hTrp-*N*His-*N*spe-*N*spe-*N*Lys-*N*spe-*N*spe-*N*Lys-*N*Lys-*N*spe-$NH_2$ |
| Peptoid 74 | H-*N*Lys-*N*hTrp-*N*hTrp-*N*Lys-*N*spe-*N*spe-*N*His-*N*spe-*N*spe-*N*Lys-*N*Lys-*N*spe-$NH_2$ |
| Peptoid 75 | H-*N*Lys-*N*hTrp-*N*hTrp-*N*Lys-*N*spe-*N*spe-*N*Lys-*N*spe-*N*spe-*N*His-*N*Lys-*N*spe-$NH_2$ |
| Peptoid 76 | H-*N*Lys-*N*hTrp-*N*hTrp-*N*Lys-*N*spe-*N*spe-*N*Lys-*N*spe-*N*spe-*N*Lys-*N*His-*N*spe-$NH_2$ |
| Peptoid 77 | H-*N*Lys-*N*spe-*N*spe-*N*Lys-*N*spe-*N*spe-*N*Lys-*N*Lys-*N*spe-*N*His-*N*hTrp-*N*hTrp-$NH_2$ |
| Peptoid 78 | H-*N*Lys-*N*spe-*N*spe-*N*Lys-*N*spe-*N*spe-*N*Lys-*N*His-*N*spe-*N*Lys-*N*hTrp-*N*hTrp-$NH_2$ |
| Peptoid 79 | H-*N*Lys-*N*spe-*N*spe-*N*Lys-*N*spe-*N*spe-*N*His-*N*Lys-*N*spe-*N*Lys-*N*hTrp-*N*hTrp-*N*$H_2$ |
| Peptoid 80 | H-*N*Lys-*N*spe-*N*spe-*N*His-*N*spe-*N*spe-*N*Lys-*N*Lys-*N*spe-*N*Lys-*N*hTrp-*N*hTrp-$NH_2$ |
| Peptoid 81 | H-*N*His-*N*spe-*N*spe-*N*Lys-*N*spe-*N*spe-*N*Lys-*N*Lys-*N*spe-*N*Lys-*N*hTrp-*N*hTrp-$NH_2$ |

More specifically, the antimicrobial peptoid may be any one or more selected from Formulas 2 to 5 below.

[Formula 2] H-*N*Lys-*N*spe-*N*spe-*N*Lys-*N*spe-*N*spe-*N*Lys-*N*Lys-*N*spe-*N*Lys-*N*hTrp-*N*hTrp-$NH_2$;

[Formula 3] H-*N*Lys-*N*hTrp-*N*hTrp-*N*Lys-*N*spe-*N*spe-*N*Lys-*N*spe-*N*spe-*N*Lys-*N*Lys-*N*spe-$NH_2$;

[Formula 4] H-*N*Lys-*N*hTrp-*N*hTrp-*N*His-*N*spe-*N*spe-*N*Lys-*N*spe-*N*spe-*N*Lys-*N*Lys-*N*spe-$NH_2$; and

[Formula 5] H-*N*Lys-*N*spe-*N*spe-*N*Lys-*N*spe-*N*spe-*N*Lys-*N*His-*N*spe-*N*Lys-*N*hTrp-*N*hTrp-$NH_2$.

The term "*N*spe" used herein refers to (S)-N-(1-phenylethyl)glycine. The *N*spe may be a hydrophobic monomer and have the structure of Formula 6 below.

[Formula 6]

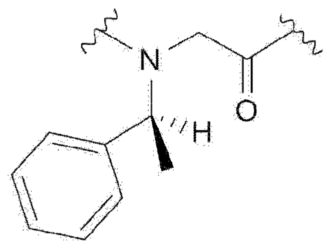

***N*spe**

The term "*N*Lys" used herein refers to N-(4-aminobutyl)glycine. The *N*Lys may have the structure of Formula 7 below, and may exhibit cationicity when dissolved in a solvent.

[Formula 7]

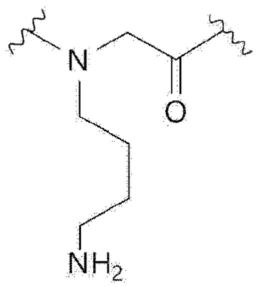

***N*Lys**

The term "*N*hTrp" used herein refers to N-[2-(1H-indol-3-yl)ethyl]glycine. The *N*hTrp is a monomer having an indole ring, and may have the structure of Formula 8 below.

[Formula 8]

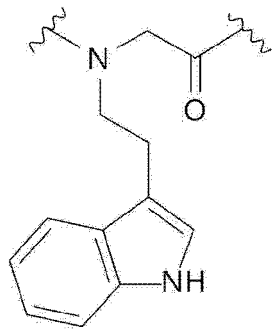

*N*hTrp

The term "*N*His" used herein refers to N-(methylimidazole)glycine. The *N*His is a monomer having an imidazole ring, and may have the structure of Formula 9 below.

[Formula 9]

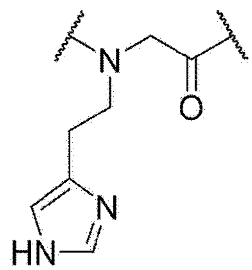

*N*His

The term "*N*pm" used herein refers to N-(benzyl)glycine. The *N*pm may have the structure of Formula 10 below.

[Formula 10]

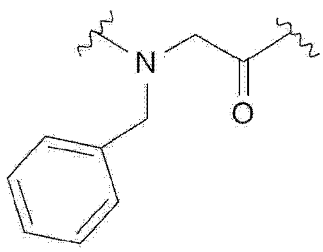

*N*pm

The term "*N*4hb" used herein refers to N-(4-hydroxybutyl)glycine. The *N*4hb may have the structure of Formula 11 below.

[Formula 11]

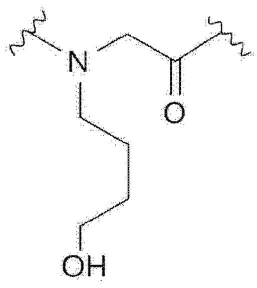

*N*4hb

The term "antimicrobial activity" used herein refers to the ability to resist bacteria, and all mechanisms performed to defend against the action of microorganisms such as bacteria, fungi, yeast, etc.

The term "gram-positive bacteria" used herein refers to bacteria appearing to be violet as a result of staining with crystal violet. Approximately 80% to 90% of the cell wall of the gram-positive bacteria may consist of peptidoglycan, which forms a thick layer to maintain the size and shape of the cell wall and make the cell wall hard.

The gram-positive bacteria may be bacteria of *Staphylococcus sp., Bacillus sp., Streptococcus sp.,* or *Enterococcus sp,* but the present invention is not limited thereto.

For example, the gram-positive bacteria may be *Staphylococcus aureus, methicillin-resistant Staphylococcus aureus* (MRSA), *Quinolone-resistant Staphylococcus aureus* (QRSA), *vancomycin-resistant enterococcus* (VRE), *vancomycin intermediate-resistant Staphylococcus aureus* (VISA), *Bacillus subtilis, Bacillus cereus, Streptococcus pneumoniae, Enterococcus faecalis, Enterococcus faecium,* or *Staphylococcus epidermidis.*

The term "gram-negative bacteria" used herein refers to bacteria which are not stained with crystal violet after staining with crystal violet, appearing to be purple as a result of decolorized by ethanol washing.

The gram-negative bacteria may be bacteria of *Salmonella sp., Acinetobacter sp., Escherichia sp., Pseudomonas sp., Enterobacter sp.,* or *Klebsiella sp.*.

For example, the gram-negative bacteria may be *Salmonella typhimurium, Acinetobacter calcoaceticus, Acinetobacter baumannii, Escherichia coli, Pseudomonas aeruginosa, Klebsiella pneumoniae,* or *Klebsiella aerogenes.*

The present invention may provide an antimicrobial composition, which includes at least one or more of the above-described antimicrobial peptoids as an active ingredient.

The antimicrobial peptoid may exhibit antimicrobial activity against gram-positive bacteria or gram-negative bacteria.

The antimicrobial peptoid of the present invention may include a monomer having an indole ring and a cationic monomer in order to solve the cytotoxic problem of a conventional peptoid.

The antimicrobial composition of the present invention may include a peptoid obtained by performing a counter ion-substitution reaction on trifluoroacetic acid (TFA) ions present in a compound. The counter ion substitution may be substitution of the TFA ions with chlorine ions or acetate ions. In other words, the synthesized peptoid compound may include TFA ions upon HPLC analysis or deprotecting group removal. Here, counter ion substitution which removes TFA ions remaining in the peptoid compound and also binds chlorine ions or acetate ions may be performed.

Hereinafter, the present invention will be described in further detail with reference to the following examples. However, the following examples are merely provided to exemplify the present invention, and the scope of the present invention is not limited thereto.

## I. Confirmation of change in peptoid activity according to substitution number and position of *N*hTrp

### 1. Preparation of peptoid submonomer

<Preparation Example 1: Preparation of N-[2-(1H-indol-3-yl)ethyl]glycine (*N*hTrp) submonomer>

As a peptoid submonomer, an NhTrp(Boc) monomer is prepared using tryptamine as a raw material. An M1 monomer is synthesized using tryptamine, an M2 monomer is synthesized using the M1 monomer, and finally, an M3 submonomer of *N*hTrp(Boc) is synthesized using the M2 monomer. In Scheme 1, the process of synthesizing the M3 peptoid submonomer of *N*hTrp(Boc) is shown.

[Scheme 1]

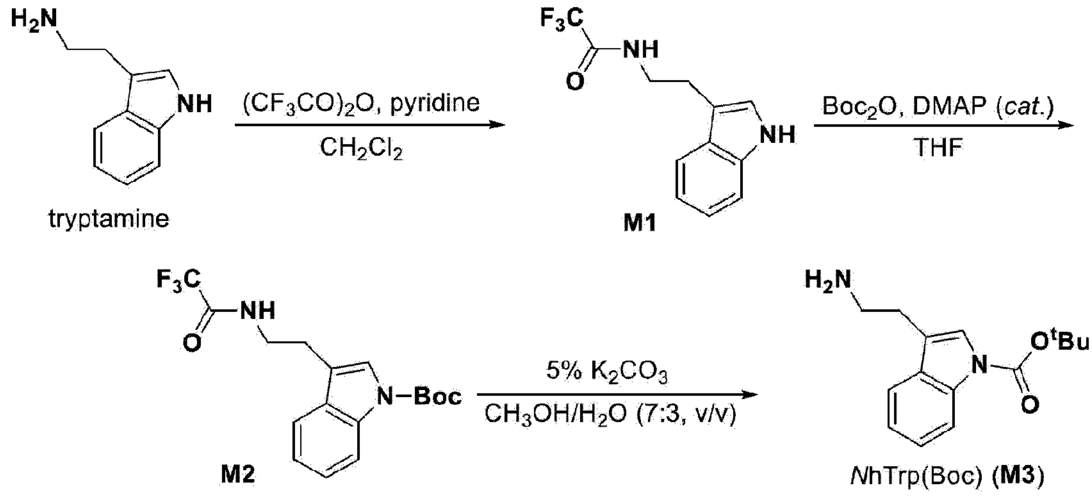


tryptamine M1 M2 *N*hTrp(Boc) (**M3**)

① Preparation of 2,2,2-trifluoro-N-[2-(1H-indol-3-yl)ethyl] acetamide (M1) monomer

Trifluoroacetic anhydride (4.5 mL, 32.6 mmol, 1.05 equiv.) is added to a dichloromethane ($CH_2Cl_2$, 280 mL) solution containing pyridine (28 mL, 346 mmol, 11.16 equiv.) and tryptamine (5 g, 31 mmol, 1 equiv.). This is performed in a cold bath at 0 °C in a nitrogen atmosphere. After 5 minutes, the cold bath is removed, and the mixture is stirred at 20 °C for 2 hours. After stirring, the mixture is diluted with dichloromethane ($CH_2Cl_2$), and washed with a saturated sodium bicarbonate aqueous solution ($NaHCO_3$). The product is dried by adding anhydrous sodium sulfate ($Na_2SO_4$) and filtered, followed by concentrating the remaining liquid *in vacuo.* The residue is used without an additional purification process.

(2) Preparation of tert-butyl-3-(2-2,2,2-trifluoroacetamido)ethyl)-1H-indole -1-carboxylate (M2) monomer

Di-tert-butyl decarbonate (5.2 g, 23.5 mmol, 1.2 equiv.) was added to a tetrahydrofuran (40 mL) solution containing the M1 monomer (5 g, 19.5 mmol, 1 equiv.), and 4-dimethylaminopyridine (DMAP) was added as a catalyst. The solution was heated for 1 hour at 40 °C, diluted with dichloromethane ($CH_2Cl_2$), and washed with water. The product was dried by adding anhydrous sodium sulfate ($Na_2SO_4$) and filtered, followed by concentrating the remaining liquid *in vacuo.* The residue was purified by chromatography using silica gel, thereby producing a colorless oil.

(3) Preparation of tert-butyl-3-(2-aminoethyl)-1H-indole-1-carboxylate (M3, NhTrp) submonomer

Potassium carbonate (5.8 g, 42 mmol, 3 equiv.) is added to a mixed solution in which the M2 monomer (4.6 g, 12 mmol, 1 equiv.) is mixed in a solvent prepared by mixing methanol and water in a volume ratio of 7:3. The mixture is stirred for 48 hours, and extract with dichloromethane ($CH_2Cl_2$) after pouring water thereinto. The extract is dried by adding anhydrous sodium sulfate ($Na_2SO_4$) and filtered, and then the remaining liquid is concentrated *in vacuo,* thereby obtaining an unpurified light-yellow oil. Afterward, when used in synthesis of a peptoid, the obtained product is used without additional purification.

<Preparation Example 2: Preparation of N-(tert-butoxycarbonyl)-1,4-butanediamine (*N*Lys(Boc)) submonomer>

1,4-diaminobutane (30.2 mL, 300 mmol, 10 equiv.) is dissolved in a dichloromethane ($CH_2Cl_2$, 1000 mL) solvent and stirred in a 500 mL round-bottom flask. Subsequently, di-tert-butyl-dicarbonate (6.55 g, 30.0 mmol, 1 equiv.) is added to the round-bottom flask containing the above solution for 3 hours. The reaction mixture is stirred overnight, and an unpurified solution is extracted with deionized water. An organic layer is transferred, and the extraction step is repeated twice. The organic layer is dried by adding anhydrous sodium sulfate anhydrous ($Na_2SO_4$), filtered, and dried *in vacuo,* thereby obtaining an unpurified light-yellow oil. The obtained product is used in the synthesis of a peptoid without additional purification.

<Preparation Example 3: Preparation of N-(methylimidazole)glycine (*N*His) submonomer>

As a peptoid submonomer, a *N*His(Trt) monomer is prepared using histamine as a raw material. An M1 monomer is synthesized using histamine, an M2 monomer is synthesized using the M1 monomer, and finally, an M3 submonomer of *N*His(Trt) is synthesized using the M2 monomer. In the following Scheme 2, the process of synthesizing the M3 submonomer of *N*His(Trt) is shown.

[Scheme 2]

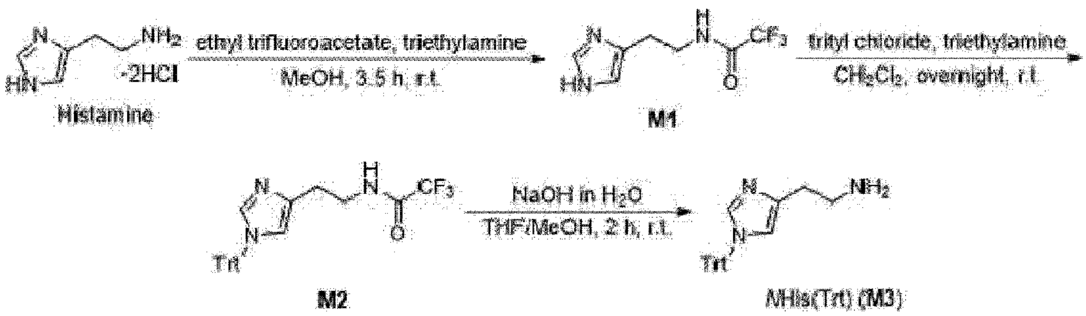


① Preparation of $N^{\alpha}$-trifluoroacetyl histamine(M1) monomer

Ethyl trifluoroacetate (1.43 mL, 12.0 mmol, 1.2 equiv.) and triethylamine (4.80 mL, 34.0 mmol, 3.4 equiv.) are slowly added to a methanol ($CH_3OH$, 30 mL) solution containing histamine dihydrochloride (1.84 g, 10.0 mmol, 1 equiv.) for 30 minutes. This is performed in a cold bath at 0 °C in a nitrogen atmosphere. After 5 minutes, the cold bath is removed, and the resulting mixture is stirred at 20 °C for 3.5 hours. After stirring, the mixture is diluted with dichloromethane($CH_2Cl_2$), and washed with water. The product is dried by adding anhydrous sodium sulfate ($Na_2SO_4$), and filtered, followed by concentrating the remaining liquid *in vacuo.* The residue is used without additional purification.

(2) Preparation of 2,2,2-trifluoro-N-[2-(1-trityl-1H-imidazol-4-yl)ethyl] acetamide(M2) monomer

Trityl chloride (3.33 g, 12.0 mmol, 1.2 equiv.) is slowly added to a methanol (30 mL) and dichloromethane (15 mL) solution containing the M1 monomer (2.07 g, 10.0 mmol, 1 equiv.) and triethylamine (4.24 mL, 30 mmol, 3.0 equiv.). The solution is stirred at 20 °C overnight. The resulting solution is diluted with chloroform ($CHCl_3$), and washed twice with water. The product is dried by adding anhydrous sodium sulfate ($Na_2SO_4$), and filtered, followed by concentrating the remaining liquid *in vacuo.* The residue is purified using hexane.

(3) Preparation of 2-(1-trityl-1H-imidazol-4-yl)-ethylamine(M3, *N*His(Trt)) submonomer

Sodium hydroxide (0.45 g, 11.3 mmol, 5 equiv.) is dissolved in water (5 mL) and added to a mixed solution in which the M2 monomer (1.00 g, 2.5 mmol, 1 equiv.) is mixed in a solvent in which tetrahydrofuran (9 mL) and methanol (12 mL) are mixed in a volume ratio of 3:4. The mixture is stirred for 2 hours, and concentrated *in vacuo.* Water is poured into the remaining liquid and extraction is performed with chloroform ($CHCl_3$). The organic extract is dried by adding anhydrous sodium sulfate ($Na_2SO_4$) and filtered, and then the remaining liquid is concentrated *in vacuo,* thereby obtaining an unpurified light-yellow oil. Afterward, when used in the synthesis of a peptoid, the obtained product is used without additional purification.

<Preparation Example 4: Preparation of 4-[tris(1-methylethyl)silyl]oxy]-1-butanamine (*N*4hb(TIPS)) submonomer>

Triisopropylsilyl chloride (24 mL, 0.12 mol, 1.1 equiv.) dissolved in dichloromethane (50 mL) was added to a cold solution in which 4-aminobutanol (10 g, 0.11 mol, 1 equiv.) and triethylamine (16.5 mL, 0.12 mol) were dissolved in dichloromethane (100 mL). The mixed solution was stirred at room temperature for 20 hours. The resulting suspension was extracted with water (150 mL). A process of extracting an aqueous phase with dichloromethane ($CH_2Cl_2$) was repeated three times. After combining the organic layers, sodium sulfate anhydrous ($Na_2SO_4$) was added for drying, and concentration was performed *in vacuo,* thereby obtaining a product. The product was used in the synthesis of a

peptoid without additional purification.

2. Preparation of antimicrobial peptoid

A method of preparing an antimicrobial composition of the present invention may include preparing deprotected polymer resin beads (S 100); preparing a bromoacetylate peptoid by adding the deprotected polymer resin beads, bromoacetic acid, diisopropylcarbodiimide (DIC), and an organic solvent and performing a bromoacetylation reaction (S200); adding a submonomer to the bromoacetylate peptoid and performing an amine substitution reaction (S300); obtaining an antimicrobial peptoid having a desired sequence by repeating S200 and S300 (S400); and separating a polymer resin from the antimicrobial peptoid using a cleavage solution (S500).

The antimicrobial peptoid of the present invention may be composed of monomers with various functional groups, that is, the above-described sequences of peptoid submonomer. The submonomer may be at least one selected from *N*hTrp(Boc), *N*Lys(Boc), *N*4hb(TIPS), *N*pm, and *N*spe, but the present invention is not limited thereto.

The antimicrobial peptoid of the present invention may be prepared by solid-phase peptoid synthesis (SPPS).

FIG. 1 is a schematic diagram illustrating the process of SPPS for preparing an antimicrobial peptoid of the present invention.

Referring to FIG. 1, a peptoid having a desired sequence may be obtained by repeating a two-step reaction of a bromoacetylation reaction and an amine substitution reaction several times using polymer beads with an amine group as a starting material. As amine submonomers, *N*hTrp(Boc), *N*Lys(Boc), *N*4hb(TIPS), *N*pm, *N*spe, and *N*His(Trt) (1.0 M, 20 equiv.) were dissolved in an NMP solvent and used.

<Example 1: Preparation of Peptoids 1 to 66>

Peptoids were synthesized on resin beads using a synthesis method in accordance with a solid-phase submonomer protocol, and synthesized by heating with a microwave. For heating with a microwave, a CEM MARS multimode microwave reaction system (CEM Corp.) was used. As a reaction scale, resin beads (0.065 mmol) were used (0.100 g of resin). First, to remove an Fmoc protecting group from the Fmoc-Rink amide MBHA resin (0.59 mmol/g), treatment with a 20% (v/v) piperidine-containing dimethylformamide (DMF) solution was performed twice at 80 °C (maximum power: 600 W, ramp 2 min, hold 2 min, stirring level 2). The peptoid sequences were determined by synthesis of a Rink Amide resin. The resin was washed sequentially with dichloromethane ($CH_2Cl_2$) three times, DMF three times, methanol once, DMF three times, and dichloromethane ($CH_2Cl_2$) three times.

Afterward, for a bromoacetylation reaction, bromoacetic acid (1.2 M in DMF, 20 equiv.) and N,N'-diisopropylcarbodiimide (DIC, 20 equiv.) were added to the deprotected resin, stirred and irradiated at 35 °C in a microwave oven (maximum power: 300 W, ramp 30 sec, hold 1 min, stirring level 2), and then the resin was washed in the same manner as the above-described washing sequence.

Subsequently, for an amine substitution reaction, amine submonomers (1.0 M, 20 equiv.) such as *N*hTrp(Boc), *N*Lys(Boc), *N*4hb(TIPS), *N*pm, and *N*spe were used. An amine substitution reaction was carried out by adding the amine submonomer to a bromoacetylate peptoid. Peptoids having desired sequences were synthesized by stirring a mixture of the amine submonomers and the bromoacetylate peptoids in a microwave oven (maximum power: 300 W, 95 °C, ramp 2 min, hold 90 sec, stirring level 2) and by using SN2 reaction for the mixture. The bromoacetylation reaction and the amine substitution reaction were repeated until a desired peptoid sequence was obtained.

Afterward, to separate the peptoid from the resin, separation was performed using a cleavage solution (trifluoroacetate (TFA):$CH_2Cl_2$:triisopropylsilane=95:2.5:2.5(v/v/v)) at room temperature for 10 minutes.

The chemical structures of Peptoids 1 to 66 according to Example 1 of the present invention are shown in Tables 5 to 7 below. Peptoids 1 and 2 are known peptoids, which are prepared to compare antimicrobial activity and cytotoxicity with the novel peptoids.

[Table 5]

| Classification | Chemical Structure |
| --- | --- |
| Peptoid 1 | 1 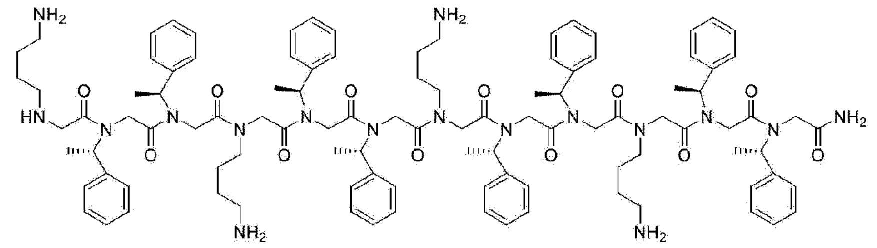 |
| Peptoid 2 | 2 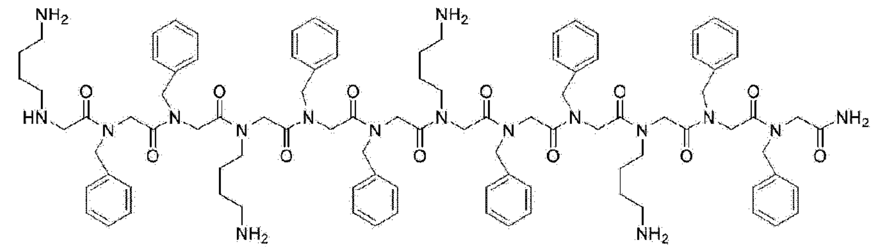 |
| Peptoid 3 | 3 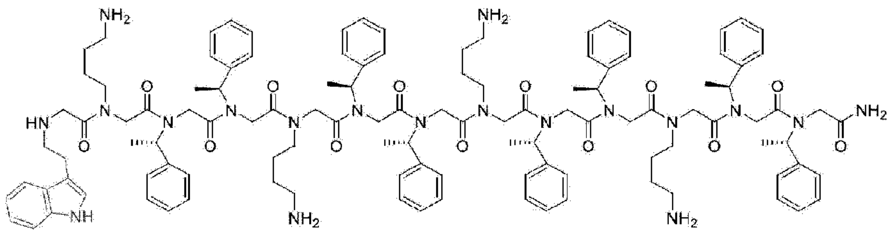 |
| Peptoid 4 | 4 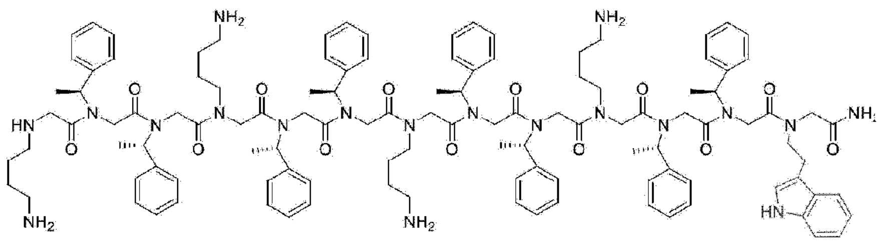 |
| Peptoid 5 | 5 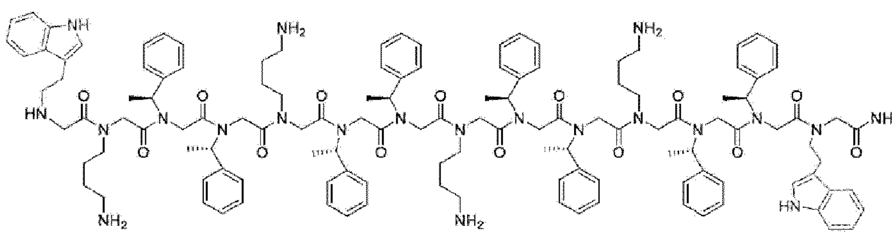 |
| Peptoid 6 | 6 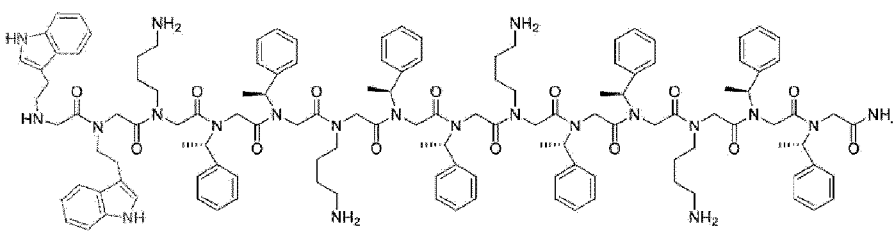 |

(continued)

| Classification | Chemical Structure |
|---|---|
| Peptoid 7 | **7** 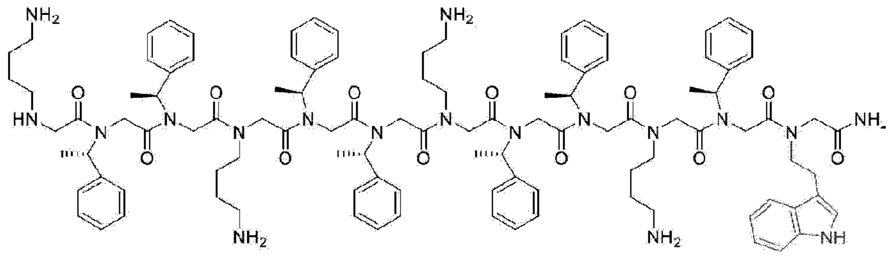 |
| Peptoid 8 | **8** 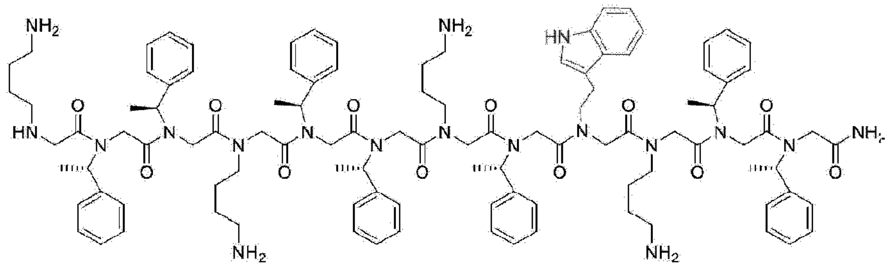 |
| Peptoid 9 | **9** 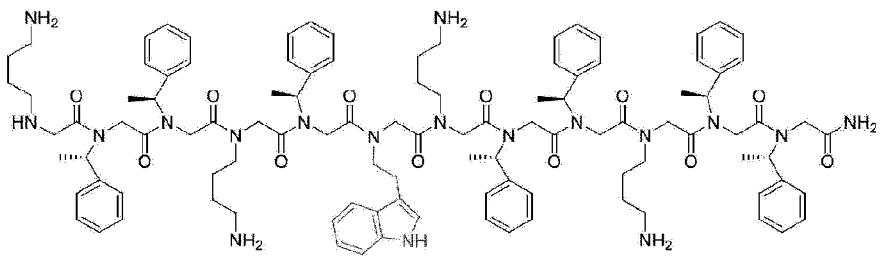 |
| Peptoid 10 | **10** 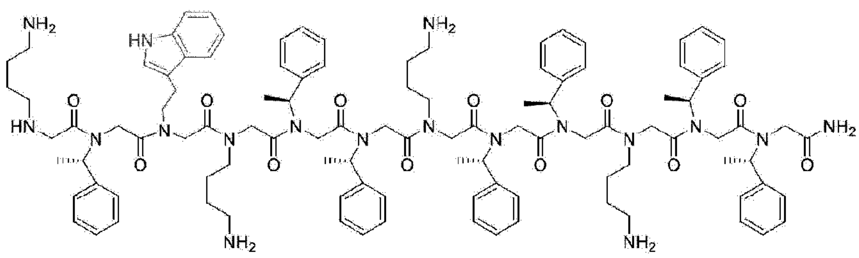 |
| Peptoid 11 | **11** 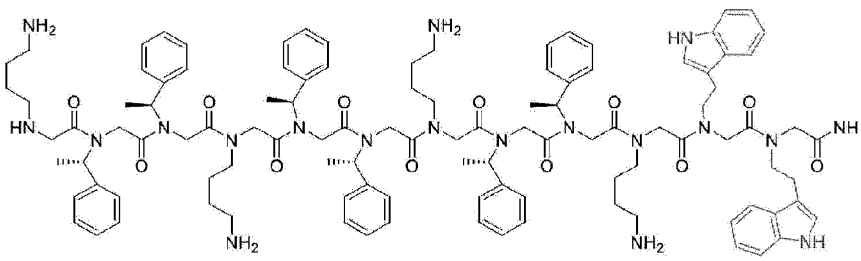 |
| Peptoid 12 | **12** 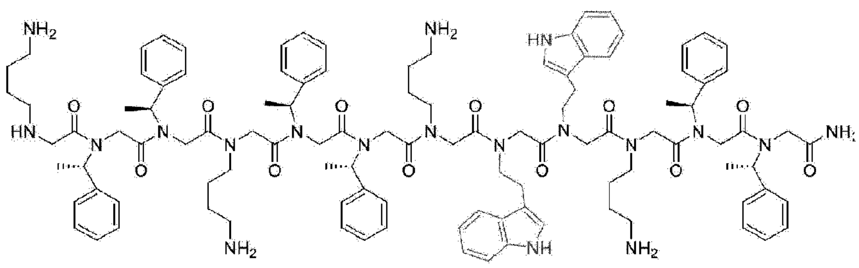 |

(continued)

| Classification | Chemical Structure |
|---|---|
| Peptoid 13 | **13** 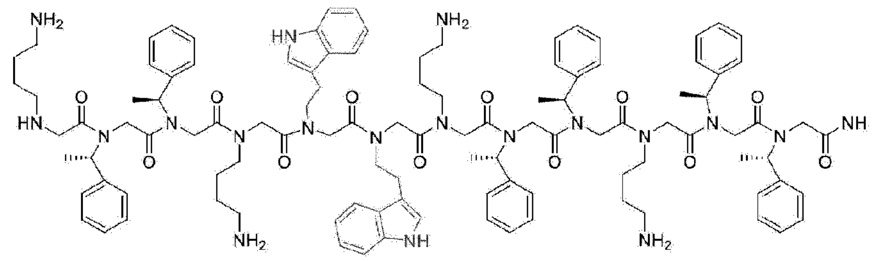 |
| Peptoid 14 | **14** 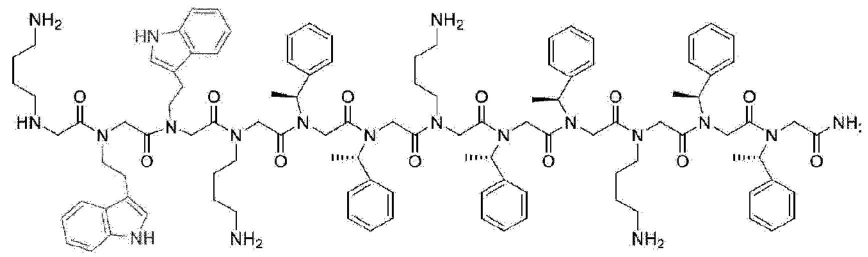 |
| Peptoid 15 | **15** 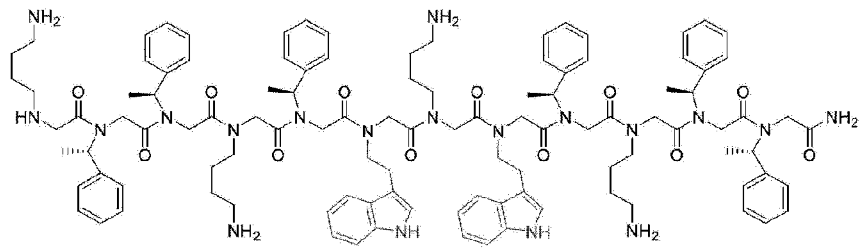 |
| Peptoid 16 | **16** 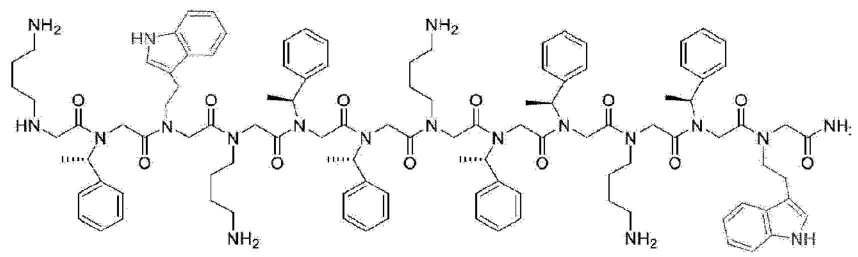 |
| Peptoid 17 | **17** 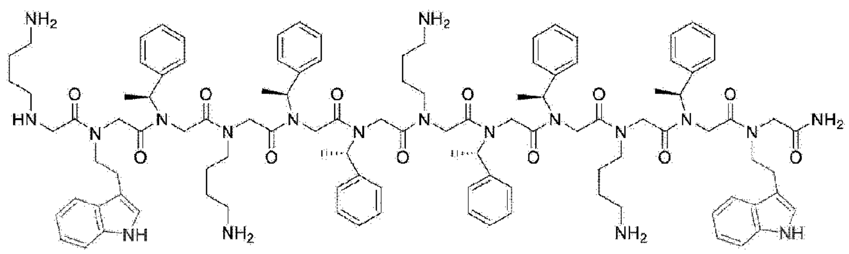 |
| Peptoid 18 | **18** 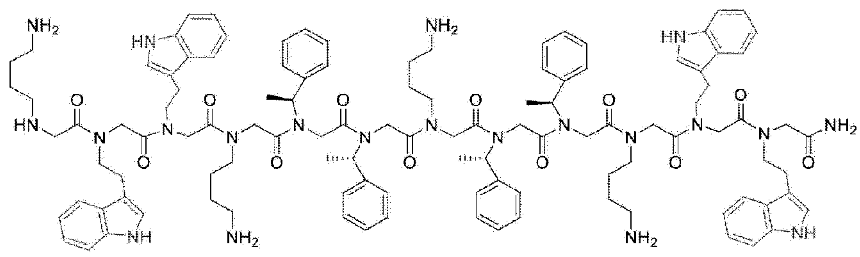 |

(continued)

| Classification | Chemical Structure |
|---|---|
| Peptoid 19 | **19** 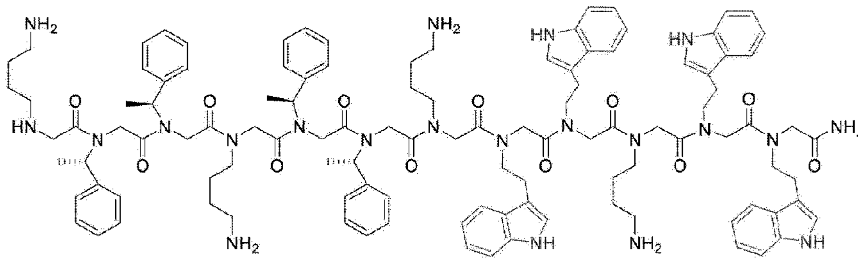 |
| Peptoid 20 | **20** 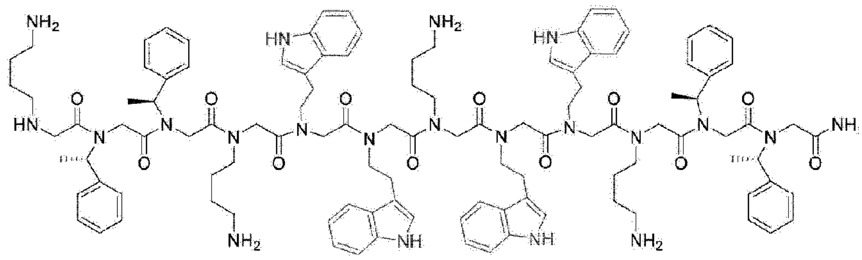 |
| Peptoid 21 | **21** 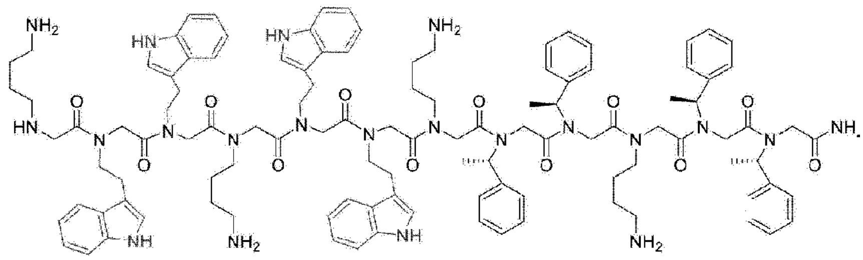 |
| Peptoid 22 | **22** 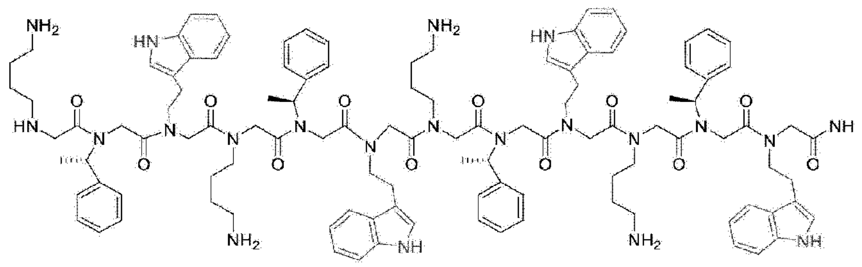 |
| Peptoid 23 | **23** 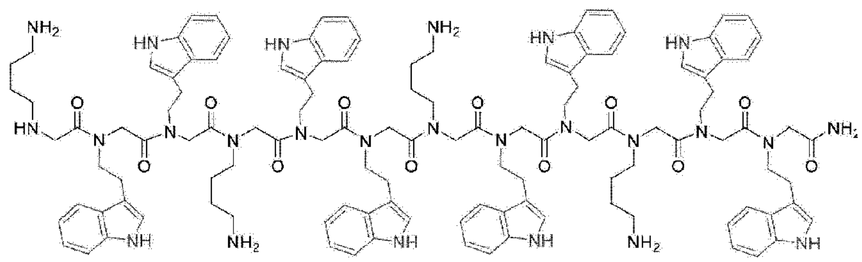 |

[Table 6]

| Classification | Chemical Structure |
|---|---|
| Peptoid 24 | **24** 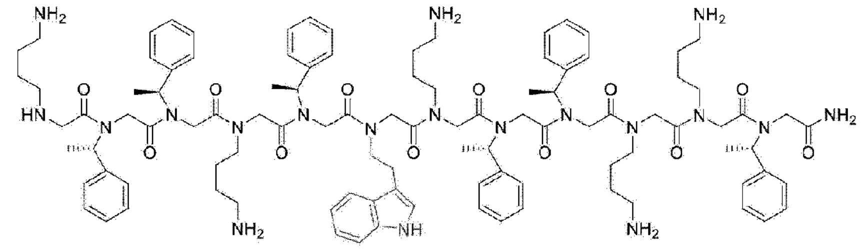 |
| Peptoid 25 | **25** 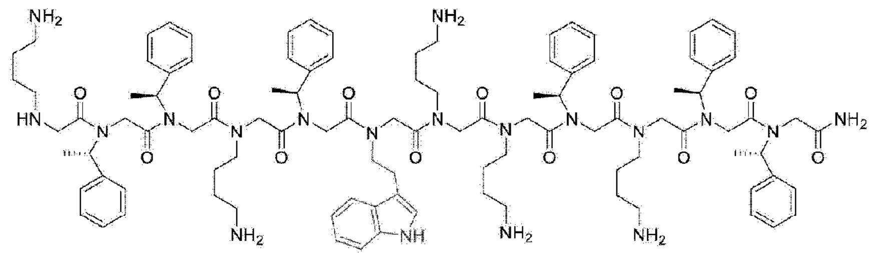 |
| Peptoid 26 | **26** 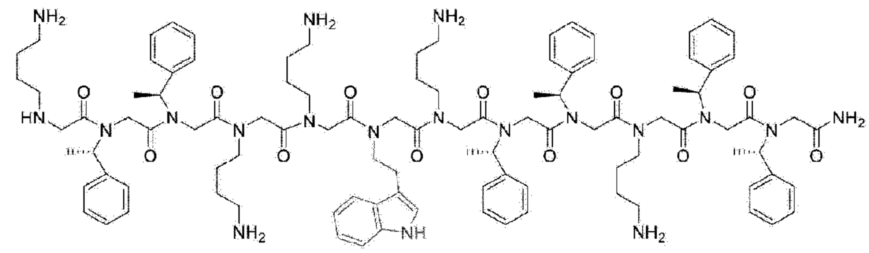 |
| Peptoid 27 | **27** 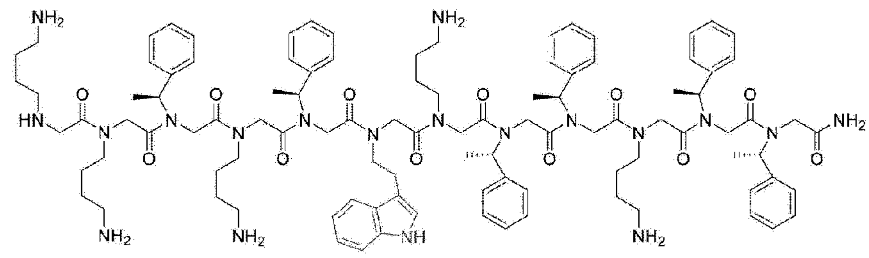 |
| Peptoid 28 | **28** 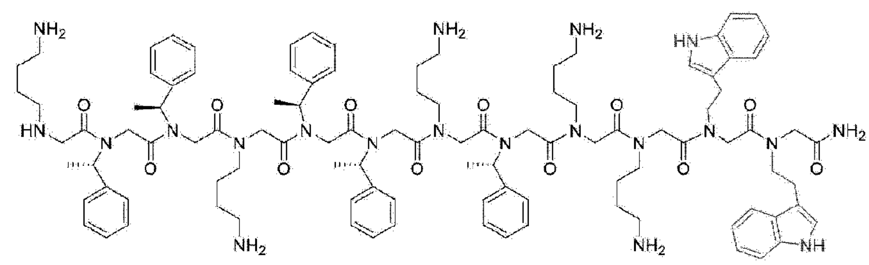 |
| Peptoid 29 | **29** 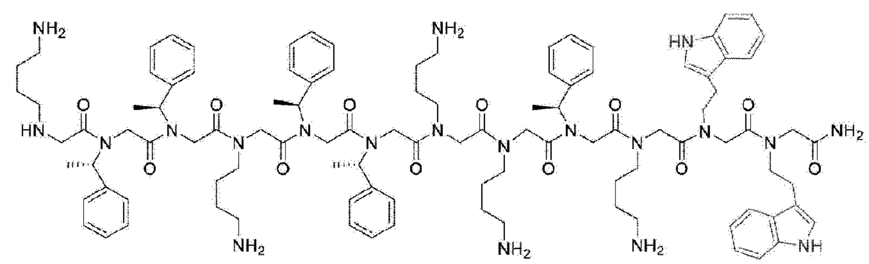 |

(continued)

| Classification | Chemical Structure |
| --- | --- |
| Peptoid 30 | 30 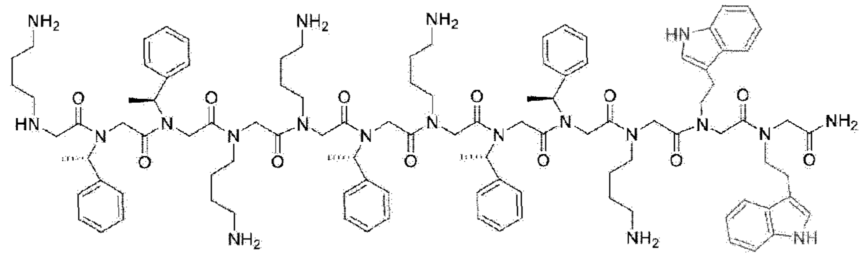 |
| Peptoid 31 | 31 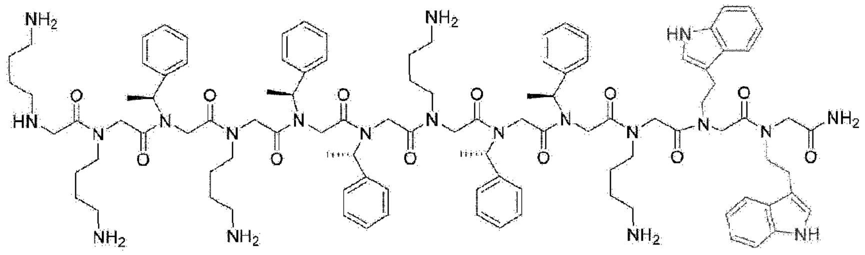 |
| Peptoid 32 | 32 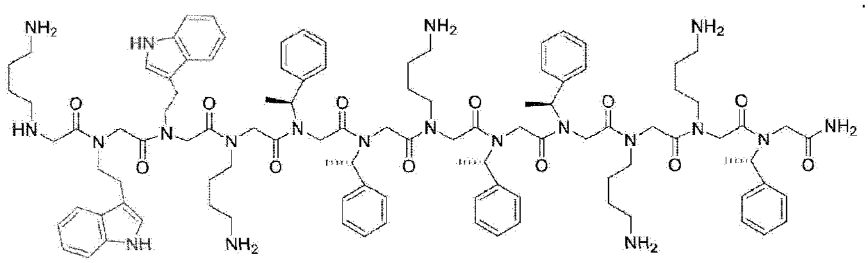 |
| Peptoid 33 | 33 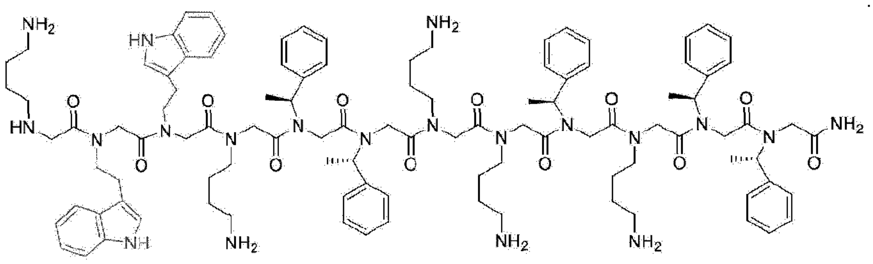 |
| Peptoid 34 | 34 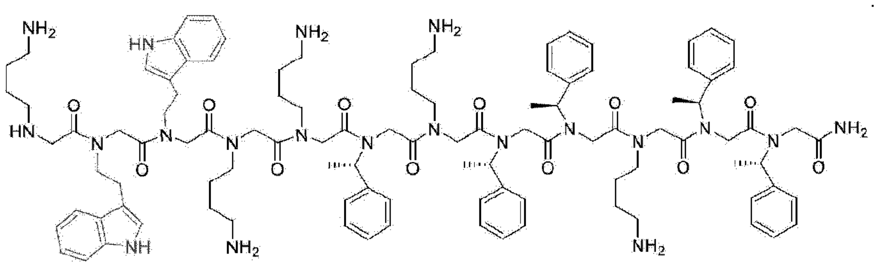 |
| Peptoid 35 | 35 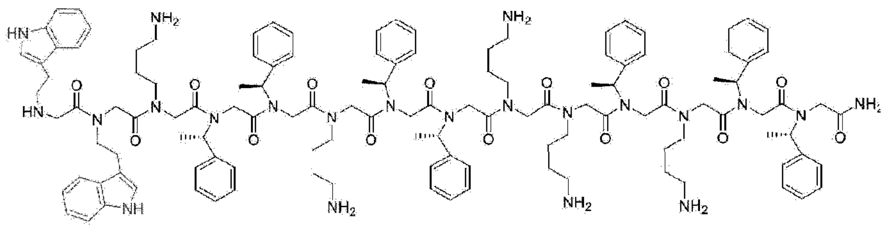 |

(continued)

| Classification | Chemical Structure |
| --- | --- |
| Peptoid 36 | **36** 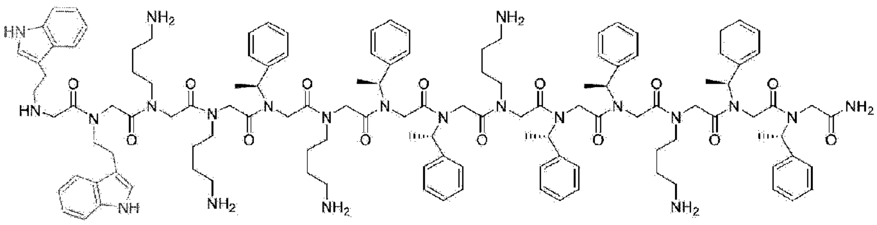 |
| Peptoid 37 | **37** 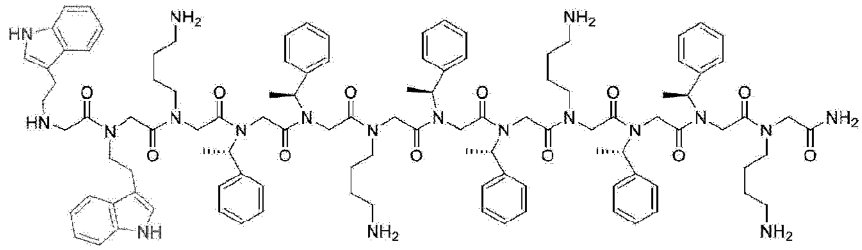 |
| Peptoid 38 | **38** 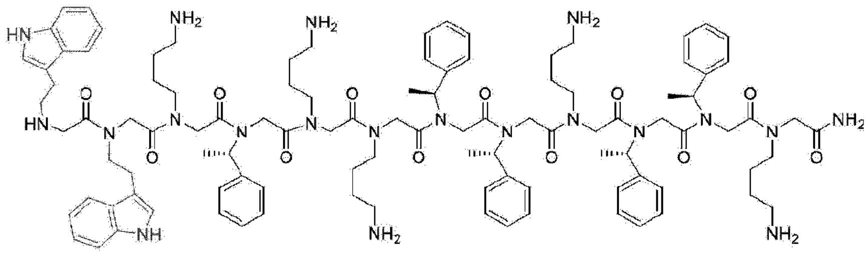 |
| Peptoid 39 | **39** 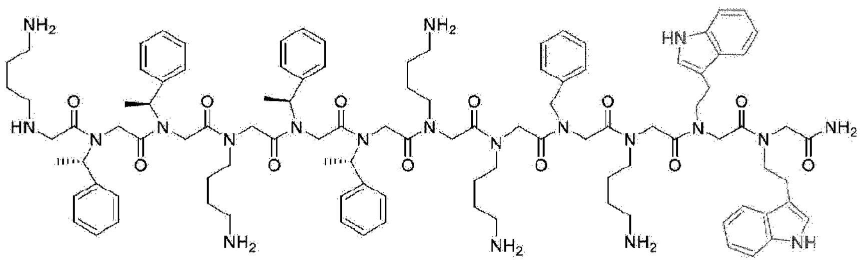 |
| Peptoid 40 | **40** 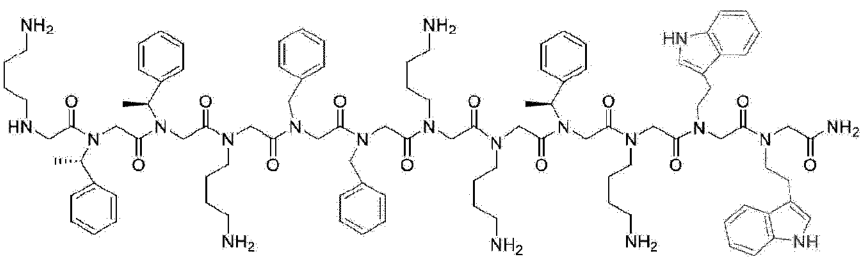 |
| Peptoid 41 | **41** 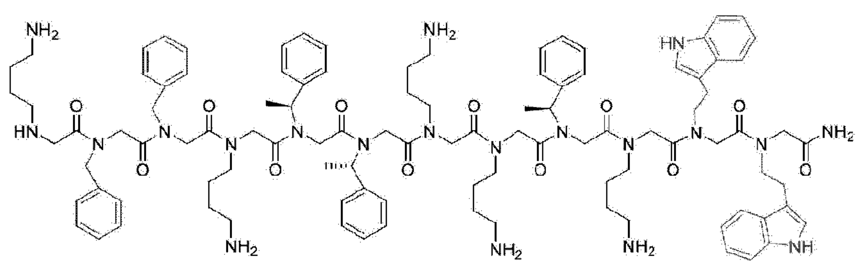 |

(continued)

| Classification | Chemical Structure |
|---|---|
| Peptoid 42 | 42 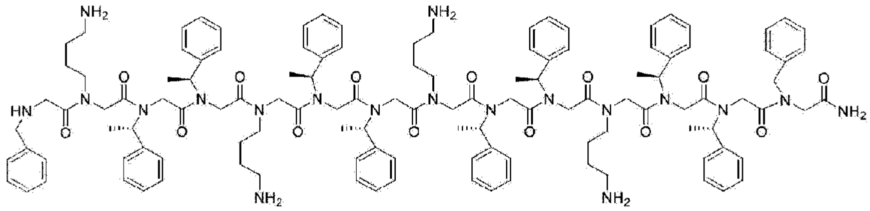 |
| Peptoid 43 | 43 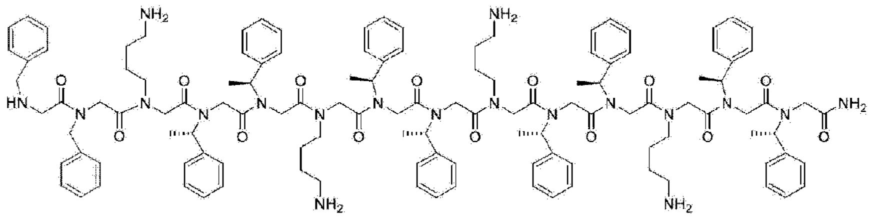 |
| Peptoid 44 | 44 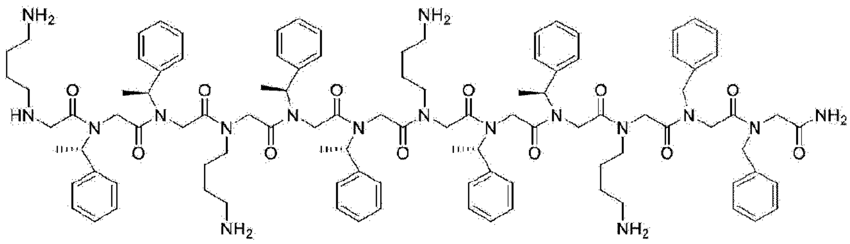 |
| Peptoid 45 | 45 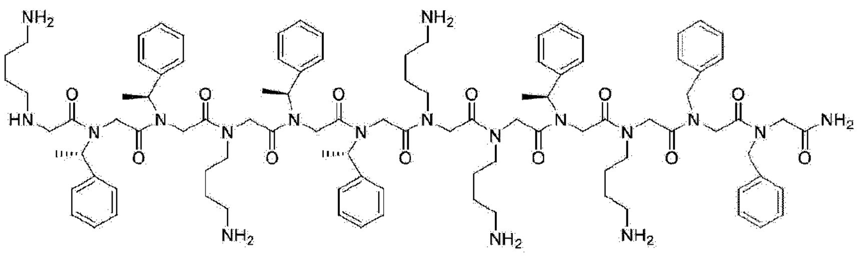 |
| Peptoid 46 | 46 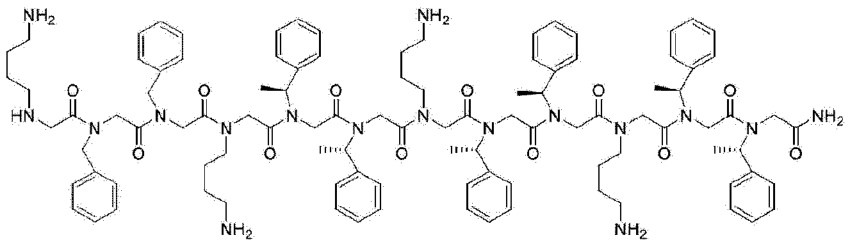 |
| Peptoid 47 | 47 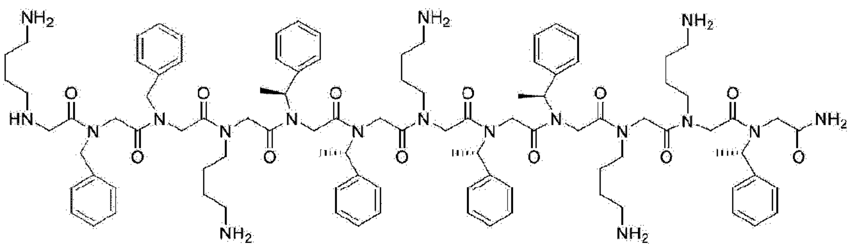 |

(continued)

| Classification | Chemical Structure |
|---|---|
| Peptoid 48 | 48 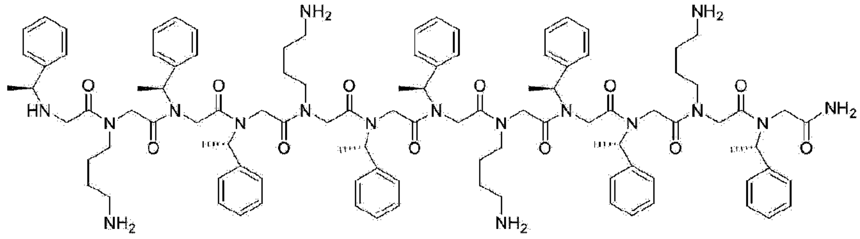 |

[Table 7]

| Classificatio n | Chemical Structure |
|---|---|
| Peptoid 49 | 49 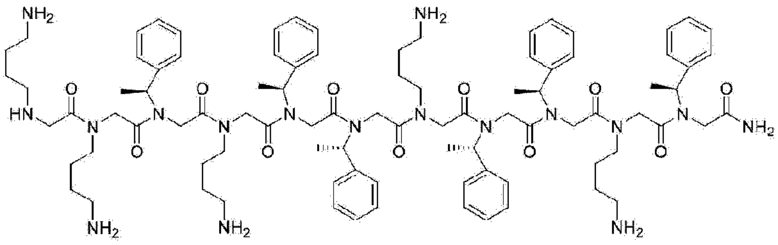 |
| Peptoid 50 | 50 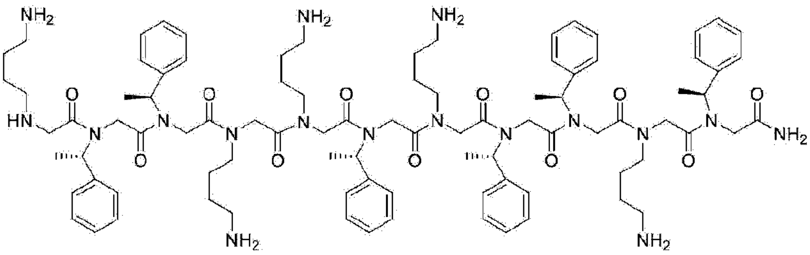 |
| Peptoid 51 | 51 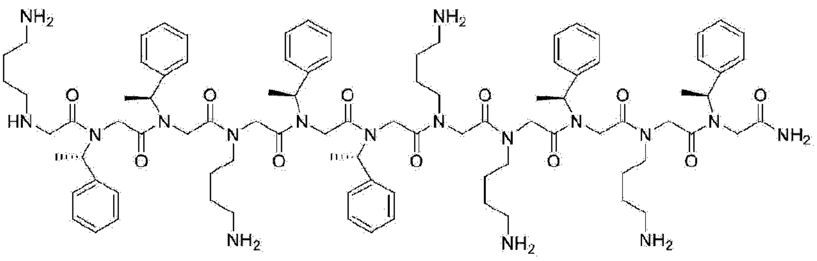 |
| Peptoid 52 | 52 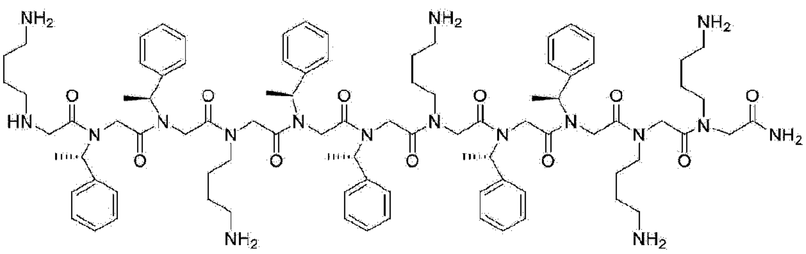 |

(continued)

| Classificatio n | Chemical Structure |
|---|---|
| Peptoid 53 | 53 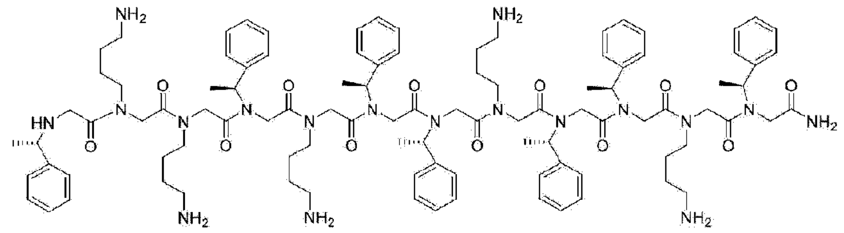 |
| Peptoid 54 | 54 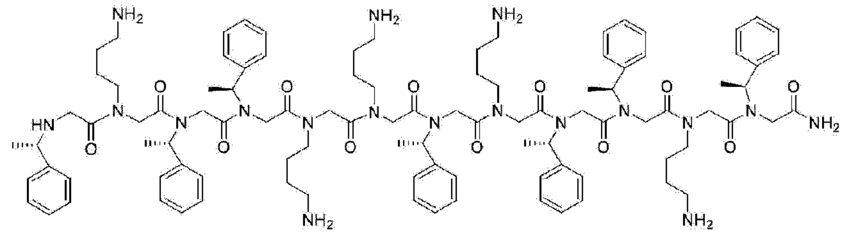 |
| Peptoid 55 | 55 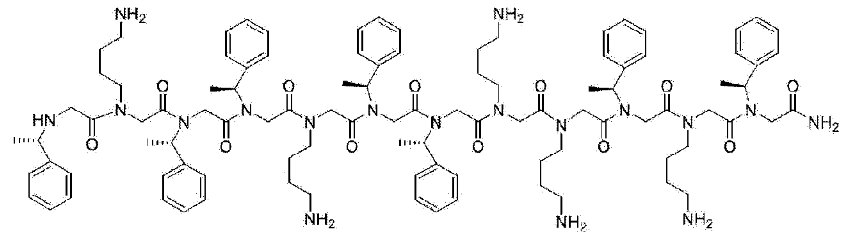 |
| Peptoid 56 | 56 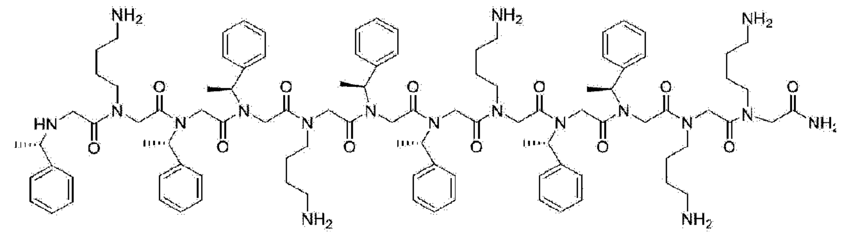 |
| Peptoid 57 | 57 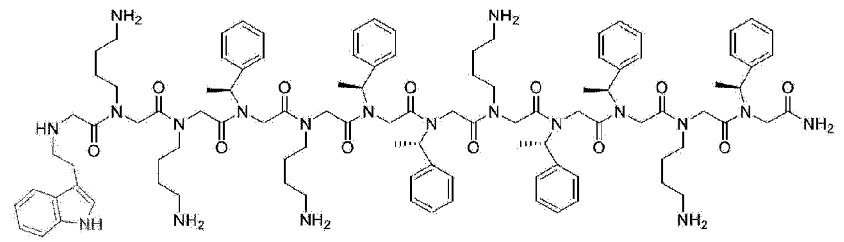 |
| Peptoid 58 | 58 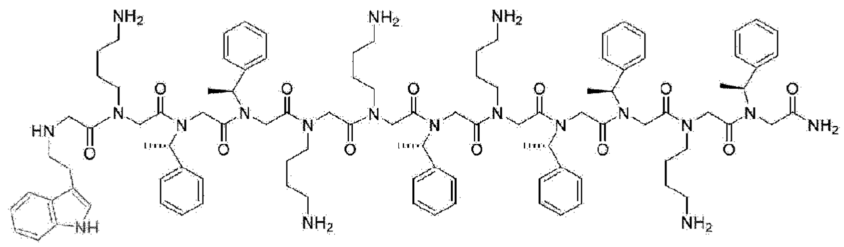 |

(continued)

| Classificatio n | Chemical Structure |
|---|---|
| Peptoid 59 | 59 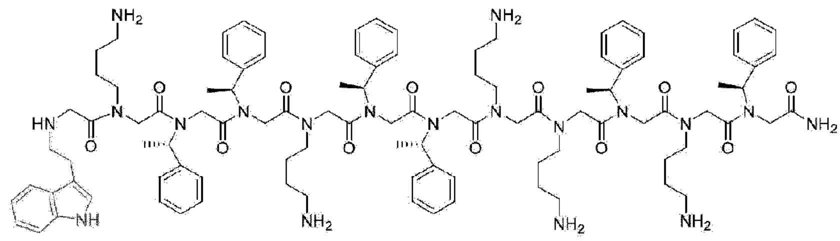 |
| Peptoid 60 | 60 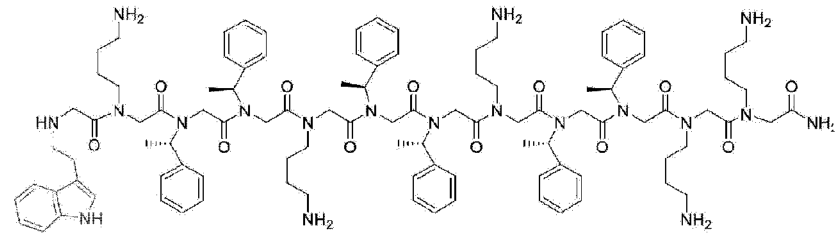 |
| Peptoid 61 | 61 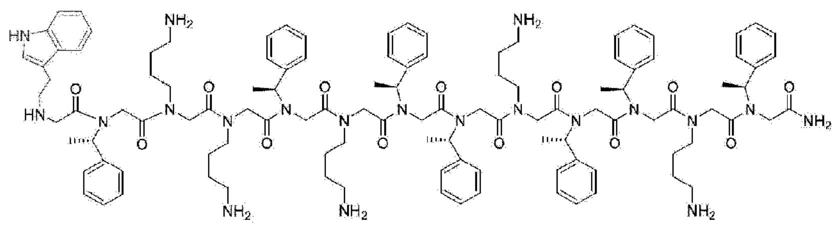 |
| Peptoid 62 | 62 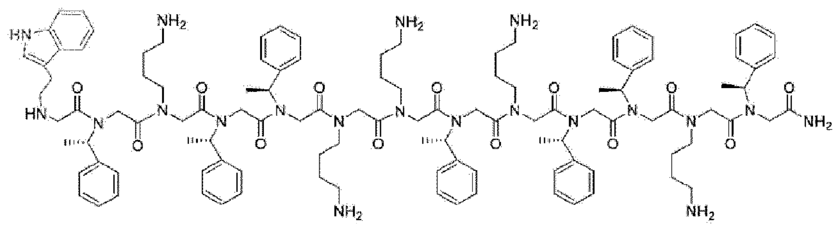 |
| Peptoid 63 | 63 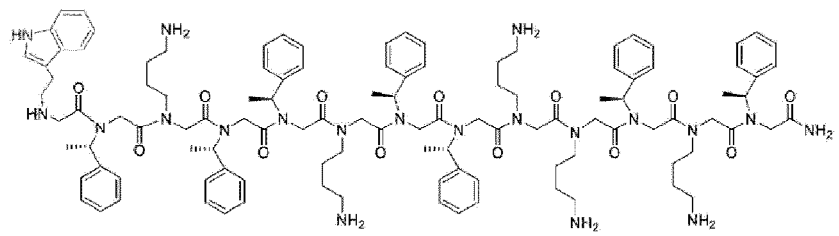 |
| Peptoid 64 | 64 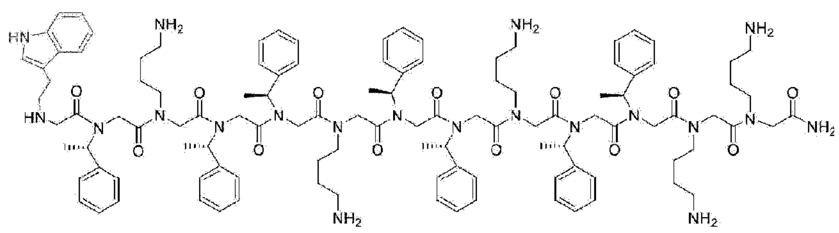 |

(continued)

| Classificatio n | Chemical Structure |
|---|---|
| Peptoid 65 | 65 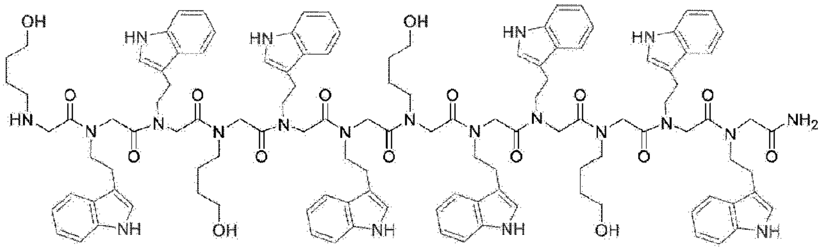 |
| Peptoid 66 | 66 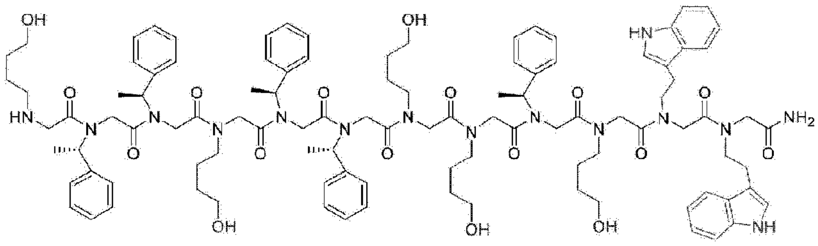 |

[Table 8]

| Classification | Number of *N*hTrps | CTLR | HPLC elution (%ACN) |
|---|---|---|---|
| Peptoid 1 | 0 | 0.33 | 54.5 |
| Peptoid 2 | 0 | 0.33 | 50.7 |
| Peptoid 3 | 1 | 0.31 | 55.8 |
| Peptoid 4 | 1 | 0.31 | 57 |
| Peptoid 5 | 2 | 0.29 | 58.6 |
| Peptoid 6 | 2 | 0.29 | 57.2 |
| Peptoid 7 | 1 | 0.33 | 54.1 |
| Peptoid 8 | 1 | 0.33 | 54.5 |
| Peptoid 9 | 1 | 0.33 | 54.6 |
| Peptoid 10 | 1 | 0.33 | 54.9 |
| Peptoid 11 | 2 | 0.33 | 54 |
| Peptoid 12 | 2 | 0.33 | 55.9 |
| Peptoid 13 | 2 | 0.33 | 56 |
| Peptoid 14 | 2 | 0.33 | 55.6 |
| Peptoid 15 | 2 | 0.33 | 53.7 |
| Peptoid 16 | 2 | 0.33 | 54.7 |
| Peptoid 17 | 2 | 0.33 | 56 |
| Peptoid 18 | 4 | 0.33 | 53.8 |
| Peptoid 19 | 4 | 0.33 | 52.8 |
| Peptoid 20 | 4 | 0.33 | 52.7 |
| Peptoid 21 | 4 | 0.33 | 53.2 |
| Peptoid 22 | 4 | 0.33 | 54.1 |

(continued)

| Classification | Number of *N*hTrps | CTLR | HPLC elution (%ACN) |
|---|---|---|---|
| Peptoid 23 | 8 | 0.33 | 53 |

[Table 9]

| Classification | Number of *N*hTrps | CTLR | HPLC elution (%ACN) |
|---|---|---|---|
| Peptoid 24 | 1 | 0.42 | 49.4 |
| Peptoid 25 | 1 | 0.42 | 50.8 |
| Peptoid 26 | 1 | 0.42 | 49.5 |
| Peptoid 27 | 1 | 0.42 | 50.7 |
| Peptoid 28 | 2 | 0.42 | 48.6 |
| Peptoid 29 | 2 | 0.42 | 48.6 |
| Peptoid 30 | 2 | 0.42 | 48.8 |
| Peptoid 31 | 2 | 0.42 | 50.2 |
| Peptoid 32 | 2 | 0.42 | 48.4 |
| Peptoid 33 | 2 | 0.42 | 47.9 |
| Peptoid 34 | 2 | 0.42 | 48.6 |
| Peptoid 35 | 2 | 0.36 | 51.8 |
| Peptoid 36 | 2 | 0.36 | 52.4 |
| Peptoid 37 | 2 | 0.33 | 52.7 |
| Peptoid 38 | 2 | 0.42 | 47.8 |
| Peptoid 39 | 2 | 0.42 | 47.8 |
| Peptoid 40 | 2 | 0.42 | 47.5 |
| Peptoid 41 | 2 | 0.42 | 47.5 |
| Peptoid 42 | 0 | 0.29 | 57.7 |
| Peptoid 43 | 0 | 0.29 | 56.8 |
| Peptoid 44 | 0 | 0.33 | 53.1 |
| Peptoid 45 | 0 | 0.42 | 47.3 |
| Peptoid 46 | 0 | 0.33 | 53.7 |
| Peptoid 47 | 0 | 0.42 | 47.8 |
| Peptoid 48 | 0 | 0.33 | 53.4 |

[Table 10]

| Classification | Number of *N*hTrps | CTLR | HPLC elution (%ACN) |
|---|---|---|---|
| Peptoid 49 | 0 | 0.45 | 46.6 |
| Peptoid 50 | 0 | 0.45 | 46.1 |
| Peptoid 51 | 0 | 0.45 | 45.3 |
| Peptoid 52 | 0 | 0.45 | 46. 9 |
| Peptoid 53 | 0 | 0.42 | 48.5 |

(continued)

| Classification | Number of *N*hTrps | CTLR | HPLC elution (%ACN) |
|---|---|---|---|
| Peptoid 54 | 0 | 0.42 | 48.1 |
| Peptoid 55 | 0 | 0.42 | 47.2 |
| Peptoid 56 | 0 | 0.42 | 48 |
| Peptoid 57 | 1 | 0.42 | 49.1 |
| Peptoid 58 | 1 | 0.42 | 48.7 |
| Peptoid 59 | 1 | 0.42 | 47.8 |
| Peptoid 60 | 1 | 0.42 | 48.6 |
| Peptoid 61 | 1 | 0.38 | 51.2 |
| Peptoid 62 | 1 | 0.38 | 50.8 |
| Peptoid 63 | 1 | 0.38 | 49.9 |
| Peptoid 64 | 1 | 0.38 | 50.5 |
| Peptoid 65 | 8 | 0 | 66.7 |
| Peptoid 66 | 2 | 0 | 64.7 |

<Analysis Example 1: HPLC and LC-MS analysis>

The synthesized peptoids were purified and analyzed using an HPLC system (SunFire C18, 4.6x250mm, 5 μm; Waters Corp, USA). The structure and HPLC analysis results of the synthesized peptoids are shown in Tables 8 to 10. Here, CTLR refers to a charge-to-length-ratio, defined as a ratio of the number of cationic residues to the number of total residues in each sequence. In addition, in HPLC elution analysis, water (containing 0.1% TFA) and acetonitrile (containing 0.1% TFA) were used as mobile phases. As a result, the synthesized peptoids generally showed a retention time within the range of 45 minutes to 60 minutes in the HPLC elution analysis, confirming the degree of hydrophobicity of the synthesized peptoids.

<Analysis Example 2: Circular dichroism (CD) spectroscopy>

CD spectroscopy is a spectroscopic analysis method used to identify the secondary structure of a biomolecule such as a protein, a peptide, or a nucleic acid, and CD spectra were measured using a Jasco model 815 spectropolarimeter (Jasco, USA). CD spectroscopy was performed on a peptoid salt (50 μM in ACN) at room temperature. For the peptoid concentration, the dry weight of a lyophilized peptoid based on a TFA salt was used.

FIGS. 2 to 4 are graphs showing CD spectra for examples at 190 nm to 260 nm.

Referring to FIG. 2, it can be seen that a peak is shown at 200 nm and 220 nm, indicating that Peptoid 1 has an alpha helix. Meanwhile, it can be confirmed that, in Peptoids 22 and 23 having a peptoid group substituted with NhTrp, peak intensities at 200 nm and 220 nm were reduced, and peaks disappeared.

In addition, referring to FIG. 3, the CD spectra of Peptoids 11 to 14 were measured to determine a structural change according to the position of *N*hTrp substituted in Peptode 1. In the case of Peptoid 11, compared to other peptoid isomers, the peak intensities at 200 nm and 220 nm were greatly reduced when the C-terminus was replaced with NhTrp. Accordingly, it can be seen that two adjacent *N*hTrps of C-terminus included in the Peptoid 11 affect the secondary structure of the peptoid.

Meanwhile, referring to FIG. 4, CD spectra for Peptoids 28 to 31 in which cationic charges are increased by replacing *N*spe in Peptoid 11 with *N*Lys are shown. There were no significant differences in Peptoids 28 to 31, compared to Peptoid 11.

<Analysis Example 3: Antimicrobial activity assay>

To measure the antimicrobial activity of the peptoids synthesized in Example 1, an *E. coli* ATCC25922 strain as gram-negative bacteria and an *S. aureus* KCTC25923 strain as gram-positive bacteris, were treated with the peptoids, followed by measuring the minimum inhibitory concentration (MIC). Peptoids 1 and 2 are antimicrobial peptoids known in the art, and used as controls for comparing antimicrobial activity with a novel peptoid.

Specifically, the *S. aureus* KCTC25923 or *E. coli* ATCC25922 strain was inoculated into a Mueller Hinton Broth (MHB2) liquid medium in which cations had been adjusted in the medium, cultured at 37 °C for 18 hours, subcultured, and then incubated for 4 hours. Afterward, for each bacterial strain, an optical density (OD, 0.001) was adjusted so that the bacterial count became $2 \times 10^5$ to $5 \times 10^5$ colony-forming units (CFU) per mL, which was then diluted with a MHB2 liquid medium containing 0.02% bovine serum albumin and 0.001% acetic acid, and dispensed at 100 μL into a 96-well microplate. Using sterile water, a peptoid stock 40 times stronger than a concentration to be tested was subjected to two-fold serial dilution and assessed at six different concentrations, and the maximum value of the peptoid concentrations was 25 μM. Subsequently, the plate was incubated at 37 °C for 24 hours. Afterward, optical density (OD) values at 600 nm were measured using a microplate reader (Bio-Rad).

The lowest peptoid concentration preventing turbidity observed by visual inspection was recorded as the minimum inhibitory concentration (MIC), and the measurement results are shown in Tables 11 to 13 below. Each MIC value was determined as the average value of a value obtained through three independent experiments.

As a result, Peptoids 3 to 6 obtained by adding NhTrp to Peptoid 1 showed increased MIC values for *E. coli,* compared to Peptoid 1. However, MIC and $HC_{10}$ values for *S. aureus* were reduced or maintained constant.

Particularly, Peptoids 5 and 6 lost antimicrobial activities against *E. coli* even though only two *N*hTrps were added to Peptoid 1. Peptoids 7 to 10 in which one *N*spe in Peptoid 1 was substituted with *N*hTrp exhibited very similar or stronger antimicrobial activities and cytotoxicity against red blood cells. Peptoids 11 to 14, and 15 to 17 in which two *N*spes were substituted with *N*hTrp exhibited similar antimicrobial activity and cytotoxicity, compared to Peptoid 1.

On the other hand, Peptoids 18 to 22 in which four *N*spe groups were substituted with *N*hTrp had decreased antimicrobial activity against *E. coli,* but the other aspect, cytotoxicity, remained the same.

When all *N*spe groups of Peptoid 1 were replaced with *N*hTrp, along with the minimal change in antimicrobial activity against *S. aureus* or red blood cells, the antimicrobial activity against *E. coli* was reduced.

That is, it can be seen that the antimicrobial activity against *E. coli* tended to decrease when the helical structure is weakened and replaced with an indole ring, but the toxicities against gram-positive bacteria and red blood cells were maintained at a level similar to that of a benzene ring compound (*N*spe).

In addition, when samples have the same ionic charges, it was confirmed that a helical structure is associated with the MIC against *E. coli.* Accordingly, it can be seen that, as more NhTrps are substituted, the MIC against *E. coli* is lowered, and it can be confirmed that, as the position of the substituted NhTrp approaches the N-terminus, antimicrobial activity increases.

<Analysis Example 4: Hemolysis assay>

To confirm cytotoxicity by the hemolytic activity of each peptoid synthesized in Example 1, hemolysis analysis was performed. The used blood was collected from a 13-week-old male Sprague-Dawley mouse in a tube containing 158 UPS sodium heparin for preventing coagulation, and centrifuged at 4 °C for 10 minutes at 1,000 rpm. Afterward, the plasma was carefully removed, red blood cells were washed with phosphate-buffered saline (PBS: a mixed solution of 35 mM phosphate-buffered solution/150 mM NaCl, pH 7.4) three times and centrifuged at 1,000 rpm for 10 minutes. A 10% (v/v) red blood cell solution diluted in PBS was dispensed at 150 μL into a 96-well plate (microtiter plate), a peptoid solution was added at 50 μL, and 1% Triton X-100 was added.

Afterward, the red blood cells were incubated at 37 °C for 1 hour, and the incubated 96-well plate was centrifuged at 1,000 rpm for 15 minutes. A supernatant was obtained and transferred to a new 96-well plate at 150 μL, and absorbance was measured using a spectrophotometer at 540 nm. Percent hemolysis may be calculated by the following Equation 1, and the corresponding results are shown in Tables 11 to 13.

[Equation 1]

$$\% \text{ Hemolysis} = (A-C)/(B-C) \times 100$$

In Equation 1, A is the absorbance of the peptoid solution at 540 nm, B is the absorbance of the 0.1% Triton X-100 at 540 nm, and C is the absorbance of the PBS solution at 540 nm.

In Tables 11 to 13 below, $HD_{10}$ indicates the concentration of a peptoid that induces hemolysis in 10% red blood cells, and $HD_{50}$ indicates the concentration of a peptoid that induces hemolysis in 50% red blood cells. In addition, $H_{max}$ is the hemolysis rate (%) at 200 μM, which is the maximum concentration of a peptoid used in the experiment. The term "nd" in the following Tables 11 to 13 is the abbreviation for not determined, and indicates a value that is too small or too large to be measured.

<Analysis Example 5: Analysis of selectivity of peptoid>

The term "selectivity" used herein refers to a comparative value for antimicrobial activity and mammalian cytotoxicity, and the higher the selectivity value, the lower the toxicity. To confirm peptoid selectivity, the selectivity of each peptoid was calculated using the results measured in Experimental Examples 1 and 2. For selectivity, values calculated by dividing $HD_{10}$ values by MIC against *E. coli* were shown in Tables 11 to 13 below. All values were determined by the average value of the numbers obtained through three independent experiments, and an experimental error was less than 10%.

Referring to Tables 11 to 13 below, the highest selectivity was shown in Peptoids 29 and 32. Accordingly, among Peptoids 1 to 66, Peptoids 29 and 32 were able to be selected as peptoids with high antimicrobial activity and low red blood cell toxicity.

[Table 11]

| Peptoid | MIC (μM) | | $HC_{10}$/ $HC_{50}$ (μM) | Hmax (100 μM) | Selectivity [$HD_{10}$/MIC (*E. coli*)] |
|---|---|---|---|---|---|
| | *E. coli* | *S. aureus* | | | |
| | ATCC 25922 | ATCC 25923 | | | |
| 1 | 6.3 | 1.6 | 8.3/22.9 | 112.3±0.7 | 1.3 |
| 2 | 12.5 | 3.1 | 20.4/>100.0 | 17.7±1.1 | 1.6 |
| 3 | 6.3 | 1.6 | 6.3/13.9 | 100.5±9.9 | 1 |
| 4 | 12.5 | 1.6 | <6.3/6.7 | 103.2±0.9 | <0. 5 |
| 5 | >25 | 3.1 | <6.3/15.3 | 108.1±2.7 | <0. 3 |
| 6 | 25 | <0.8 | 9.6/22.0 | 101.6±5.8 | 0.4 |
| 7 | 6.3 | 3.1 | 7.8/23.8 | 94.1±4.3 | 1.2 |
| 8 | 6.3 | 1.6 | 128/28.4 | 105.1±2.2 | 2 |
| 9 | 3.1 | 1.6 | 10.6/31.9 | 106.4±0.6 | 3.4 |
| 10 | 3.1 | 1.6 | 6.6/17.0 | 108.1±1.7 | 2.1 |
| 11 | 6.3 | 1.6 | 8.3/22.2 | 103.4±3.2 | 1.3 |
| 12 | 6.3 | 1.6 | 29.1/65.2 | 87.4±2.4 | 4.6 |
| 13 | 6.3 | <0.8 | 20.2/41.3 | 96.8±1.6 | 3.2 |
| 14 | 3.1 | <0.8 | 23.6/43.4 | 90.0±7.3 | 7.6 |
| 15 | 6.3 | 1.6 | 28.1/60.9 | 91.0±2.5 | 4.5 |
| 16 | 6.3 | 1.6 | 13.8/25.0 | 100.3±1.7 | 2.2 |
| 17 | 6.3 | 1.6 | 18.0/39.7 | 87.3±6.7 | 2.9 |
| 18 | 12.5 | 3.1 | 14.3/37.8 | 103.5±4.3 | 1.1 |
| 19 | 12.5 | 0.8 | 3.8/12.5 | 101.5±1.3 | 0.3 |
| 20 | 6.3 | 1.6 | 8.2/33.9 | 106.7±3.5 | 1.3 |
| 21 | 6.3 | 1.6 | 23.2/64.9 | 83.7±2.0 | 3.7 |
| 22 | 12.5 | 1.6 | 6.9/19.4 | 108.6±1.1 | 0.6 |
| 23 | 25 | 1.6 | 17.1/45.2 | 72.2±0.3 | 0.7 |

[Table 12]

| Peptoid | MIC (μM) | | $HC_{10}$/ $HC_{50}$ (μM) | $H_{max}$ (100 μM) | Selectivity [$HD_{10}$/MIC (*E. coli*)] |
|---|---|---|---|---|---|
| | *E. coli* | *S. aureus* | | | |
| | ATCC 25922 | ATCC 25923 | | | |
| 24 | >25 | 5.3 | >100/>100 | 1.5±0.0 | nd |
| 25 | >25 | 3.1 | >100/>100 | 2.7±0.4 | nd |
| 26 | >25 | 5.3 | >100/>100 | 0.7±0.1 | nd |
| 27 | 25 | 3.1 | >100/>100 | 5.9±0.2 | nd |
| 28 | 12.5 | 6.3 | >100/>100 | 8.9±1.4 | >8.0 |
| 29 | 6.3 | 5.3 | >100/>100 | 9.8±1.8 | >15.9 |
| 30 | 12.5 | 3.1 | >100/>100 | 8.4±0.5 | >8.0 |
| 31 | 6.3 | 3.1 | 59.0/>100 | 18.4±5.0 | 9.3 |
| 32 | 6.3 | 3.1 | 77.7/>100 | 15.9±1.5 | 12.3 |
| 33 | 12.5 | 3.1 | >100/>100 | 5.0±0.2 | >8.0 |
| 34 | 25 | 3.1 | >100/>100 | 8.5±1.1 | 4 |
| 35 | 3.1 | 0.8 | 20.4/63.0 | 82.6±3.2 | 6.6 |
| 36 | 3.1 | <0.8 | 15.3/39.4 | 89.1±3.4 | 4.9 |
| 37 | 3.1 | 1.6 | 21.1/57.0 | 97.4±6.5 | 6.8 |
| 38 | 12.5 | 6.3 | >1001>100 | 5.9±0.5 | >8.0 |
| 39 | 25 | 6.3 | 100/>100 | 10.2±0.3 | 4 |
| 40 | 12.5 | 12.5 | >100/>100 | 8.9±0.2 | >8.0 |
| 41 | 25 | 6.3 | >1001>100 | 7.1±0.1 | >4.0 |
| 42 | 25 | 0.8 | <3.1/6.6 | 105.9±1.1 | <0.1 |
| 43 | 12.5 | 0.8 | <3.1/6.4 | 105.6±3.8 | <0.2 |
| 44 | 6.3 | 1.6 | 6.3/28.6 | 97.9±3.7 | 1 |
| 45 | >25 | 25 | >100/>100 | 4.5±0.2 | nd |
| 46 | 6.3 | 0.8 | 6.9/36.5 | 90.8±2.7 | 1.1 |
| 47 | 25 | 12.5 | >100/>100 | 4.8±0.2 | >4.0 |
| 48 | 12.5 | 3.1 | 25.7/48.4 | 79.9±3.6 | 2.1 |

[Table 13]

| Peptoid | MIC (μM) | | $HC_{10}$/$HC_{50}$(μM) | Hmax (100 μM) | Selectivity [$HD_{10}$/MIC (*E. coli*)] |
|---|---|---|---|---|---|
| | *E. coli* | *S. aureus* | | | |
| | ATCC 25922 | ATCC 25923 | | | |
| 49 | >25 | >25 | >100/>100 | 0.3±0.3 | nd |
| 50 | >25 | >25 | Nd | nd | nd |
| 51 | >25 | >25 | Nd | nd | nd |
| 52 | 25 | >25 | Nd | nd | nd |
| 53 | 25 | >25 | Nd | nd | nd |
| 54 | 25 | >25 | Nd | nd | nd |

(continued)

| Peptoid | MIC (μM) | | $HC_{10}/HC_{50}$ (μM) | Hmax (100 μM) | Selectivity [$HD_{10}$/MIC (*E. coli*)] |
|---|---|---|---|---|---|
| | *E. coli* | *S. aureus* | | | |
| | ATCC 25922 | ATCC 25923 | | | |
| 55 | 25 | >25 | Nd | nd | nd |
| 56 | 25 | 25 | Nd | nd | nd |
| 57 | 25 | 12.5 | Nd | nd | nd |
| 58 | 25 | 12.5 | Nd | nd | nd |
| 59 | >25 | 25 | Nd | nd | nd |
| 60 | >25 | 12.5 | Nd | nd | nd |
| 61 | 6.3 | 3.1 | 19.3/65.4 | 79.9±0.7 | 3.1 |
| 62 | 6.3 | 3.1 | 36.2/>100 | 44.8±8.1 | 57 |
| 63 | 12.5 | 3.1 | 85.0/>100 | 12.7±0.9 | 6.8 |
| 64 | 12.5 | 3.1 | 70.7/>100 | 18.9±2.9 | 57 |
| 65 | >25 | >25 | Nd | nd | nd |
| 66 | >25 | >25 | Nd | nd | nd |

<Analysis Example 6: Confirmation of antimicrobial activity of peptoid in multidrug-resistant bacteria>

The MIC of the prepared peptoid in multidrug-resistant bacteria was measured using a modified microdilution method in a Difco Mueller Hinton medium, and a test was performed on a bacterial pathogen panel with resistant and tolerance to general antibiotics.

Briefly, a medium containing bovine serum albumin and 0.001% acetic acid was used to continuously dilute a peptoid concentrate 10-times stronger than the final concentration. 10 μL of the 10-time stronger peptoid concentrate and 90 μL of a Muleller Hinton medium were added to each well of a 96-well plate. Bacteria were added to the plate until the final concentration reached $2 \times 10^5$ CFU/mL and then incubated at 37 °C overnight. MIC was defined as a concentration at which bacterial growth is not observed.

In the following Table 14, the antimicrobial activity of a peptoid against multidrug-resistant bacteria is shown. Similar to the antimicrobial activities against the gram-negative bacteria, *E. coli* ATCC 25922, and the gram-positive bacteria, *S. aureus* ATCC 25923, Peptoid 1 showed a relatively low MIC against multidrug-resistant bacteria. The antimicrobial peptoid of the present invention showed an MIC against gram-negative bacteria, such as *Enterobacter SP* KCTC 2625 and *E. coli* KCTC 2411, which is similar to that of Peptoid 1. Meanwhile, for the gram-positive bacteria, *Enterococcus Faecium* KCTC 13225 and *Enterococcus faecalis* KCTC 3511, a relatively high MIC was confirmed in the peptoid of the present invention, compared to Peptoid 1.

[Table 14]

| Peptoid | *Enterobacter SP* | *Enterococcus Faecium* | *Enterococcus faecalis* | *E. coli* | *P. aeruginosa* |
|---|---|---|---|---|---|
| Peptoid | KCTC 2625 | KCTC 13225 | KCTC 3511 | KCTC 2411 | KCTC 1637 |
| 1 | 6.3 | <0.8 | 0.8 | 6.3 | 25 |
| 11 | 6.3 | <0.8 | <0.8 | 12.5 | - |
| 14 | 3.1 | <0.8 | 1.6 | 6.3 | - |
| 29 | 3.1 | 1.6 | 3.1 | 6.3 | 50 |
| 32 | 3.1 | 3.1 | 5.3 | 6.3 | 25 |

<Analysis Example 7: Analysis of antimicrobial activity against *E. coli* according to number and substitution position of NhTrp>

FIGS. 5 and 6 are graphs that analyze the antimicrobial activity against *E. coli* according to the number and substitution position of *N*hTrp of peptoids according to one embodiment of the present invention.

Referring to FIG. 5, for Peptoids 7 to 23 prepared according to one embodiment of the present invention, MIC values against *E. coli* according to the number of substitutions of NhTrp of the peptoids are shown. Accordingly, the tendency to have a relatively low MIC value was confirmed in a peptoid with a small number of *N*hTrp substitutions. In addition, referring to FIG. 6, for Peptoids 1 to 41 prepared according to an embodiment of the present invention, MIC values for each *N*hTrp substitution position of the peptoids are shown. According to this, it was confirmed that a peptoid in which *N*hTrp is substituted at the N-terminus position, rather than the C-terminus position, tends to have a relatively lower MIC value. Accordingly, it can be seen that a novel peptoid in which *N*hTrp is less substituted at the N-terminus position of the present invention exhibits effective antimicrobial activity.

<Analysis Example 8: Analysis of correlation between HPLC elution and antimicrobial activity and red blood cell toxicity>

FIGS. 7 and 8 are graphs that analyze the relationship between the HPLC elution, and antimicrobial activity and red blood cell toxicity of a peptoid according to an embodiment of the present invention.

Referring to FIGS. 7 and 8, the antimicrobial activity against *E. coli* was not significantly correlated with hydrophobicity. However, in the results for red blood cell toxicity, an $HC_{50}$ value tended to decrease as the HPLC elution amount of ACN increased. Accordingly, it can be seen that, in the case of a peptoid with an increased cationic charge although having a hydrophobic residue, the lower the HPLC elution of the peptoid, the lower the cytotoxicity.

<Analysis Example 9: HPLC and LC-MS analysis>

FIGS. 9 to 76 are graphs showing the HPLC and LC-MS analysis results for Peptoids 1 to 66 according to Example 1 of the present invention.

Referring to FIGS. 9 to 76, in high performance liquid chromatography (HPLC) analysis, the hydrophobicity of the peptoids may be compared according to the type of monomer. When comparing the graphs of Peptoids 1 and 2, it was confirmed that, hydrophobicity is reduced as the number of carbon atoms was reduced according to the change from *N*spe to *N*pm, the retention time (RT) slightly decreased (17.46 $\rightarrow$ 16.43 min).

In addition, it was confirmed that the retention time (RT) of each of Peptoids 3 to 6 in which one *N*hTrp monomer is added to the structure of Peptoid 1 increases, compared to Peptoid 1. In addition, it was confirmed that, compared to Peptoid 1, Peptoids 7 to 23 in which *N*spe is substituted with one to four *N*hTrps had changes in retention time (RT) in the range of -0.4 min to +0.6 min. In addition, for Peptoids 24 to 38, compared to Peptoids 9, 11 and 14 in which one Nspe is substituted with *N*Lys as a control, the retention time (RT) was reduced by approximately 1.5 min, and it was determined that this was a result of reducing hydrophobicity due to the removal of Nspe and the addition of *N*Lys.

In addition, comparing Peptoids 48 to 64, the degree of hydrophobicity of each peptoid can be determined according to a monomer at the N-terminal position. Specifically, comparing Peptoid 48 with Peptoids 53 to 56, it was shown that, when *N*spe is substituted with *N*Lys, hydrophobicity was reduced. In addition, comparing Peptoids 49 to 52 with Peptoids 53 to 56, it was confirmed that the retention time (RT) was reduced after removing *N*spe from the N-terminus. In Peptoids 57 to 60 in which *N*hTrp is substituted at the N-terminus, it was confirmed that the retention time (RT) is increased by 0.2 min compared to that of each *N*spe control compound. In addition, in Peptoids 61 to 64, it was confirmed that as *N*hTrp was added to the N-terminus of each of Peptoids 53 to 56, compared to the control compound, the retention time (RT) was increased by approximately 0.8 min. In the case of Peptoids 65 and 66, as all of the amine groups of *N*Lys are substituted with hydroxyl groups, the retention time (RT) was greatly increased by approximately 5 min, compared to a peptoid with an amine group, and Peptoids 65 and 66 were not dissolved in water due to a higher hydrophobicity than other peptoids.

<Analysis Example 10: Electrospray ionization-mass spectrometry>

Electrospray ionization-mass spectrometry (ESI-MS) was performed on the antimicrobial peptoids of the present invention, and the calculation and analysis results are shown in Tables 15 to 17 below. The term "nd" shown in Tables 15 to 17 below is the abbreviation for not determined, and indicates a value that is too small or too large to be measured.

[Table 15]

| Peptoid | Calculated mass | | Observed mass | |
|---|---|---|---|---|
| | [M+H]+ | [M+2H]2+ | [M+H]+ | [M+2H]2+ |
| 1 | 1819.1 | 910.1 | 1819.9 | 910.5 |
| 2 | 1707 | 854 | 1707.8 | 854.2 |
| 3 | 2019.2 | 1010.1 | nd | 1010.2 |
| 4 | 2019.2 | 1010.1 | nd | 1010.8 |
| 5 | 2219.3 | 1110.2 | nd | 1110.8 |
| 6 | 2219.3 | 1110.2 | nd | 1110.7 |
| 7 | 1858.1 | 929.6 | 1858.9 | 930 |
| 8 | 1858.1 | 929.6 | 1858.9 | 930 |
| 9 | 1858.1 | 929.6 | 1858.9 | 929.6 |
| 10 | 1858.1 | 929.6 | 1858.9 | 930 |
| 11 | 1897.1 | 949.1 | 1897.9 | 949.1 |
| 12 | 1897.1 | 949.1 | 1898 | 949.2 |
| 13 | 1897.1 | 949.1 | 1898 | 949.2 |
| 14 | 1897.1 | 949.1 | 1897.9 | 949.2 |
| 15 | 1897.1 | 949.1 | 1898 | 949.6 |
| 16 | 1897.1 | 949.1 | 1898 | 949.3 |
| 17 | 1897.1 | 949.1 | 1897.9 | 949.2 |
| 18 | 1975.1 | 988.1 | 1976 | 988.5 |
| 19 | 1975.1 | 988.1 | 1976 | 988.5 |
| 20 | 1975.1 | 988.1 | 1976 | 988.6 |
| 21 | 1975.1 | 988.1 | 1976 | 988.7 |
| 22 | 1975.1 | 988.1 | 1976 | 988.2 |
| 23 | 2131.2 | 1066.1 | nd | 1066.6 |

[Table 16]

| Peptoid | Calculated mass | | Observed mass | |
|---|---|---|---|---|
| | [M+H]+ | [M+2H]2+ | [M+H]+ | [M+2H]2+ |
| 24 | 1825.1 | 913.1 | 1826 | 913.2 |
| 25 | 1825.1 | 913.1 | 1825.9 | 913.6 |
| 26 | 1825.1 | 913.1 | 1825.9 | 913.2 |
| 27 | 1825.1 | 913.1 | 1826 | 913.2 |
| 28 | 1864.1 | 932.6 | 1864.9 | 932.6 |
| 29 | 1864.1 | 932.6 | 1865 | 932.7 |
| 30 | 1864.1 | 932.6 | 1865 | 933 |
| 31 | 1864.1 | 932.6 | 1865 | 933.1 |
| 32 | 1864.1 | 932.6 | 1864.9 | 933 |

(continued)

| Peptoid | Calculated mass | | Observed mass | |
|---|---|---|---|---|
| | $[M+H]^+$ | $[M+2H]^{2+}$ | $[M+H]^+$ | $[M+2H]^{2+}$ |
| 33 | 1864.1 | 932.6 | 1864.9 | 932.7 |
| 34 | 1864.1 | 932.6 | 1865 | 932.7 |
| 35 | 2186.3 | 1093.7 | nd | 1094.2 |
| 36 | 2186.3 | 1093.7 | nd | 1094.2 |
| 37 | 1897.1 | 949.1 | 1897.9 | 949.2 |
| 38 | 1864.1 | 932.6 | 1864.9 | 933.1 |
| 39 | 1850.1 | 925.6 | 1851 | 925.7 |
| 40 | 1836.1 | 918.6 | 1836.9 | 919 |
| 41 | 1836.1 | 918.6 | 1836.9 | 919.1 |
| 42 | 2113.2 | 1057.1 | nd | 1057.3 |
| 43 | 2113.2 | 1057.1 | nd | 1057.3 |
| 44 | 1791.1 | 896.1 | 1791.9 | 896.6 |
| 45 | 1758.1 | 879.6 | 1758.9 | 879.7 |
| 46 | 1791.1 | 896.1 | 1791.9 | 896.6 |
| 47 | 1758.1 | 879.6 | 1758.9 | 880.1 |
| 48 | 1819.1 | 910.1 | 1818.9 | 910.1 |

[Table 17]

| Peptoid | Calculated mass | | Observed mass | |
|---|---|---|---|---|
| | $[M+H]^+$ | $[M+2H]^{2+}$ | $[M+H]^+$ | $[M+2H]^{2+}$ |
| 49 | 1625 | 813 | 1624.8 | 813.1 |
| 50 | 1625 | 813 | 1625.8 | 813.1 |
| 51 | 1625 | 813 | 1625.8 | 813.2 |
| 52 | 1625 | 813 | 1625.8 | 813.5 |
| 53 | 1786.1 | 893.6 | 1786.9 | 893.6 |
| 54 | 1786.1 | 893.6 | 1786.9 | 893.6 |
| 55 | 1786.1 | 893.6 | 1786.9 | 893.6 |
| 56 | 1786.1 | 893.6 | 1786.9 | 893.6 |
| 57 | 1825.1 | 913.1 | 1826 | 913.2 |
| 58 | 1825.1 | 913.1 | 1825.9 | 913.2 |
| 59 | 1825.1 | 913.1 | 1825.9 | 913.6 |
| 60 | 1825.1 | 913.1 | 1825.9 | 913.2 |
| 61 | 1986.2 | 993.6 | 1987 | 993.7 |
| 62 | 1986.2 | 993.6 | 1987 | 993.8 |
| 63 | 1986.2 | 993.6 | 1987 | 993.7 |
| 64 | 1986.2 | 993.6 | 1987 | 994 |

(continued)

| Peptoid | Calculated mass | | Observed mass | |
|---|---|---|---|---|
| | $[M+H]^+$ | $[M+2H]^{2+}$ | $[M+H]^+$ | $[M+2H]^{2+}$ |
| 65 | 2135.1 | 1068.1 | nd | 1068.3 |
| 66 | 1869 | 935 | nd | 935.4 |

Electrospray ionization (ESI) is a method of analyzing the weight of a sample by forming a charged droplet by ionization while an organic sample is sprayed in an electric field and detecting the charged sample molecule by mass spectrometry, and is used particularly for a biomolecule with a high molecular weight and an active nonvolatile compound. In Tables 15 to 17, $[M+H]^+$ indicates a value (m/z) obtained by dividing the measured mass of an ionized sample molecule to which one proton is attached by an electric charge, and $[M+2H]^{2+}$ indicates a value (m/z) obtained by dividing the measured mass of an ionized sample molecule to which two protons are attached by an electric charge. Each peptoid sample was prepared by being dissolved at a concentration of 10 $\mu$M in a solvent in which water and acetonitrile were mixed at 1:1 (v/v). Referring to Tables 15 to 17, the result of analyzing each peptoid sample revealed that a calculated mass is the same as an observed mass, thereby confirming that a peptoid with a predicted structure and a peptoid with an actual structure are manufactured in the same manner.

**II. Confirmation of change in activity of peptoid according to counter ion substitution**

Preparation of counter ion-substituted peptoid

The method of preparing an antimicrobial composition of the present invention may further include, after S500 described above, a counter ion substitution process (S600) of removing trifluoroacetate (TFA) ions included in a composition using a carbonate ion exchange resin and adding an acidic solution.

The acidic solution may be acetic acid or hydrochloric acid.

Similar to a peptide, a peptoid is synthesized through the above-described solid-phase synthesis, in which trifluoroacetate (TFA) is included in a cleavage solution in the cleavage and removal of a protecting group such as an Fmoc or tert-butyl group. In addition, TFA is used as a mobile phase additive for ion pairing with amino groups of basic side chains and the N-terminus in reverse-phase high performance liquid chromatography (RP-HPLC). However, the presence of the TFA ion in the peptoid compound may change the behavior or shape of the peptoid, degrading analysis accuracy. In the present invention, to improve the selectivity of a peptoid, counter ion substitution was performed.

<Example 2: Preparation of counter ion-substituted peptoid>

For Peptoids 1, 10, 11, 14, 15, 29, 32 and 37 selected from among Peptoids 1 to 66 prepared in Example 1, TFA ions were removed using a carbonate ion exchange resin (VariPure columns, USA). Each of the TFA ion-removed peptoids was dissolved in an $H_2O$/ACN solution, and the resulting solution was immediately used as a HPLC eluent. The resin was adjusted with 2 to 3 bed volumes of methanol and 2 to 3 bed volumes of the mobile phase. and washed with 1 bed volume of methanol to allow the peptoid to be completely collected. Afterward, a desired fraction and a separated peptoid were combined using rotary evaporation. Subsequently, diluted acetic acid (AcOH) or hydrochloric acid (HCl) was added, and the eluent sample was dried.

<Analysis Example 11: $^{19}$F-NMR analysis for confirming TFA ion removal after counter ion substitution>

FIG. 75 is a graph showing $^{19}$F-NMR quantitative analysis for Peptoid 1 to measure a residual fluorine amount resulting from TFA ions after counter ion substitution.

Referring to FIG. 75, it was confirmed that 98.83% and 99.18% of TFA were removed from peptoid compounds substituted with a chloride and an acetate, respectively.

<Analysis Example 12: HPLC to confirm peptoid decomposition after counter ion substitution>

FIGS. 76 to 83 are the HPLC results for Peptoids 1, 10, 11, 14, 15, 29, 32 and 37 of the present invention to confirm the decomposition of the peptoids after counter ion substitution using (a) HCl and (b) AcOH.

Referring to FIGS. 78 to 83, HPLC was performed for Peptoids 1, 10, 11, 14, 15, 29, 32 and 37 of the present invention. Since the chloride (HCl) is a stronger acid than TFA, it can decompose acid-vulnerable peptoid compounds.

However, the HPLC result did not show the peak of an acid used in counter ion substitution at 220 nm, confirming that almost no peptoid decomposition occurred even after counter ion substitution.

<Analysis Example 13: Confirmation of antimicrobial activity and hemolytic activity of counter ion-substituted peptoids and analysis of selectivity thereof>

In Table 18 below, the confirmation of antimicrobial activity and hemolytic activity of a counter ion-substituted peptoid of the present invention against red blood cells and the analysis of the selectivity thereof are summarized.

Referring to Table 18, as the result of counter ion substitution, in Peptoids 10, 15 and 37, antimicrobial activity was reduced, but the antimicrobial activity was maintained in the other peptoids. That is, it can be seen that most of the peptoids of the present invention tend to show the same antimicrobial activity as before substitution even after counter ion substitution. In addition, it is confirmed that, compared to a TFA salt-containing peptoid, the peptoids that are subjected to counter ion substitution with HCl and AcOH salts show overall low cytotoxicity. Particularly, it can be confirmed that Peptoid 29-AcOH and Peptoid 32-HCl have the maximum selectivity for red blood cells.

[Table 18]

| Classification | Compound | MIC (μM) | | $HC_{10}/HC_{50}$ (μM) | $H_{max}$ (100μM) | Selectivity |
|---|---|---|---|---|---|---|
| | | *E. coli* ATCC25922 | *S. aureus* ATCC25923 | | | |
| Peptoid 1 | 1 | 6.3 | 1.6 | 15.9/49.0 | 72.1±2.1 | 2.5 |
| Peptoid 1-1 | 1-HCl | 6.3 | 1.6 | 27.6/83.3 | 61.5±0.9 | 4.4 |
| Peptoid 1-2 | 1-AcOH | 12.5 | 3.1 | 25.0/78.6 | 58.2±1.7 | 2 |
| Peptoid 10 | 10 | 3.1 | 0.8 | 6.9/25.8 | 101.4±5.1 | 2.2 |
| Peptoid 10-1 | 10-HCl | 6.3 | 3.1 | 15.3/37.8 | 81.5±7.4 | 2.4 |
| Peptoid 10-2 | 10-AcOH | 6.3 | 1.6 | 14.1/29.4 | 102.3±11.3 | 2.2 |
| Peptoid 11 | 11 | 3.1 | 1.6 | 5.9/27.5 | 111.9±2.7 | 2.2 |
| Peptoid 11-1 | 11-HCl | 3.1 | 3.1 | 10.1/21.5 | 113.9±1.7 | 3.3 |
| Peptoid 11-2 | 11-AcOH | 3.1 | 1.6 | 8.7/29.7 | 110.4±0.2 | 2.8 |
| Peptoid 14 | 14 | 3.1 | 1.6 | 12.5/21.1 | 112.9±2.1 | 4 |
| Peptoid 14-1 | 14-HCl | 3.1 | 3.1 | 19.2/68.2 | 75.3±5.0 | 6.2 |
| Peptoid 14-2 | 14-AcOH | 3.1 | 1.6 | 14.9/51.8 | 100.7±17.2 | 4.8 |
| Peptoid 15 | 15 | 3.1 | 1.6 | 20.5/89.2 | 53.4±3.1 | 6.6 |
| Peptoid 15-1 | 15-HCl | 6.3 | 3.1 | 50.4/>100 | 33.4±9.7 | 8 |
| Peptoid 15-2 | 15-AcOH | 6.3 | 3.1 | 39.2/>100 | 34.1±1.1 | 6.2 |
| Peptoid 29 | 29 | 6.3 | 12.5 | 88.6/>100 | 12.3±1.3 | 14.1 |
| Peptoid 29-1 | 29-HCl | 12.5 | 12.5 | >100/>100 | 4.3±0.3 | >8.0 |
| **Peptoid 29-2** | **29-AcOH** | 6.3 | 12.5 | >100/>100 | 5.2±0.2 | >15.9 |
| Peptoid 32 | 32 | 6.3 | 3.1 | >100/>100 | 4.2±1.0 | >15.9 |
| **Peptoid 32-1** | **32-HCl** | 6.3 | 6.3 | >100/>100 | 8.7±0.6 | >15.9 |
| Peptoid 32-2 | 32-AcOH | 6.3 | 6.3 | 83.0/>100 | 13.8±1.1 | 13.2 |
| Peptoid 37 | 37 | 3.1 | 1.6 | 13.2/38.6 | 80.7±12.8 | 4.3 |
| Peptoid 37-1 | 37-HCl | 6.3 | 3.1 | 14.4/56.5 | 86.6±5.7 | 2.3 |
| Peptoid 37-2 | 37-AcOH | 6.3 | 1.6 | 12.5/26.0 | 98.9±8.0 | 2 |

<Analysis Example 14: Confirmation of cytotoxicity of counter ion-substituted peptoid>

Human lung cells (MRC-5) were purchased from the Korean Cell Line Bank, and human keratinocytes (HaCaT) were purchased from the AmorePacific R&D Center. All cells were grown in a Dulbecco's modified Eagle's medium containing 10 % fetal bovine serum under conditions of 5% $CO_2$ and 37 °C. To confirm the effect of a peptoid on cell growth, 3-(4,5-dimethylthiazol-2-yl)-5-(3-carboxymethoxyphenyl)-2-(4-sulfophenyl)-2H-tetrazolium (MTS) cell assay was performed to determine cell viability. An aliquot (100 μL) of a batch including $1.3\times10^4$ MRC-5 cells/mL or $3\times10^4$ HaCaT cells/mL was dispensed into each well of a 96-well plate. The cells were grown at 37 °C in a 5% $CO_2$ humid atmosphere and allowed to adhere for 24 hours. When the cells reached approximately 70% confluency, the medium was replaced with serial dilutions of the peptoid stock and incubated for 24 hours. 20 μL of a CellTiter 96 aqueous non-radioactive cell proliferation assay reagent (Promega, USA) including a tetrazolium compound was added to each well.

The contents in the plate were metabolized by incubating at 37 °C for 4 hours. An MTS-formazan product of viable cells was measured at 490 nm using a microplate reader, and cell viability (%) was calculated by Equation 2 below and compared with untreated cells.

[Equation 2]

$$\text{Cell viability (\%)} = [A/(A_{control})] \times 100$$

In Equation 2, A is the absorbance of a test well, and $A_{control}$ is the average absorbance of a well containing untreated cells.

The abbreviation "$LC_{50}$" shown in Table 19 below is lethal dose 50, indicating cell viability, and is defined as the concentration of a compound that causes 50% cell lethality. Particularly, Peptoids 29 and 32 showing high selectivity for red blood cells showed 20% lower toxicity against MRC-5 and HaCaT cells compared to the peptoid having a TFA salt after counter ion substitution. Accordingly, as representative peptoid compounds, Peptoid 29-2 and Peptoid 32-1 were selected. Particularly, Peptoid 32-1, which has high activity against gram-negative bacteria and high selectivity for red blood cell hemolysis, exhibited lower animal cell toxicity than Peptoid 29-2. The term "nd" shown in Table 19 below is the abbreviation for not determined, and indicates a value that is too small or too large to be measured.

[Table 19]

| Classification | Compound | $LC_{50}$(μM) | |
|---|---|---|---|
| | | MRC-5 | HaCaT |
| Peptoid 1 | 1 | 8.1 | 7.3 |
| Peptoid 1-1 | 1-HCl | 11.8 | nd |
| Peptoid 1-2 | 1-AcOH | 8.6 | nd |
| Peptoid 10 | 10 | nd | nd |
| Peptoid 10-1 | 10-HCl | nd | nd |
| Peptoid 10-2 | 10-AcOH | nd | nd |
| Peptoid 11 | 11 | 6.9 | nd |
| Peptoid 11-1 | 11-HCl | 9.6 | nd |
| Peptoid 11-2 | 11-AcOH | 8.3 | nd |
| Peptoid 14 | 14 | 9.7 | nd |
| Peptoid 14-1 | 14-HCl | 12.1 | nd |
| Peptoid 14-2 | 14-AcOH | 13.8 | nd |
| Peptoid 15 | 15 | nd | nd |
| Peptoid 15-1 | 15-HCl | nd | nd |
| Peptoid 15-2 | 15-AcOH | nd | nd |
| Peptoid 29 | 29 | 11.8 | 18.8 |

(continued)

| Classification | Compound | $LC_{50}$(μM) | |
|---|---|---|---|
| | | MRC-5 | HaCaT |
| Peptoid 29-1 | 29-HCl | 23.9 | >25.0 |
| **Peptoid 29-2** | **29-AcOH** | 16 | 22 |
| Peptoid 32 | 32 | 14.5 | 17.1 |
| **Peptoid 32-1** | **32-HCl** | 18.1 | 21.4 |
| Peptoid 32-2 | 32-AcOH | 16.9 | 21.3 |
| Peptoid 37 | 37 | nd | nd |
| Peptoid 37-1 | 37-HCl | nd | nd |
| Peptoid 37-2 | 37-AcOH | nd | nd |

FIG. 84 is a graph showing the rate of change in the $LC_{50}$ value for MRC-5 cells of a counter ion-substituted peptoid according to one embodiment of the present invention, and FIG. 85 shows the rate of change in the $LC_{50}$ value for HaCaT cells.

The rate of change was calculated using Equation 3 below.

[Equation 3]

$$\text{Rate of change} = (D-E)/D$$

In Equation 3, D indicates the $L_{50}$ value of the TFA salt, and E indicates the $L_{50}$ value of an exchanged salt (HCl or AcOH).

Referring to FIGS. 84 and 85, comparing Peptoids 29-1 (29-HCl), 29-2 (29-AcOH), 32-1 (32-HCl) and 32-2 (32-AcOH), it was confirmed that, through counter ion substitution, for both MRC-5 and HaCaT cells, the $LC_{50}$ values of the peptoids increase. Accordingly, it was confirmed that the cytotoxicity of antimicrobial peptoids can be effectively reduced through counter ion substitution. In addition, except for Peptoid 29-HCl in which a counter ion is changed into a chloride, as the MIC against *E. coli* was constantly maintained at 6.3 μM, it can be seen that the effect of antimicrobial activity was maintained regardless whether the counter ion substitution was performed or not. As a result, the peptoids containing NhTrp can maintain the effect of antimicrobial activity and can reduce cytotoxicity through the counter ion substitution.

**III. Confirmation of change in peptoid activity according to *N*His substitution**

<Example 3: Preparation of Peptoids 67 to 81>

Antimicrobial peptoids were prepared in the same manner as the method of preparing a peptoid described in Example 1, except that *N*hTrp(Boc), *N*Lys(Boc), *N*4hb(TIPS), *N*pm and *N*spe were used as submonomers and *N*His(Trt) was additionally used.

The chemical structures of Peptoids 67 to 81 according to Example 3 of the present invention are shown in Table 20 below.

[Table 20]

| Chemical structure | |
|---|---|
| Peptoid 67 | MK-3-40 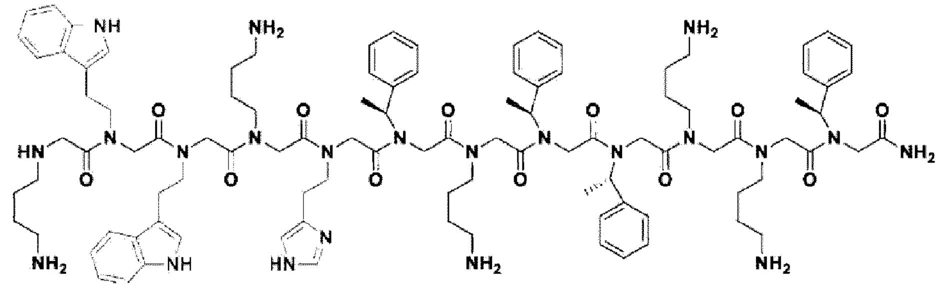 |
| Peptoid 68 | MK-3-41 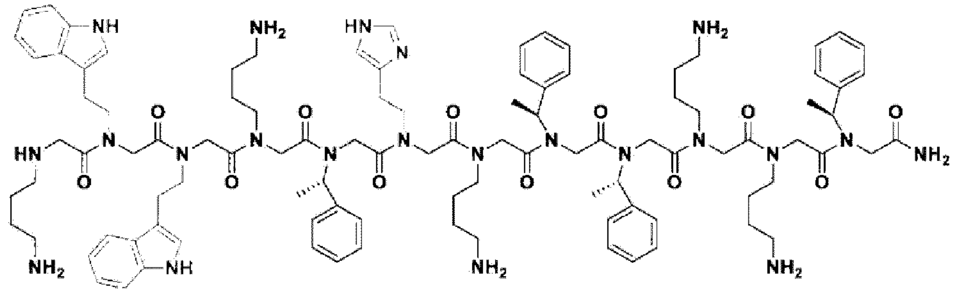 |
| Peptoid 69 | MK-3-42 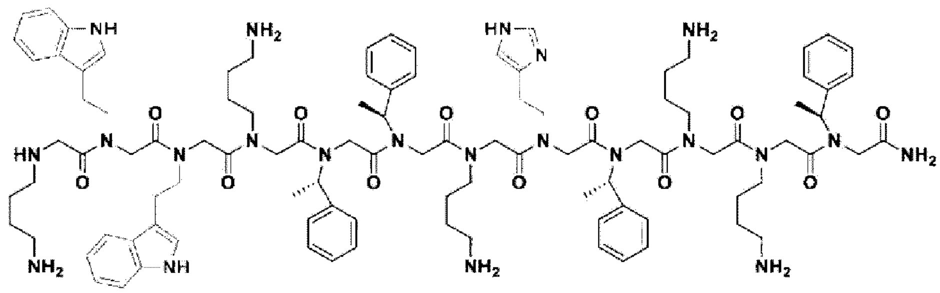 |
| Peptoid 70 | MK-3-43 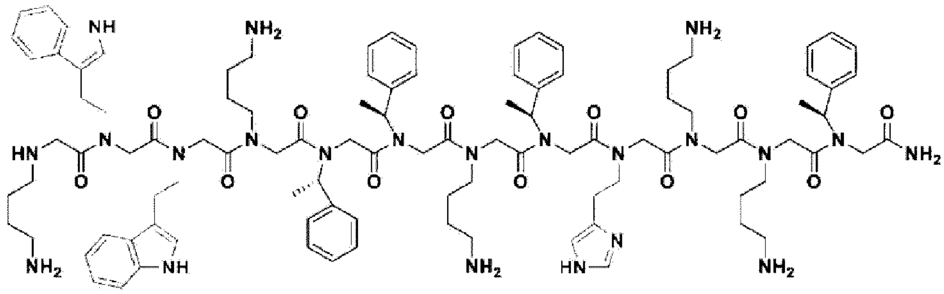 |

(continued)

| Chemical structure | |
|---|---|
| Peptoid 71 | MK-3-44 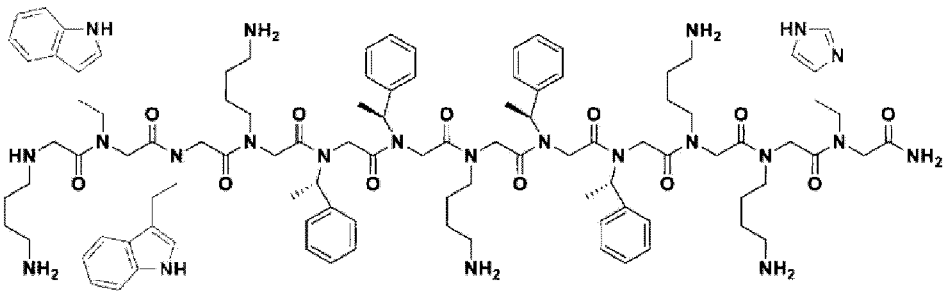 |
| Peptoid 72 | MK-3-61 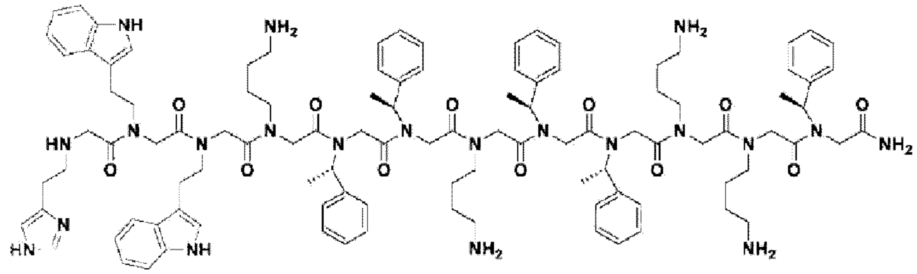 |
| Peptoid 73 | MK-3-62 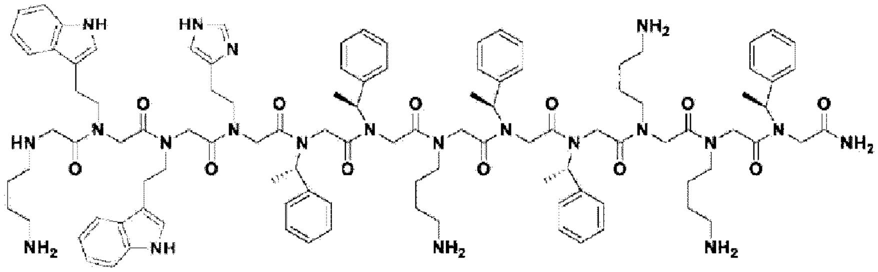 |
| Peptoid 74 | MK-3-63 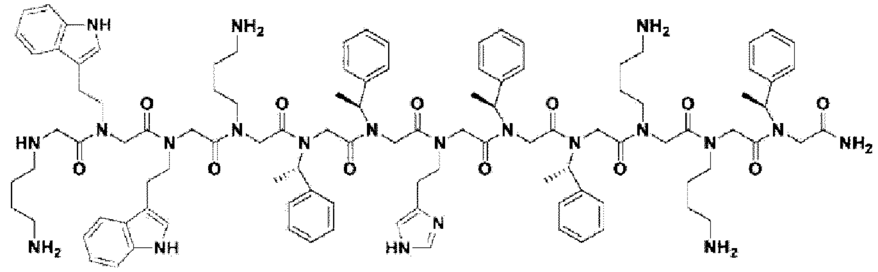 |

(continued)

| Chemical structure | |
|---|---|
| Peptoid 75 | MK-3-64<br>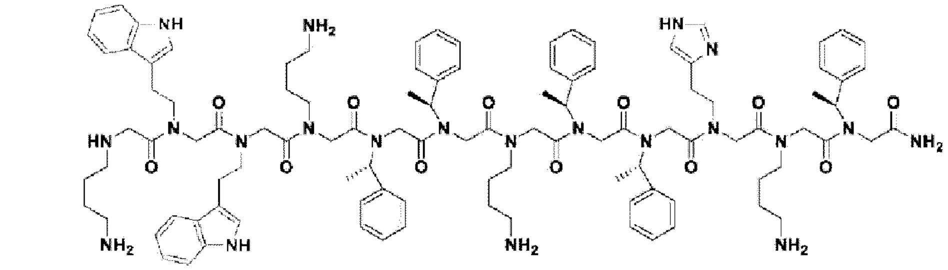 |
| Peptoid 76 | MK-3-65<br>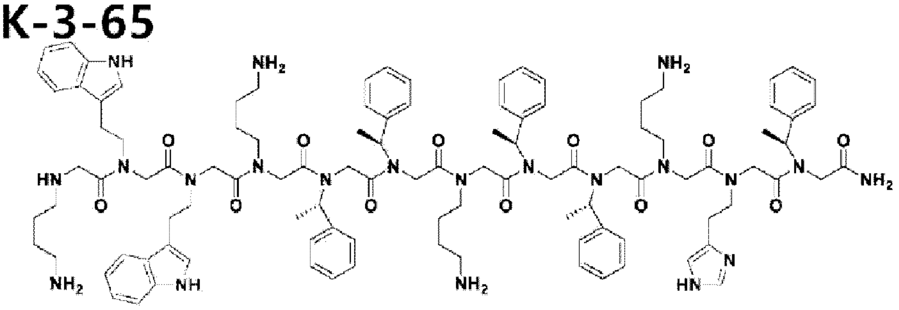 |
| Peptoid 77 | MK-3-66<br>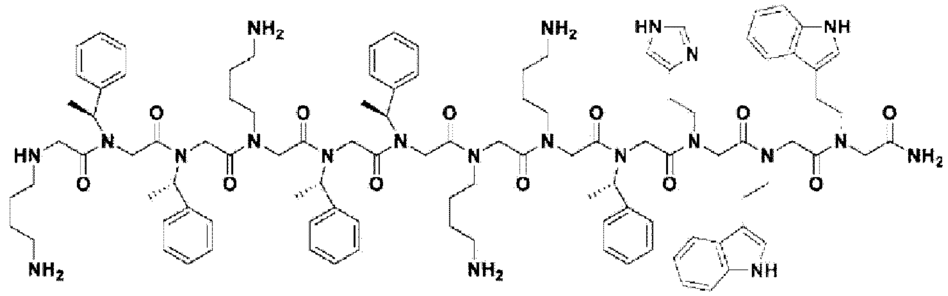 |
| Peptoid 78 | MK-3-67<br>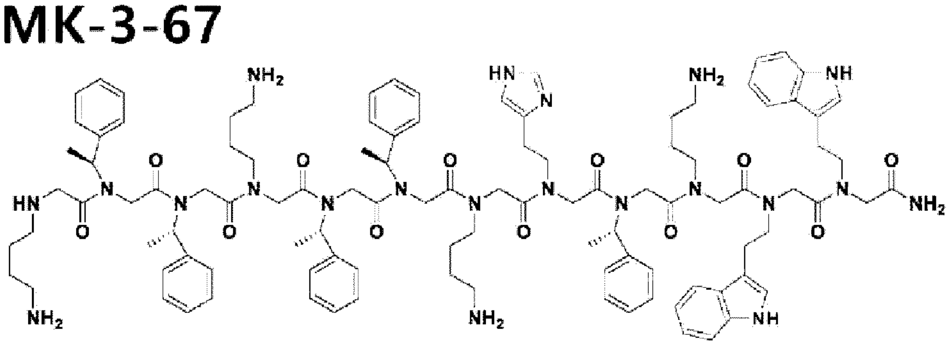 |

(continued)

<table>
<tr><td colspan="2">Chemical structure</td></tr>
<tr><td>Peptoid 79</td><td>MK-3-68<br>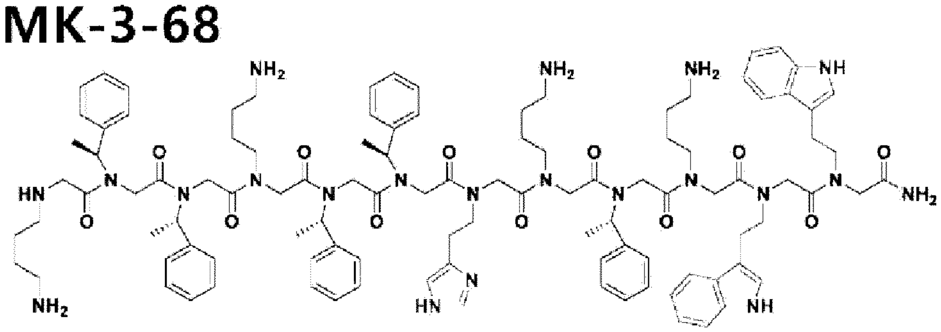</td></tr>
<tr><td>Peptoid 80</td><td>MK-3-69<br>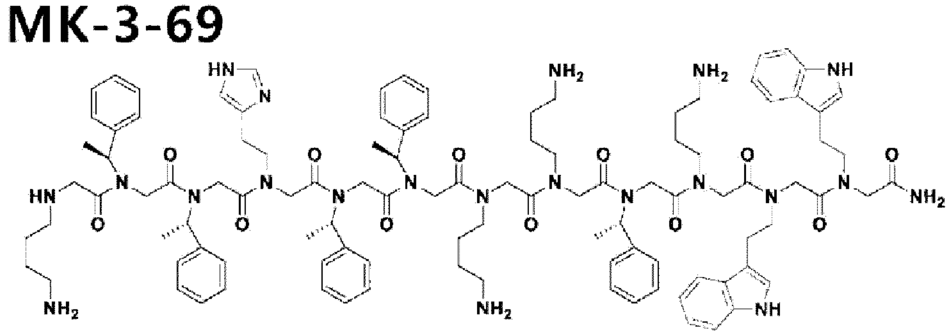</td></tr>
<tr><td>Peptoid 81</td><td>MK-3-70<br>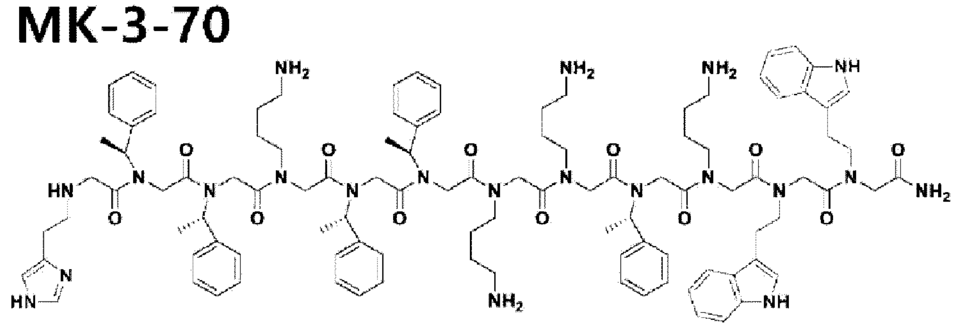</td></tr>
</table>

[Table 21]

| Peptoid | Compound | Molecular weight | Position of *N*His | Net charge | CTLR | HPLC elution (%$CH_3CN$) |
|---|---|---|---|---|---|---|
| 1 | *1* | 1819.36 | - | +4 | 0.33 | 54.1 |
| 67 | MK-3-40 | 1854.37 | 5 | +6 | 0.5 | 44.1 |
| 68 | MK-3-41 | 1854.37 | 6 | +6 | 0.5 | 44.3 |
| 69 | MK-3-42 | 1854.37 | 8 | +6 | 0.5 | 44.7 |
| 70 | MK-3-43 | 1854.37 | 9 | +6 | 0.5 | 44.2 |
| 71 | MK-3-44 | 1854.37 | 12 | +6 | 0.5 | 45.6 |
| 72 | MK-3-61 | 1887.4 | 1 | +5 | 0.42 | 49.3 |
| 73 | MK-3-62 | 1887.4 | 4 | +5 | 0.42 | 49.5 |
| 74 | MK-3-63 | 1887.4 | 7 | +5 | 0.42 | 49.5 |
| 75 | MK-3-64 | 1887.4 | 10 | +5 | 0.42 | 49.2 |

(continued)

| Peptoid | Compound | Molecular weight | Position of *N*His | Net charge | CTLR | HPLC elution (%$CH_3CN$) |
|---|---|---|---|---|---|---|
| 76 | MK-3-65 | 1887.4 | 11 | +5 | 0.42 | 49.3 |
| 77 | MK-3-66 | 1887.4 | 10 | +5 | 0.42 | 48.5 |
| 78 | MK-3-67 | 1887.4 | 8 | +5 | 0.42 | 49.2 |
| 79 | MK-3-68 | 1887.4 | 7 | +5 | 0.42 | 48.9 |
| 80 | MK-3-69 | 1887.4 | 4 | +5 | 0.42 | 49.1 |
| 81 | MK-3-70 | 1887.4 | 1 | +5 | 0.42 | 49 |

<Analysis Example 15: HPLC and LC-MS for Peptoids 67 to 81>

FIGS. 86 to 100 show the HPLC results for antimicrobial Peptoids 67 to 81 according to Example 3 of the present invention.

Table 21 shows the HPLC and LC-MS results for the antimicrobial peptoids according to Example 3 of the present invention.

Referring to FIGS. 86 to 100, and Table 21, HPLC and LC-MS were performed in the same manner as in Analysis Example 1 described above. As a result, the synthesized antimicrobial peptoids generally showed a retention time in the range of 44 minutes to 50 minutes in the HPLC elution assay.

[Table 22]

| Peptoid | Compound | MIC (μM) | | $HD_{10}/HD_{50}$ | $H_{max}$ (100 μM) | Selectivity |
|---|---|---|---|---|---|---|
| | | *E. coli* | *S. aureus* | | | |
| | | ATCC25922 | ATCC25923 | | | |
| 1 | *1* | 3.1 | 1.6 | 22.9/79.0 | 75.8 | 7.4 |
| - | omiganan | 12.5 ~ 25 | 12.5 | - | 0 | - |
| 67 | MK-3-40 | >25 | 25 | nd | nd | nd |
| 68 | MK-3-41 | >25 | 25 | nd | nd | nd |
| 69 | MK-3-42 | 25 | 25 | nd | nd | nd |
| 70 | MK-3-43 | >25 | >25 | nd | nd | nd |
| 71 | MK-3-44 | >25 | >25 | nd | nd | nd |
| 72 | MK-3-61 | 6.3 | 6.3 | 88.9/>100.0 | 11.8 ± 1.1 | 14.1 |
| **73** | **MK-3-62** | **6.3** | **3.1** | **>100.0/>100.0** | **10.0 ± 0.3** | **>15.9** |
| 74 | MK-3-63 | 6.3 | 3.1 | 81.7/>100.0 | 14.2 ± 0.3 | 13 |
| 75 | MK-3-64 | 6.3 | 6.3 | 68.0/>100.0 | 18.3 ± 1.1 | 10.8 |
| 76 | MK-3-65 | 6.3 | 6.3 | >100.0/>100.0 | 10.1 ± 0.7 | >15.9 |
| 77 | MK-3-66 | 12.5 | 6.3 | >100.0/>100.0 | 3.2 ± 0.3 | >8.0 |
| **78** | **MK-3-67** | **6.3** | **6.3** | **>100.0/>100.0** | **5.2 ± 0.2** | **>15.9** |
| 79 | MK-3-68 | 6.3 | 6.3 | >100.0/>100.0 | 8.5 ± 0.6 | >15.9 |
| 80 | MK-3-69 | 12.5 | 6.3 | >100.0/>100.0 | 4.0 ± 0.5 | >8.0 |
| 81 | MK-3-70 | 6.3 | 6.3 | >100.0/>100.0 | 5.7 ± 0.1 | >15.9 |

Table 22 shows the result of antimicrobial activity analysis for antimicrobial peptoids according to Example 3 of the present invention. In Table 22, the term "nd" is the abbreviation for not determined, and indicates a value that is too small or too large to be measured.

Referring to Table 22, to measure the antimicrobial activity of the peptoids synthesized in Example 3, an MIC was measured by treating gram-negative bacteria, that is, the E. *coli* ATCC25922 strain, and gram-positive bacteria, that is, the *S. aureus* ATCC25923 strain, with the peptoids. Here, Peptoid 1 is a conventionally known antimicrobial peptoid, and Omiganan is a derivative of indolicidin, which is an antimicrobial peptide, under clinical development, and was used as a control for comparing antimicrobial activity with the novel peptoid.

A detailed method for analyzing antimicrobial activity is the same as in Analysis Example 3 described above. As a result, Peptoids 67 to 71 were prepared by substituting one Nspe in the above-described Peptoid 32 with NHis, and had low antimicrobial activity and hemolysis did not proceed. In addition, Peptoids 72, 73, 74 and 76 were prepared by substituting one *N*Lys in Peptoid 32 with NHis, and they retained antimicrobial activity and were improved in toxicity. In addition, Peptoids 78, 79 and 81 were prepared by substituting one NLys in the above-described Peptoid 29 with NHis, and they retained antimicrobial activity and were improved in toxicity. On the other hand, Peptoids 77 and 80 exhibited reduced antimicrobial activity and improved toxicity. That is, as one Nspe or NLys in an antimicrobial peptoid was substituted with *N*His, the change in antimicrobial activity of an antimicrobial peptoid could be identified.

<Analysis Example 16: ESI-MS for Peptoids 67 to 81>

The calculation and analysis results of ESI-MS for the antimicrobial peptoids according to Example 3 of the present invention are shown in Table 23 below.

[Table 23]

| Peptoid | Compound | Calculated mass | | Observed mass | |
|---|---|---|---|---|---|
| | | $[M+H]^+$ | $[M+2H]^{2+}$ | $[M+H]^+$ | $[M+2H]^{2+}$ |
| 67 | MK-3-40 | 1854.1 | 927.6 | 1854.7 | 927.9 |
| 68 | MK-3-41 | 1854.1 | 927.6 | 1854.6 | 927.9 |
| 69 | MK-3-42 | 1854.1 | 927.6 | 1854.6 | 927.9 |
| 70 | MK-3-43 | 1854.1 | 927.6 | 1854.6 | 927.9 |
| 71 | MK-3-44 | 1854.1 | 927.6 | 1854.7 | 928 |
| 72 | MK-3-61 | 1887.1 | 944.1 | 1887.8 | 944.5 |
| 73 | MK-3-62 | 1887.1 | 944.1 | 1887.7 | 944.4 |
| 74 | MK-3-63 | 1887.1 | 944.1 | 1887.7 | 944.4 |
| 75 | MK-3-64 | 1887.1 | 944.1 | 1887.8 | 944.5 |
| 76 | MK-3-65 | 1887.1 | 944.1 | 1887.7 | 944.4 |
| 77 | MK-3-66 | 1887.1 | 944.1 | 1887.8 | 944.5 |
| 78 | MK-3-67 | 1887.1 | 944.1 | 1887.7 | 944.4 |
| 79 | MK-3-68 | 1887.1 | 944.1 | 1887.8 | 944.4 |
| 80 | MK-3-69 | 1887.1 | 944.1 | 1888 | 944.7 |
| 81 | MK-3-70 | 1887.1 | 944.1 | 1887.7 | 944.7 |

Referring to Table 23, the ESI-MS result for each peptoid sample revealed that a calculated mass is the same as an observed mass, thereby confirming that a peptoid with a predicted structure and a peptoid with an actual structure are manufactured in the same manner.

Meanwhile, the embodiments of the present invention shown in the specification and drawings are merely provided to present specific examples to better explain the present invention, and do not limit the scope of the present invention. Other than the embodiments disclosed herein, it is obvious to those of ordinary skill in the art that other modified embodiments based on the technical idea of the present invent ion can be implemented.

## Claims

**1.** An antimicrobial peptoid represented by Formula 1 below:

[Formula 1] $H-X_1-Y_1-Z_1-X_2-Y_2-Z_2-X_3-Y_3-Z_3-X_4-Y_4-Z_4-NH_2$

wherein $X_1$ to $Z_1$, $X_2$ to $Z_2$, $X_3$ to $Z_3$, and $X_4$ to $Z_4$ are each independently Nspe, *N*Lys, *N*hTrp, *N*His, *N*pm, or *N*4hb.

**2.** The antimicrobial peptoid of claim 1, which is at least one selected from those represented by Formulas 2 to 5 below:

[Formula 2] H-*N*Lys-*N*spe-*N*spe-*N*Lys-*N*spe-*N*spe-*N*Lys-*N*Lys-*N*spe-*N*Lys-*N*hTrp-*N*hTrp- $NH_2$;

[Formula 3] H-*N*Lys-*N*hTrp-*N*hTrp-*N*Lys-*N*spe-*N*spe-*N*Lys-*N*spe-*N*spe-*N*Lys-*N*Lys-*N*spe- $NH_2$;

[Formula 4] H-*N*Lys-*N*hTrp-*N*hTrp-*N*His-*N*spe-*N*spe-*N*Lys-*N*spe-*N*spe-*N*Lys-*N*Lys-*N*spe- $NH_2$; and

[Formula 5] H-*N*Lys-*N*spe-*N*spe-*N*Lys-*N*spe-*N*spe-*N*Lys-*N*His-*N*spe-*N*Lys-*N*hTrp-*N*hTrp- $NH_2$.

**3.** An antimicrobial composition, comprising at least one of the antimicrobial peptoids, selected in claims 1 and 2, as an active ingredient.

**4.** The composition of claim 3, wherein the antimicrobial peptoid exhibits antimicrobial activity against gram-positive bacteria or gram-negative bacteria.

**5.** The composition of claim 4, wherein the gram-positive bacteria are bacteria of *Staphylococcus sp., Bacillus sp., Streptococcus sp.,* or *Enterococcus sp.*

**6.** The composition of claim 4, wherein the gram-positive bacteria are *Staphylococcus aureus, methicillin-resistant Staphylococcus aureus* (MRSA), *Quinolone-resistant Staphylococcus aureus* (QRSA), *vancomycin-resistant enterococcus* (VRE), *vancomycin intermediate-resistant Staphylococcus aureus* (VISA), *Bacillus subtilis, Bacillus cereus, Streptococcus pneumoniae, Enterococcus faecalis, Enterococcus faecium,* or *Staphylococcus epidermidis.*

**7.** The composition of claim 4, wherein the gram-negative bacteria are bacteria of *Salmonella sp., Acinetobacter sp., Escherichia sp., Pseudomonas sp., Enterobacter sp.,* or *Klebsiella sp.*

**8.** The composition of claim 4, wherein the gram-negative bacteria are *Salmonella typhimurium, Acinetobacter calcoaceticus, Acinetobacter baumannii, Escherichia coli*, *Pseudomonas aeruginosa*, *Klebsiella pneumoniae*, or *Klebsiella aerogenes.*

**9.** A method of preparing an antimicrobial composition, comprising:

preparing deprotected polymer resin beads (S 100);
preparing a bromoacetylate peptoid by adding the deprotected polymer resin beads, bromoacetic acid, diisopropylcarbodiimide (DIC), and an organic solvent and performing a bromoacetylation reaction (S200);
adding a submonomer to the bromoacetylate peptoids and performing an amine substitution reaction (S300);
obtaining an antimicrobial peptoid having the desired sequence by repeating S200 to S300 (S400); and
separating a polymer resin from the antimicrobial peptoid using a cleavage solution (S500),
wherein the submonomer is at least one selected from Nspe, NLys, NhTrp, NHis, Npm, and N4hb.

**10.** The method of claim 9, further comprising, after S500, performing a counter ion substitution reaction by removing trifluoroacetate (TFA) ions included in the composition using a carbonate ion exchange resin and adding an acidic solution (S600).

**11.** The method of claim 10, wherein the acidic solution is acetic acid or hydrochloric acid.

FIG. 1

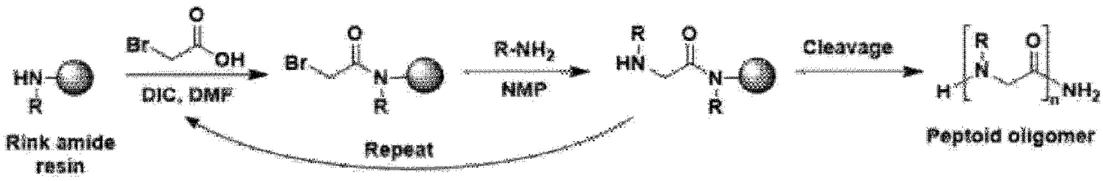
DIC, DMF
R-NH2
NMP
Cleavage
Repeat
Rink amide resin
Peptoid oligomer

FIG. 2

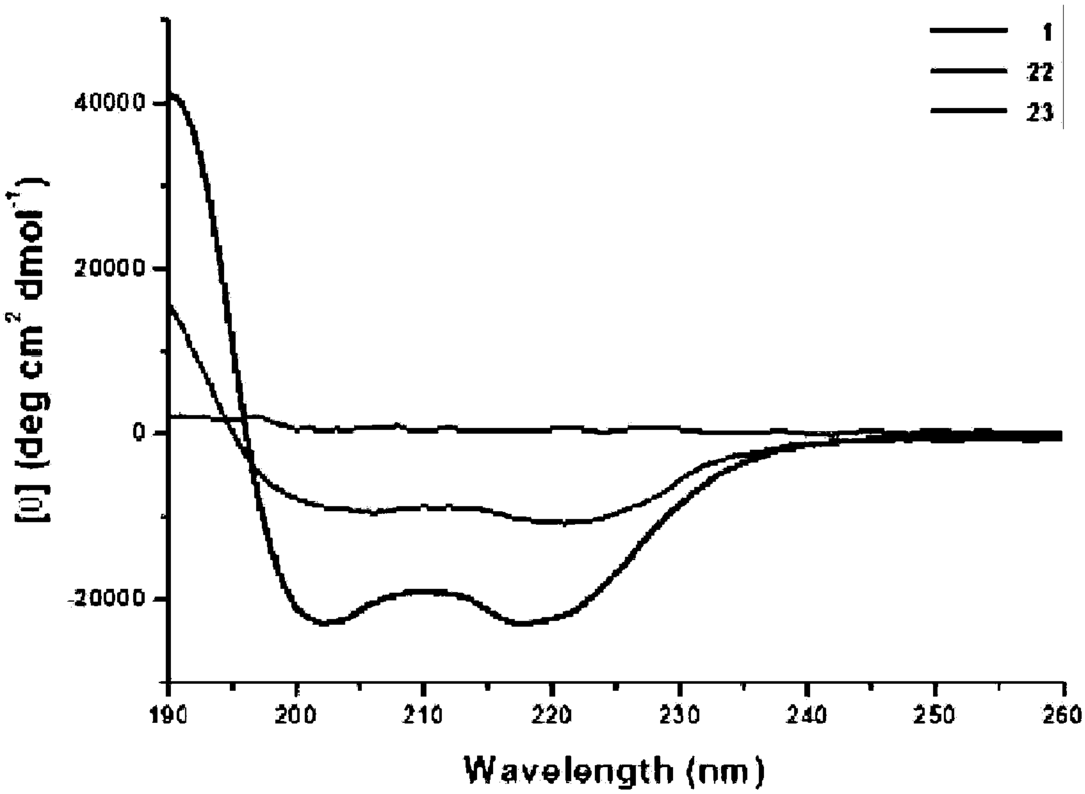
1
22
23
40000
20000
0
-20000
[θ] (deg cm2 dmol-1)
190
200
210
220
230
240
250
260
Wavelength (nm)

FIG. 3

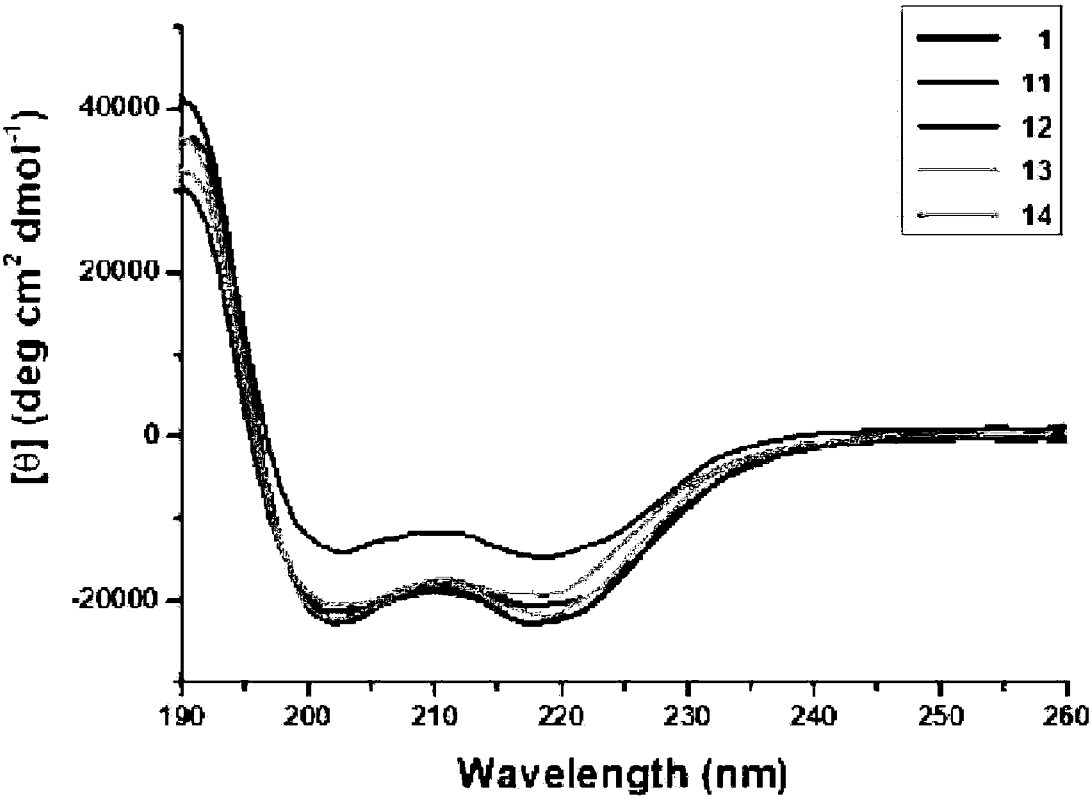
1
11
12
13
14
40000
20000
0
-20000
[θ] (deg cm$^2$ dmol$^{-1}$)
190
200
210
220
230
240
250
260
Wavelength (nm)

FIG. 4

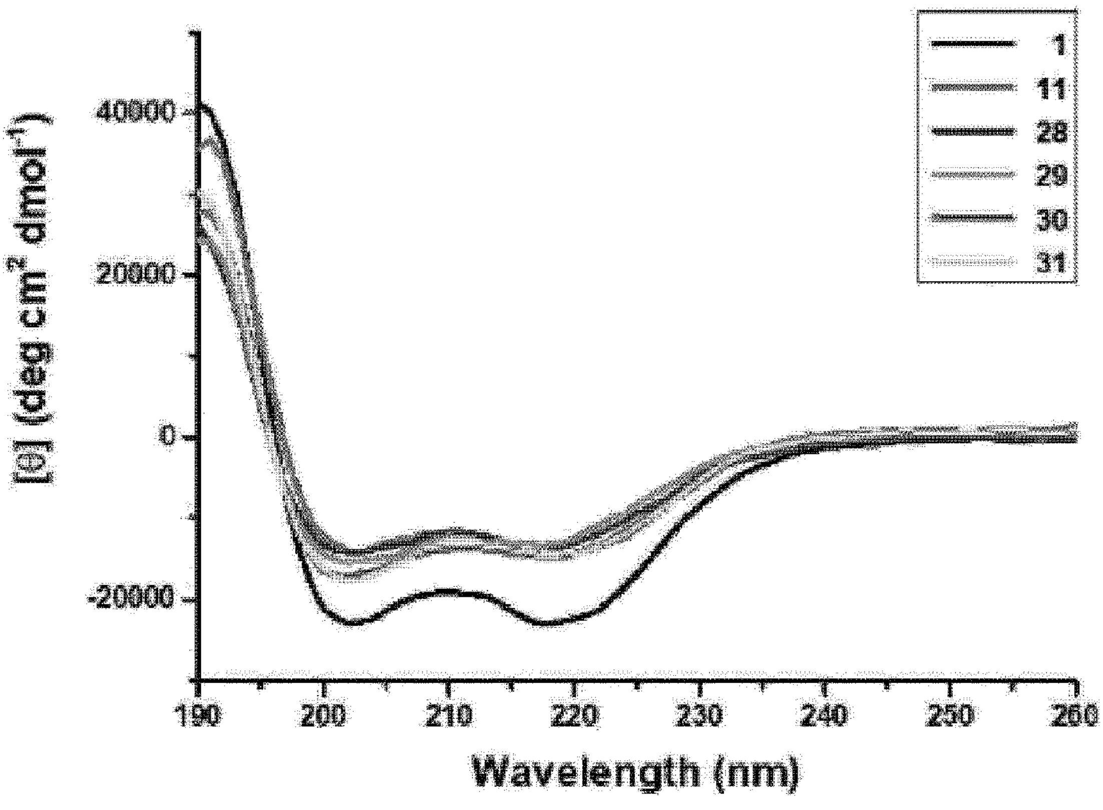
1
11
28
29
30
31
40000
20000
0
-20000
[θ] (deg cm$^2$ dmol$^{-1}$)
190
200
210
220
230
240
250
260
Wavelength (nm)

FIG. 5

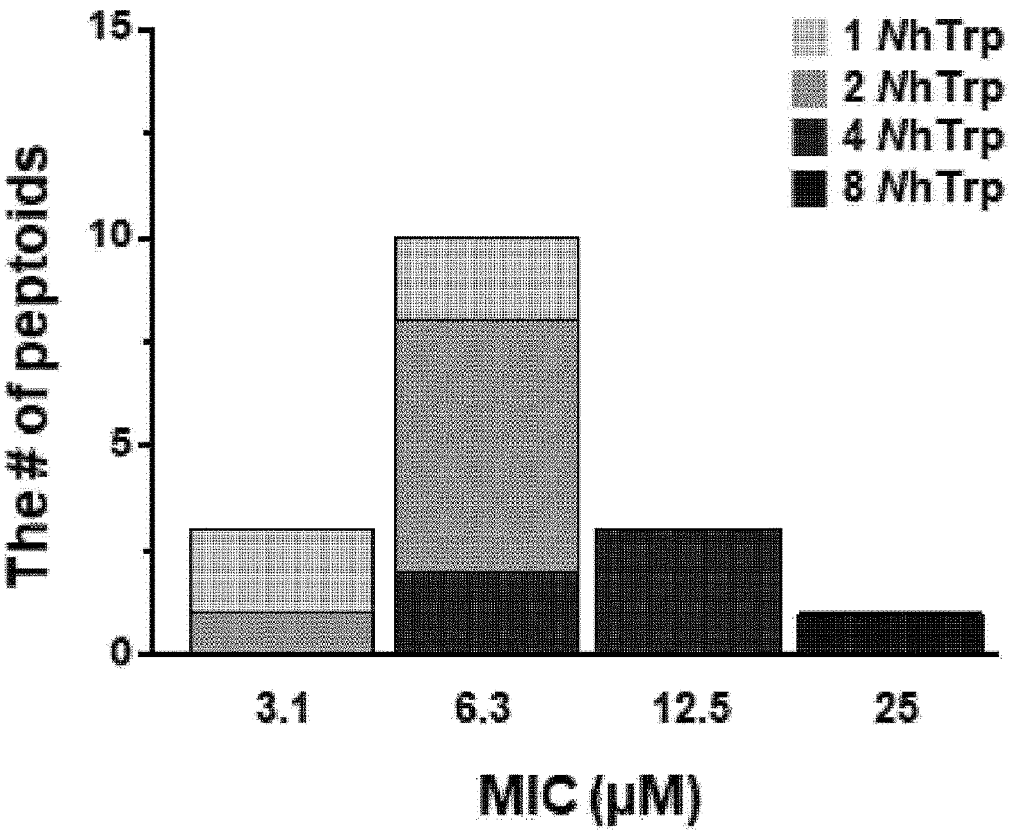
15
10
5
0
The # of peptoids
1 NhTrp
2 NhTrp
4 NhTrp
8 NhTrp
3.1
6.3
12.5
25
MIC (μM)

FIG. 6

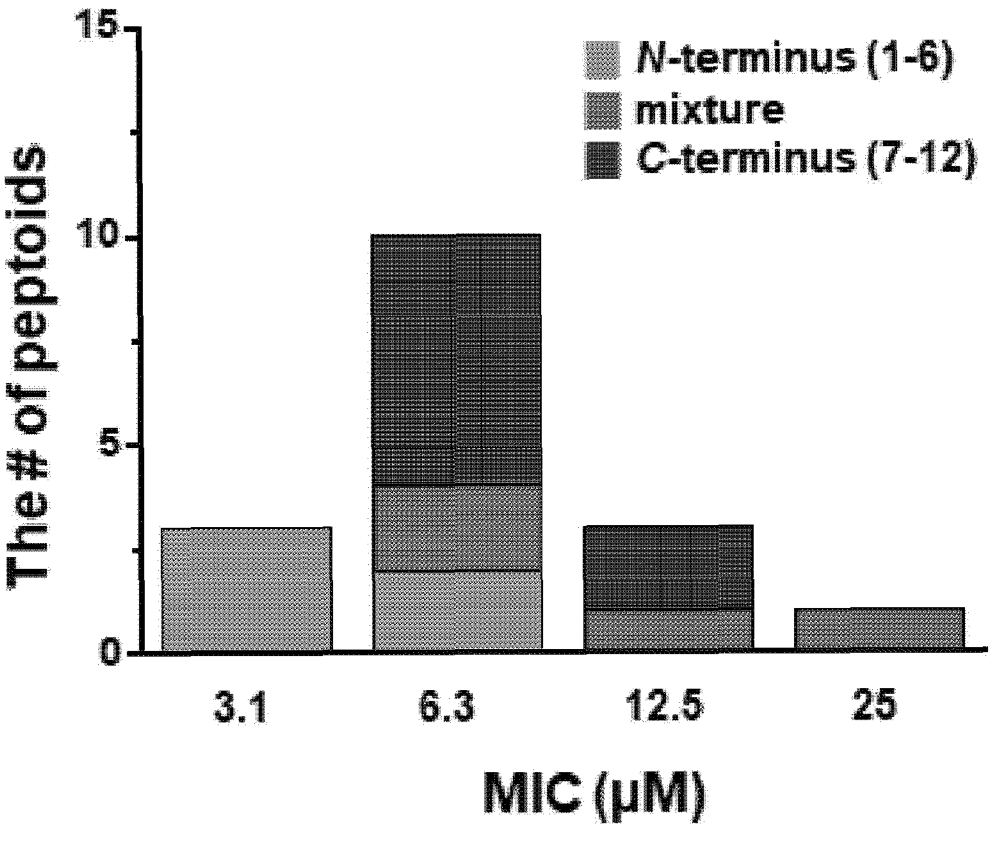
N-terminus (1-6)
mixture
C-terminus (7-12)
The # of peptoids
15
10
5
0
3.1
6.3
12.5
25
MIC (μM)

FIG. 7

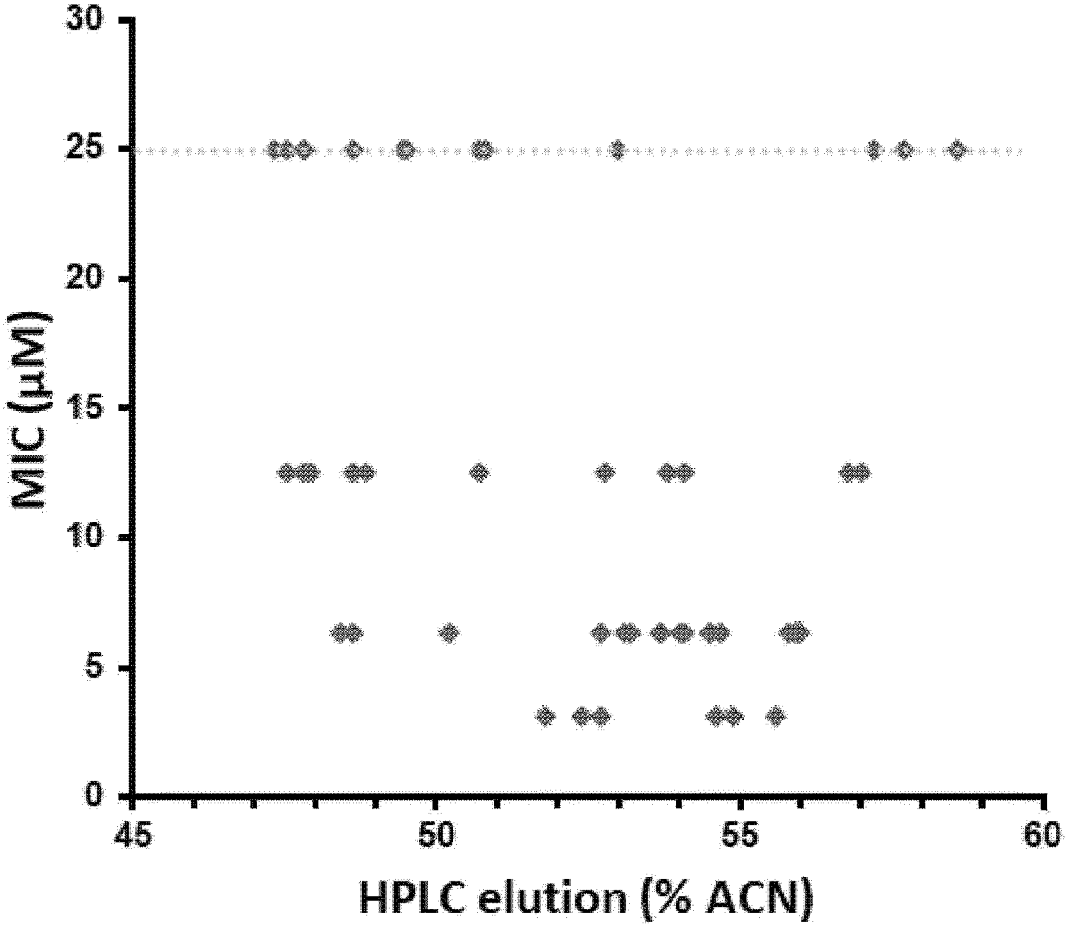

FIG. 8

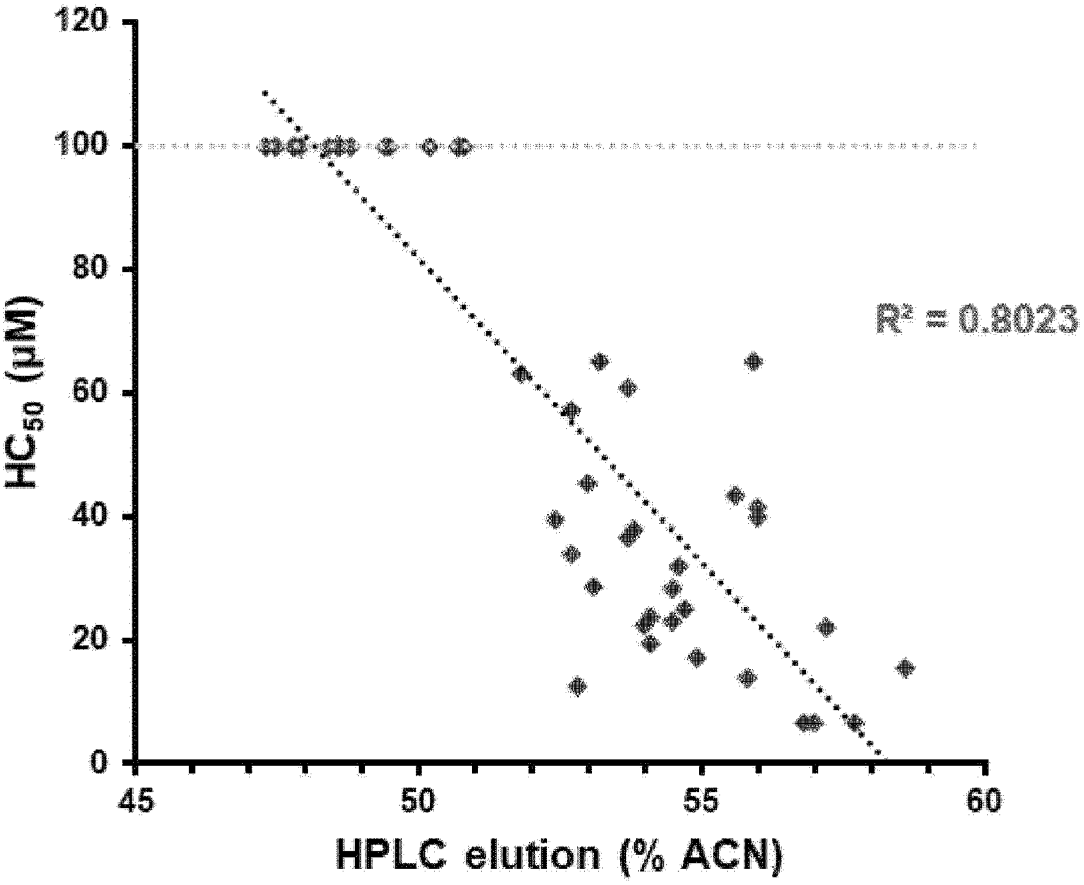
120
100
80
60
40
20
0
$HC_{50}$ (µM)
45
50
55
60
HPLC elution (% ACN)
R² = 0.8023

FIG. 9

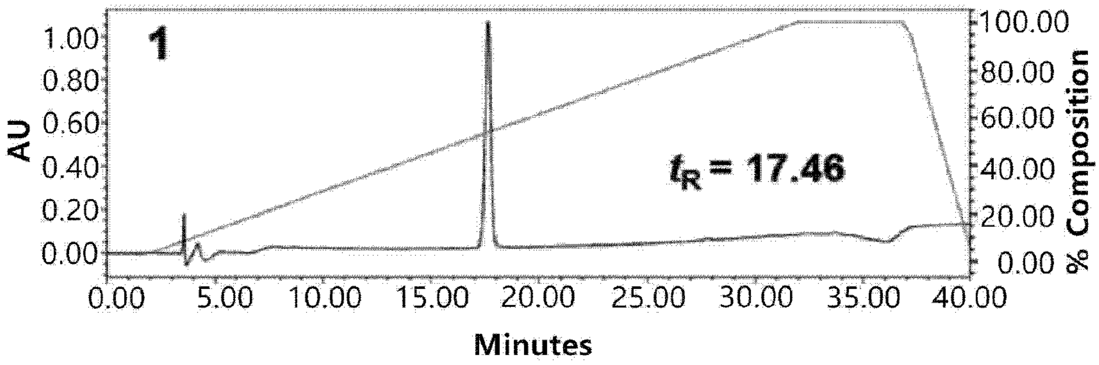


FIG. 10

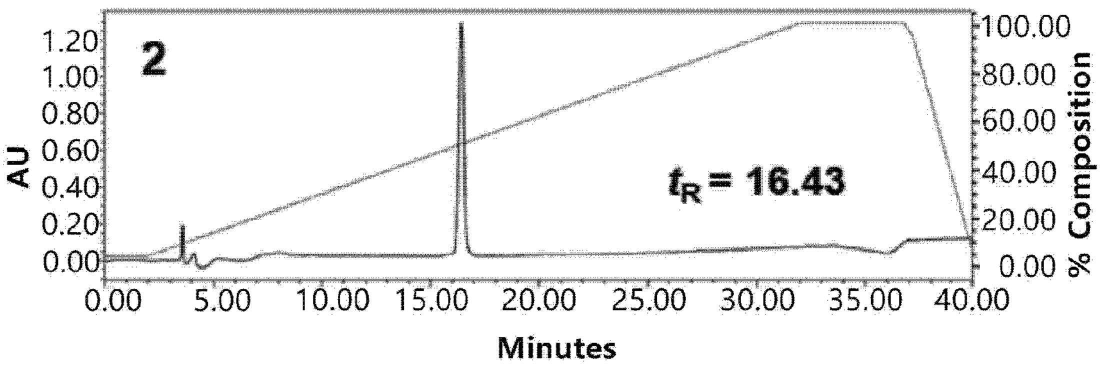


FIG. 11

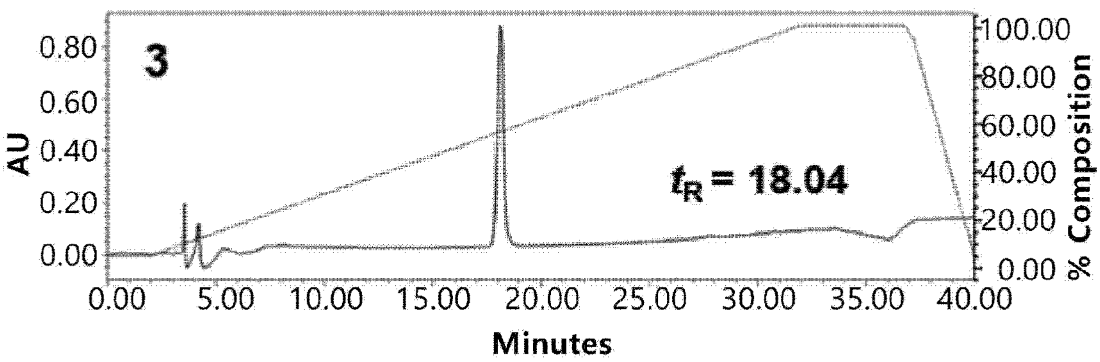


FIG. 12

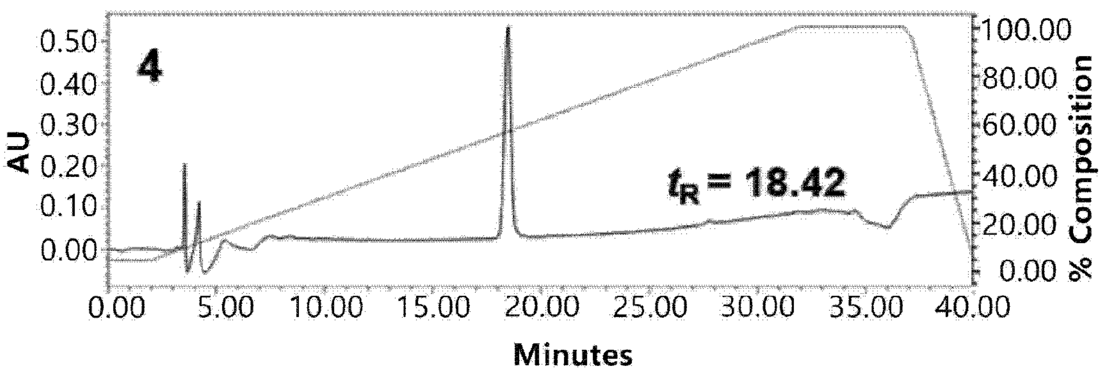
4
AU
0.50
0.40
0.30
0.20
0.10
0.00
tR = 18.42
100.00
80.00
60.00
40.00
20.00
0.00
% Composition
0.00 5.00 10.00 15.00 20.00 25.00 30.00 35.00 40.00
Minutes

FIG. 13

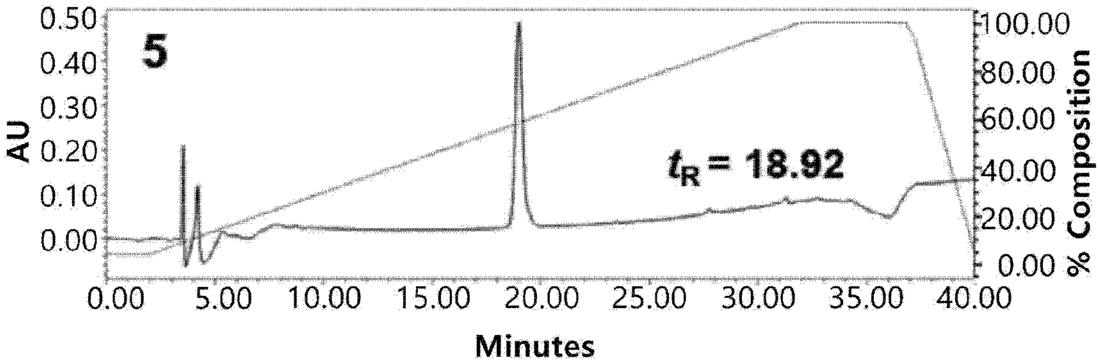


FIG. 14

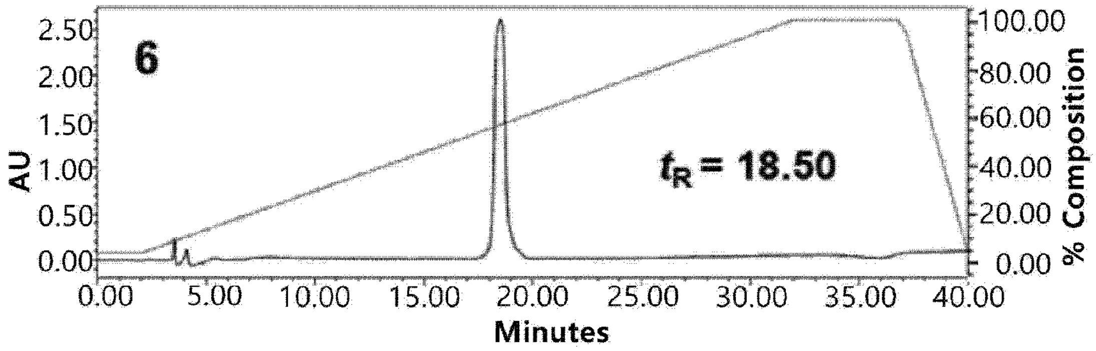


FIG. 15

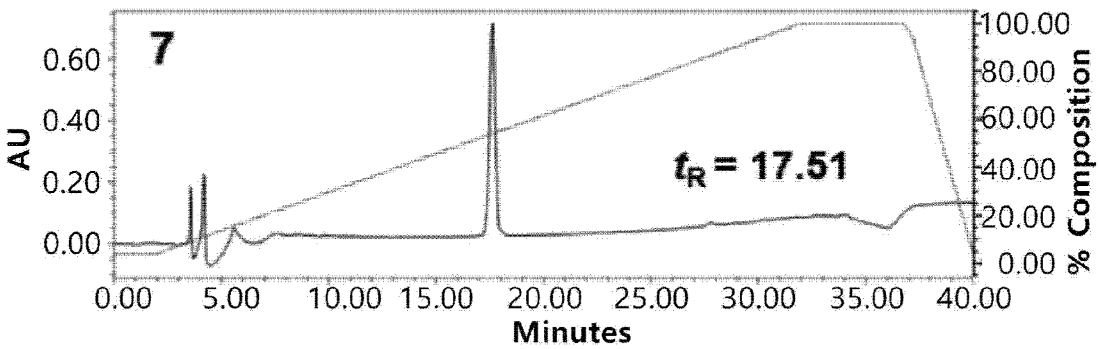


FIG. 16

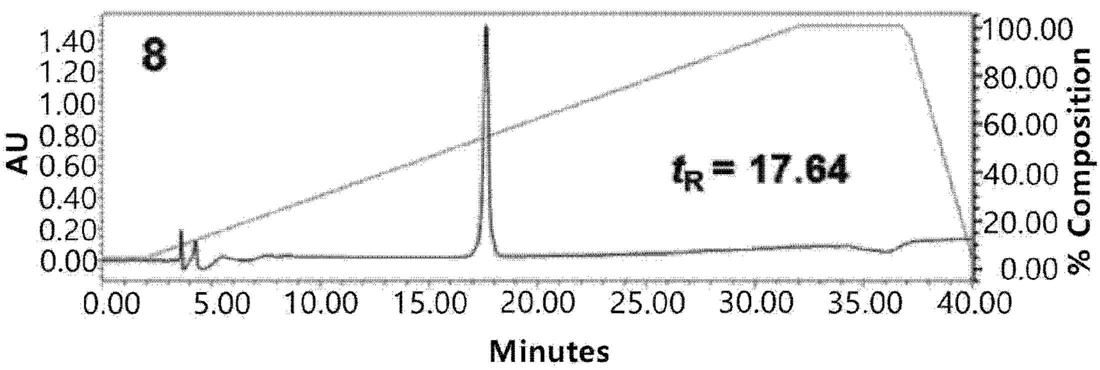

FIG. 17

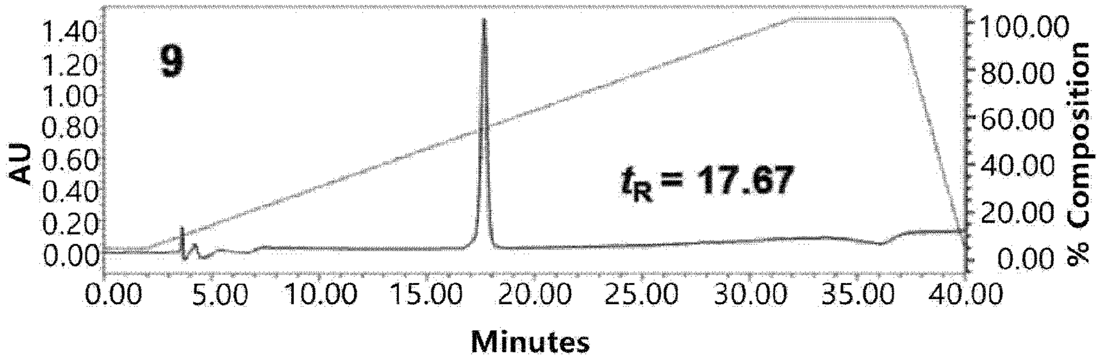


FIG. 18

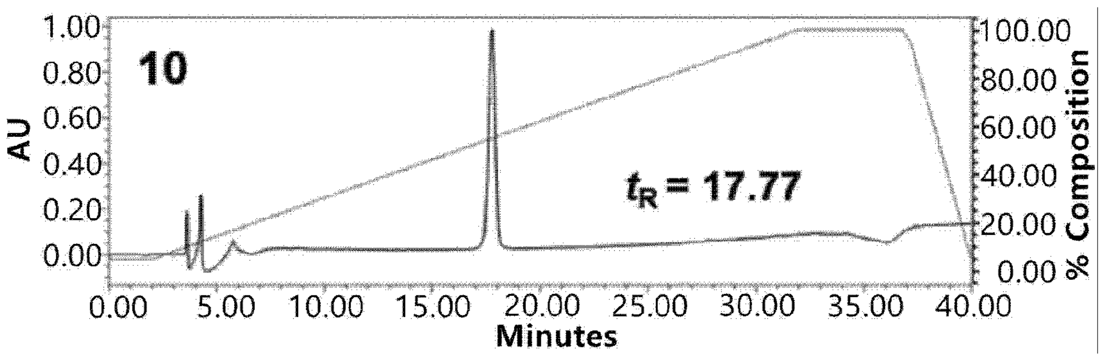


FIG. 19

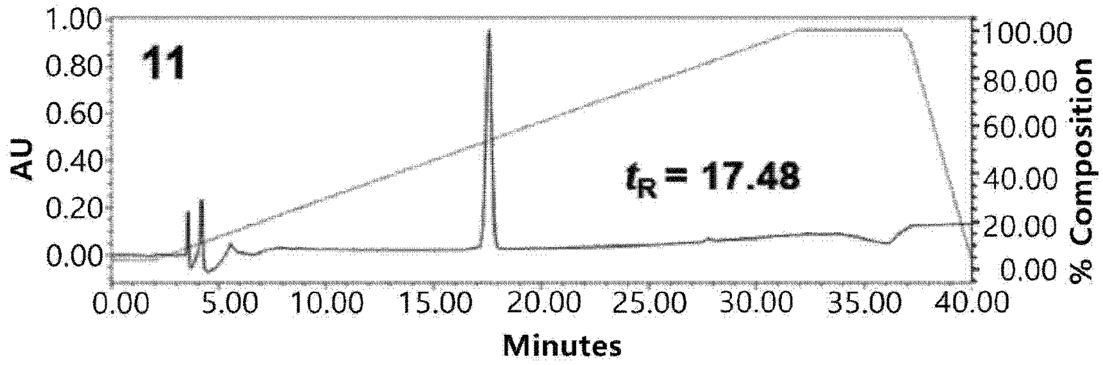


FIG. 20

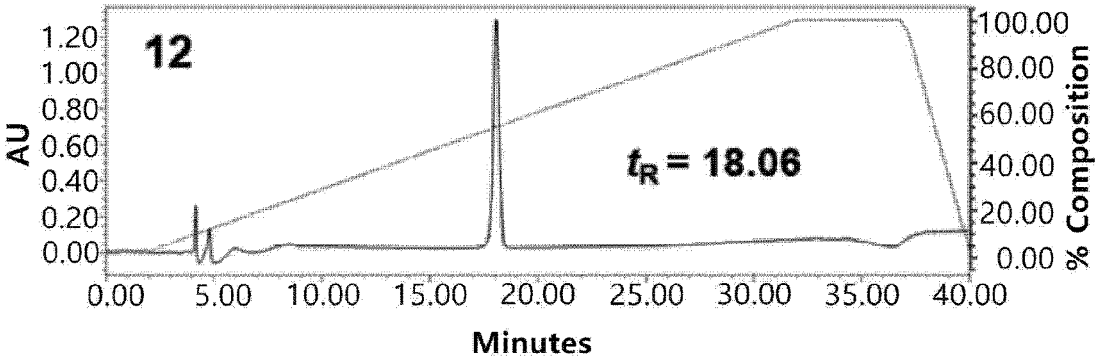


FIG. 21

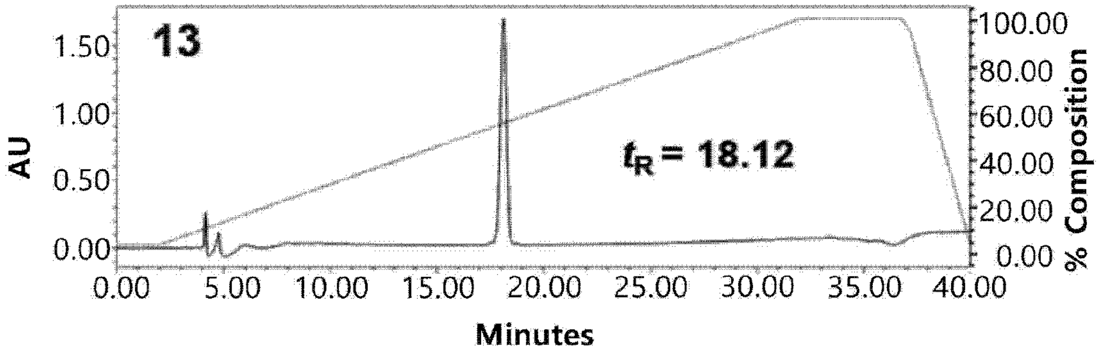


FIG. 22

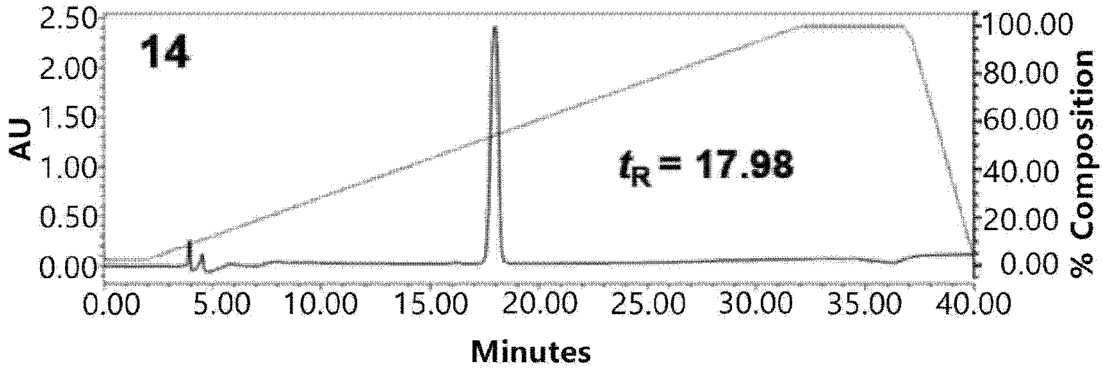


FIG. 23

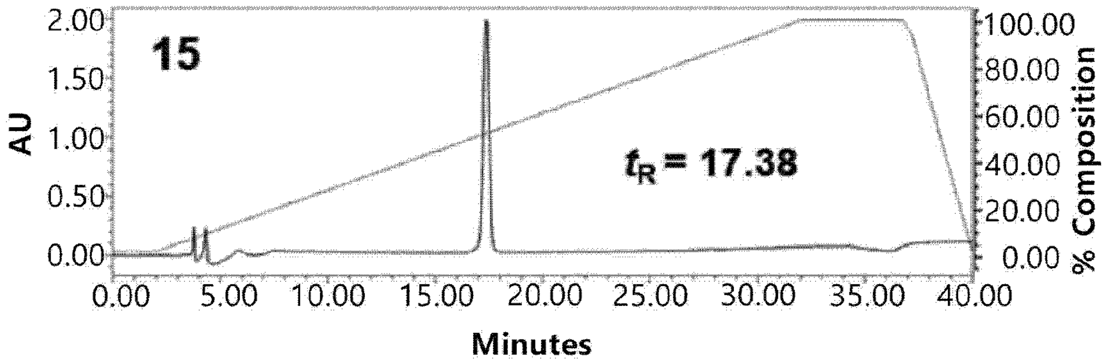

FIG. 24

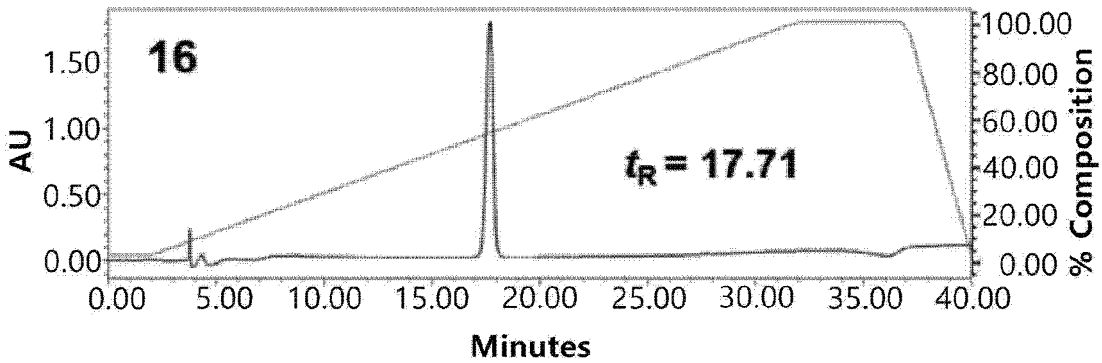
16
t_R = 17.71
AU
1.50
1.00
0.50
0.00
100.00
80.00
60.00
40.00
20.00
0.00
% Composition
0.00
5.00
10.00
15.00
20.00
25.00
30.00
35.00
40.00
Minutes

FIG. 25

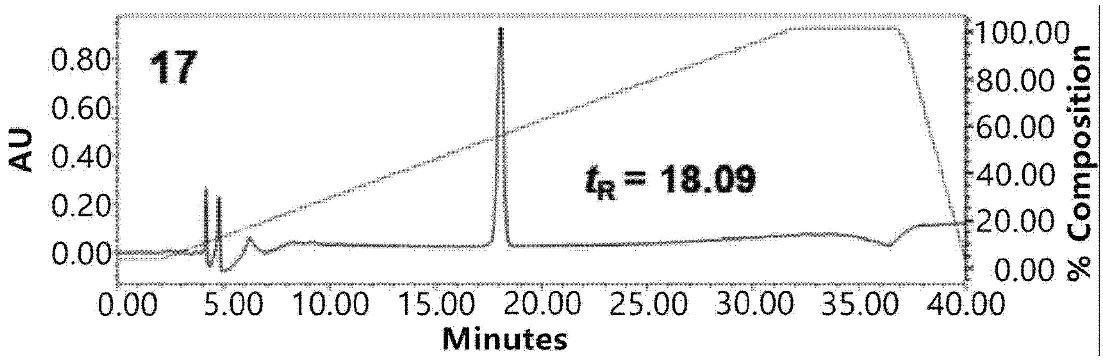


FIG. 26

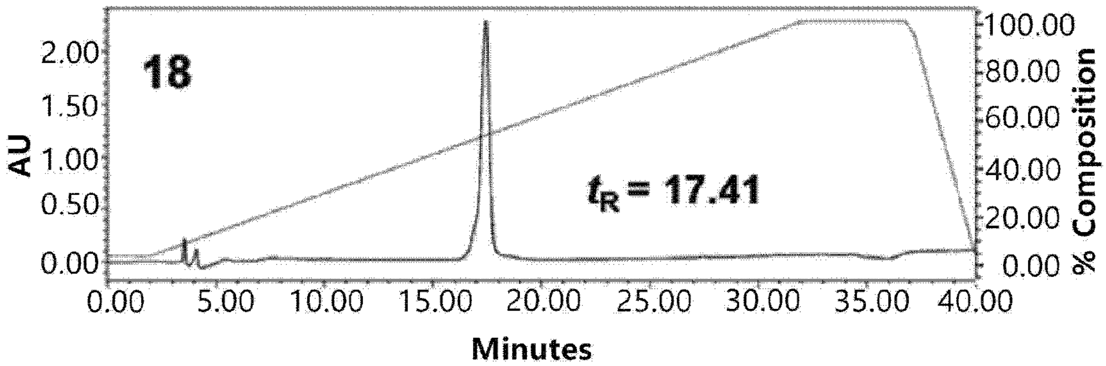


FIG. 27

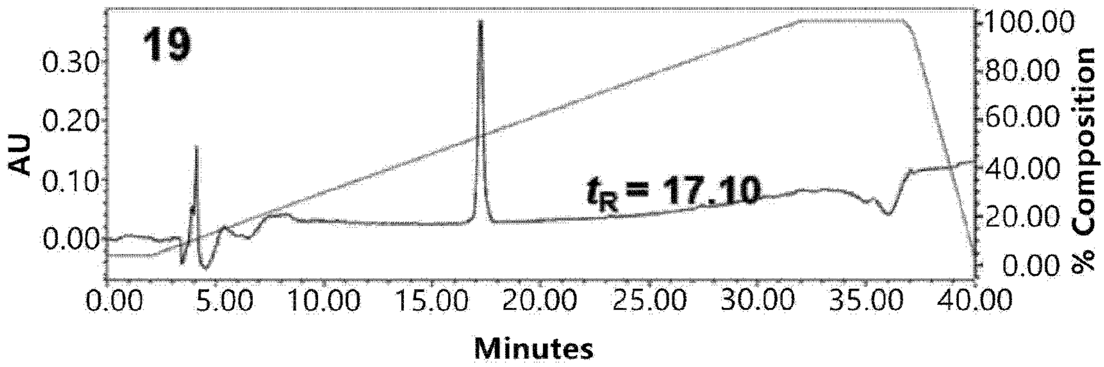


FIG. 28

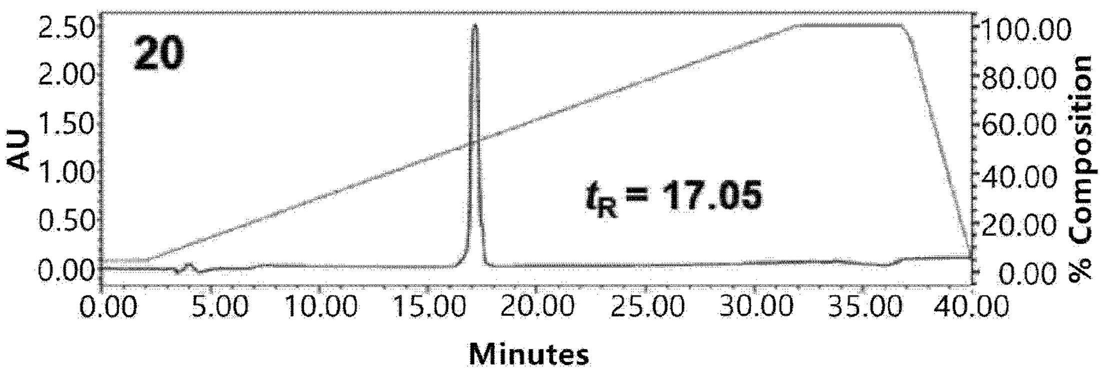
20
$t_R$ = 17.05
AU
2.50
2.00
1.50
1.00
0.50
0.00
100.00
80.00
60.00
40.00
20.00
0.00
% Composition
0.00
5.00
10.00
15.00
20.00
25.00
30.00
35.00
40.00
Minutes

FIG. 29

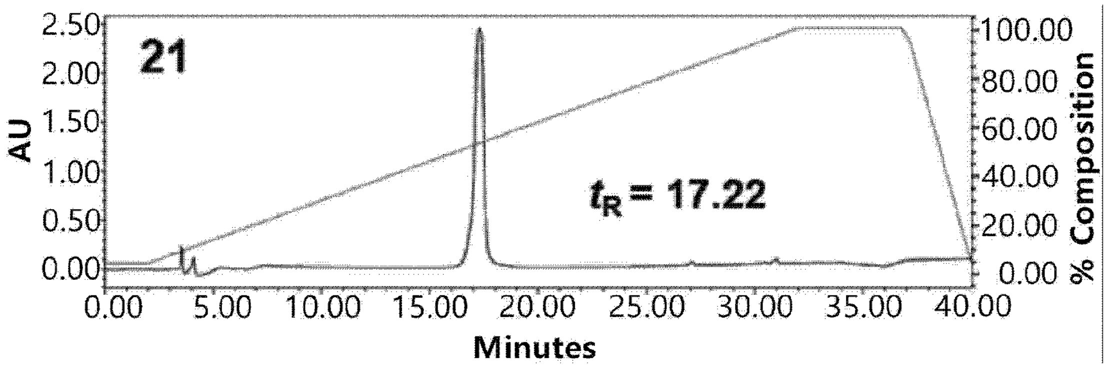


FIG. 30

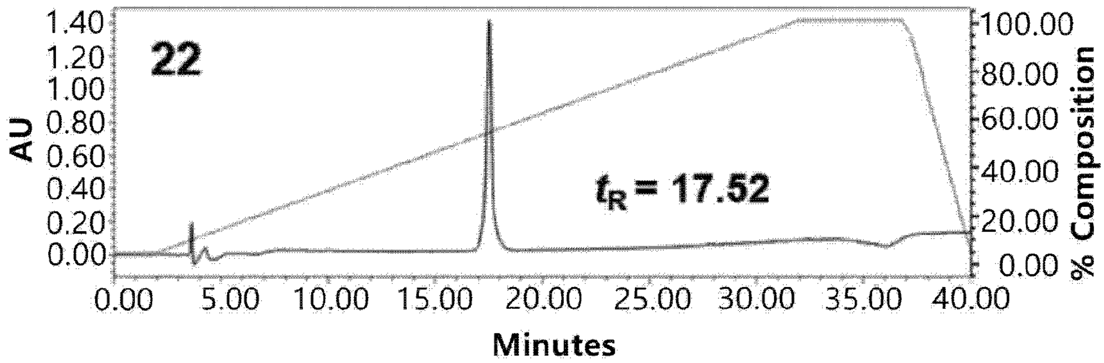


FIG. 31

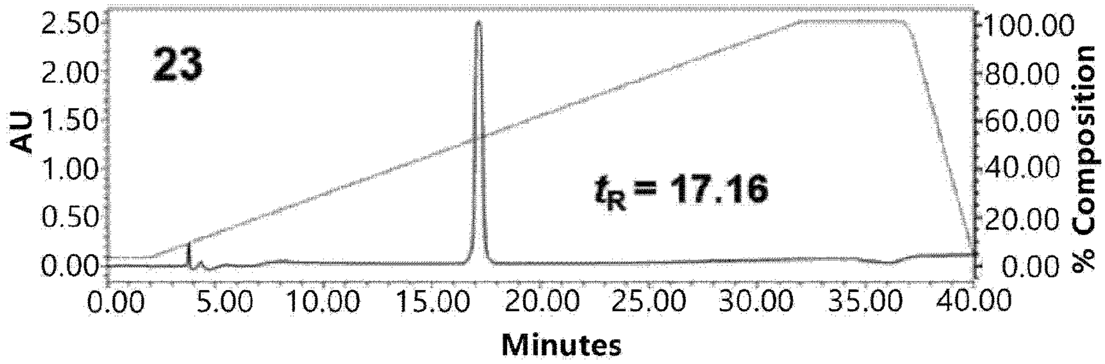


FIG. 32

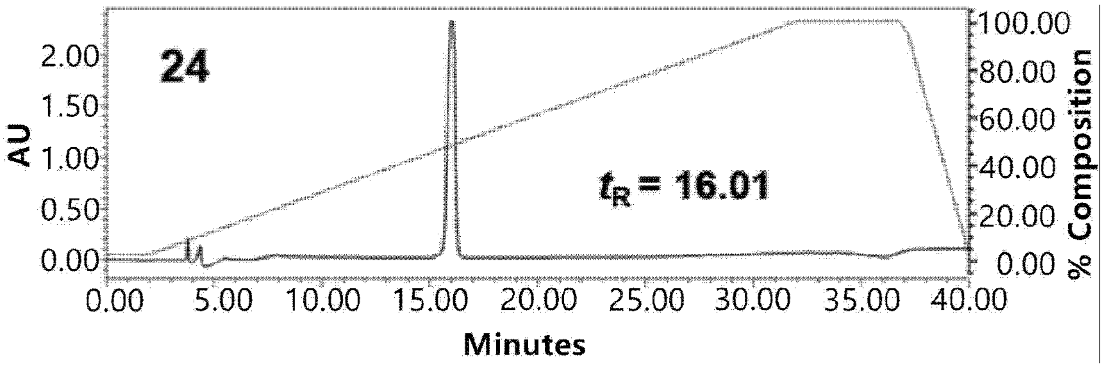


FIG. 33

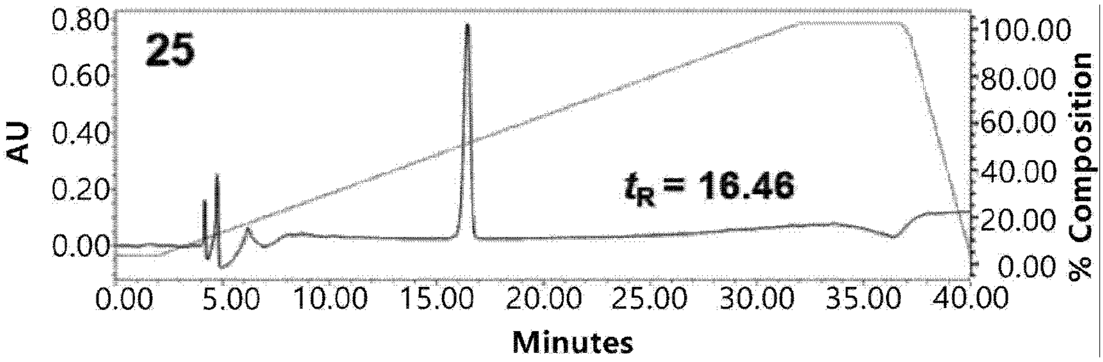


FIG. 34

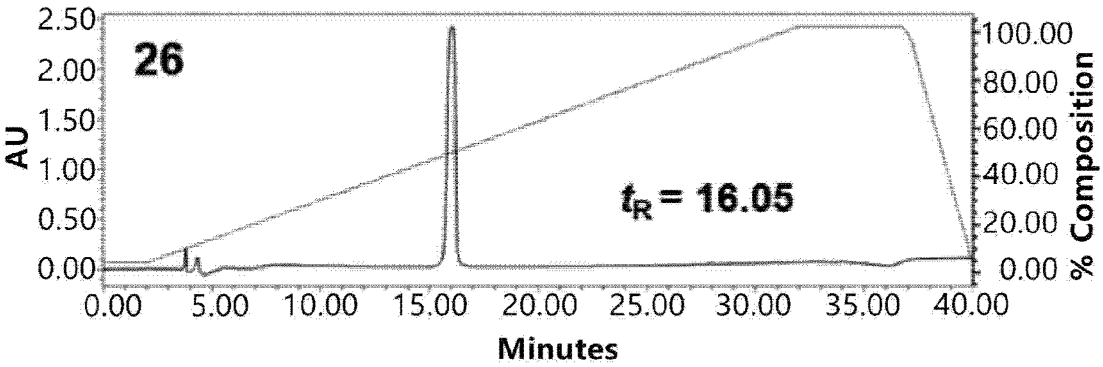


FIG. 35

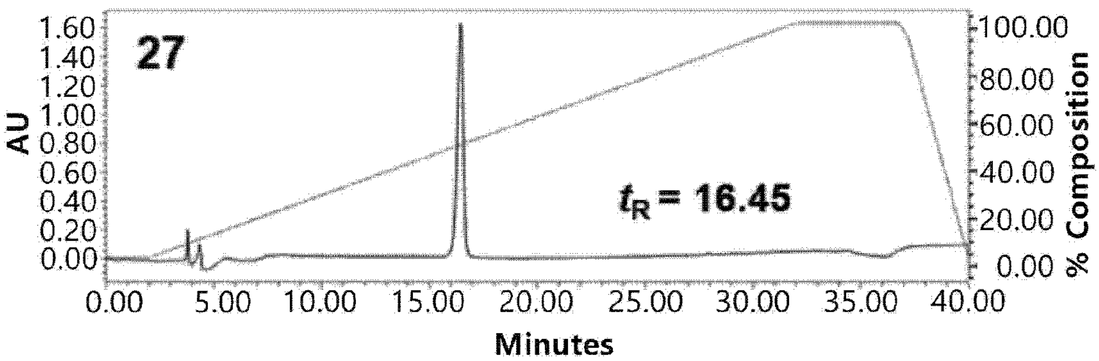

FIG. 36

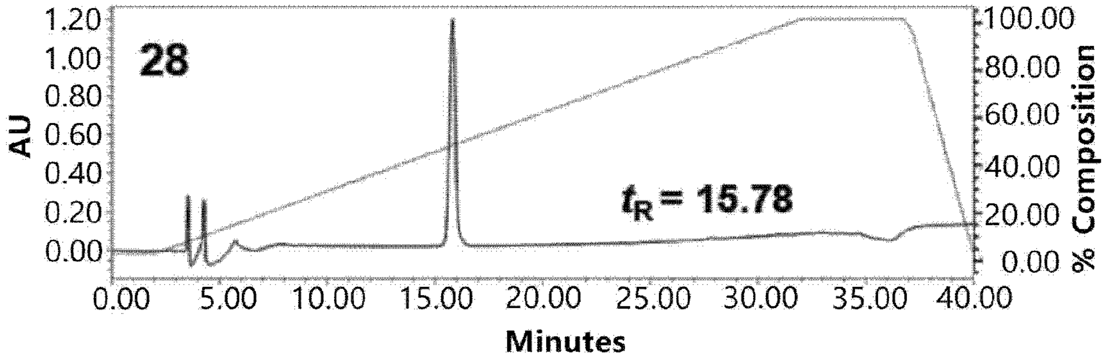
28
AU
1.20
1.00
0.80
0.60
0.40
0.20
0.00
100.00
80.00
60.00
40.00
20.00
0.00
% Composition
$t_R$ = 15.78
0.00
5.00
10.00
15.00
20.00
25.00
30.00
35.00
40.00
Minutes

FIG. 37

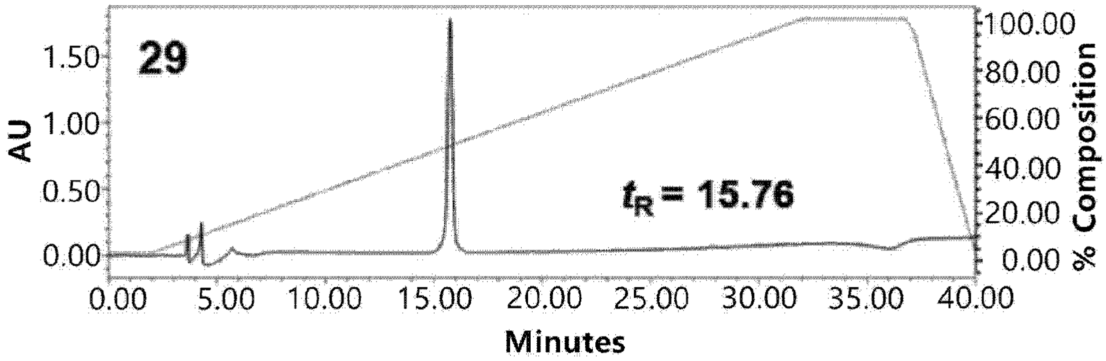


FIG. 38

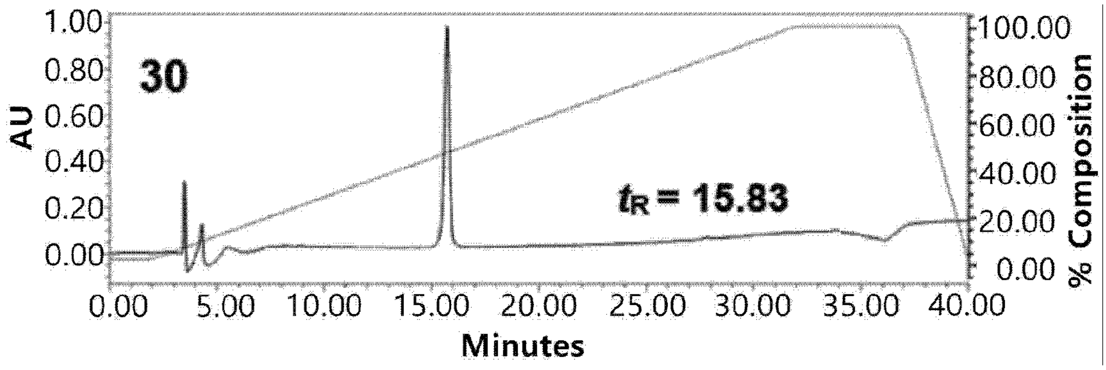


FIG. 39

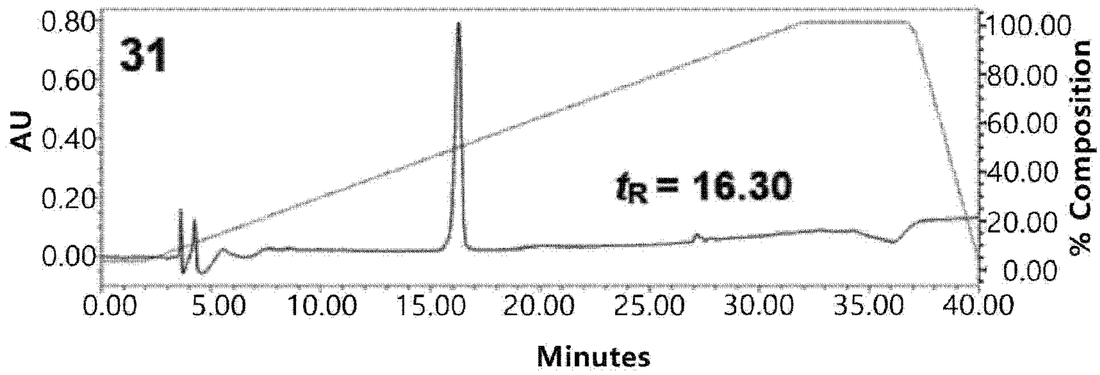


FIG. 40

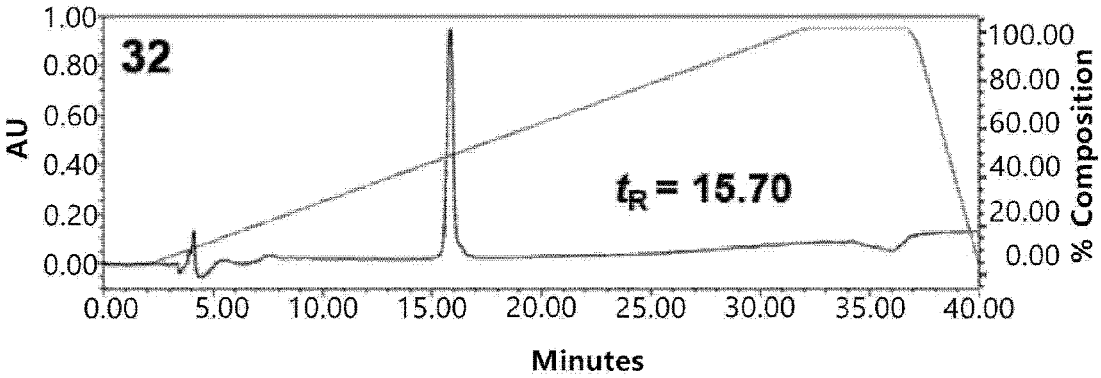
32
$t_R$ = 15.70
AU
1.00
0.80
0.60
0.40
0.20
0.00
100.00
80.00
60.00
40.00
20.00
0.00
% Composition
0.00
5.00
10.00
15.00
20.00
25.00
30.00
35.00
40.00
Minutes

FIG. 41

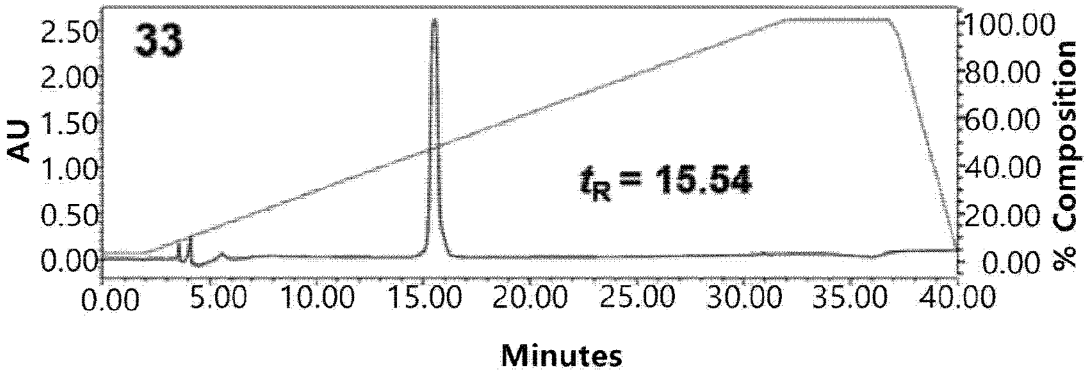


FIG. 42

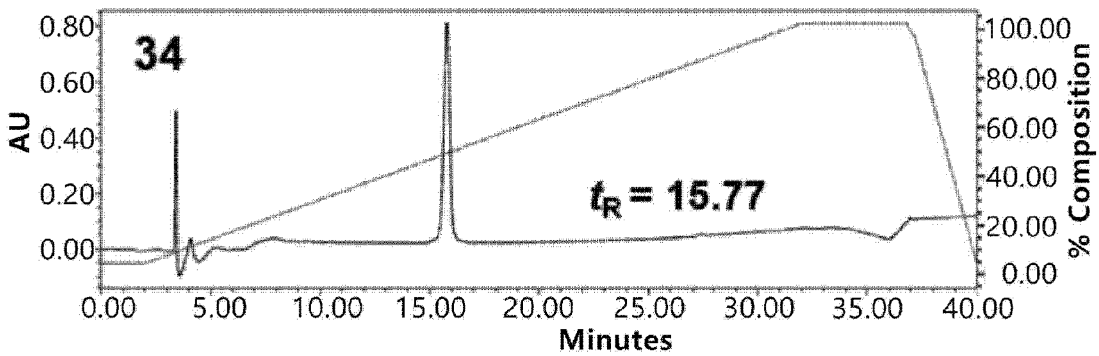


FIG. 43

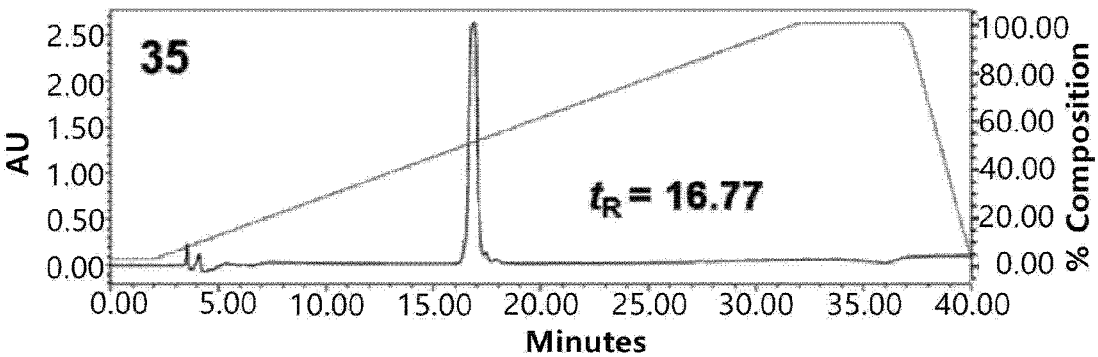


FIG. 44

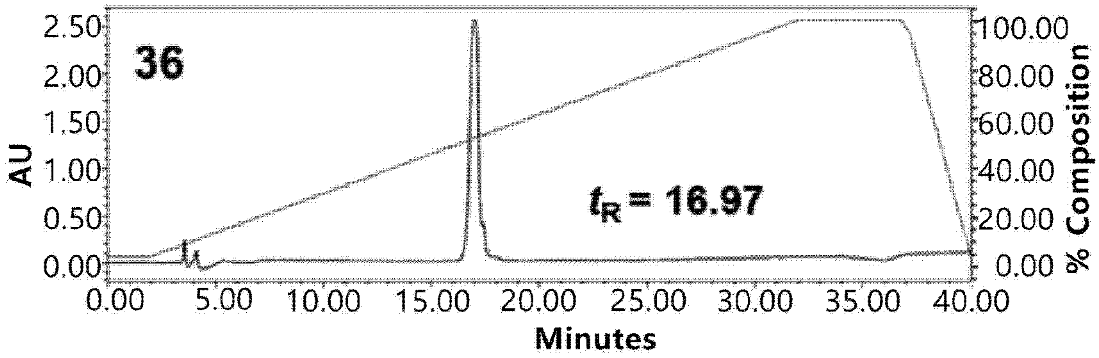


FIG. 45

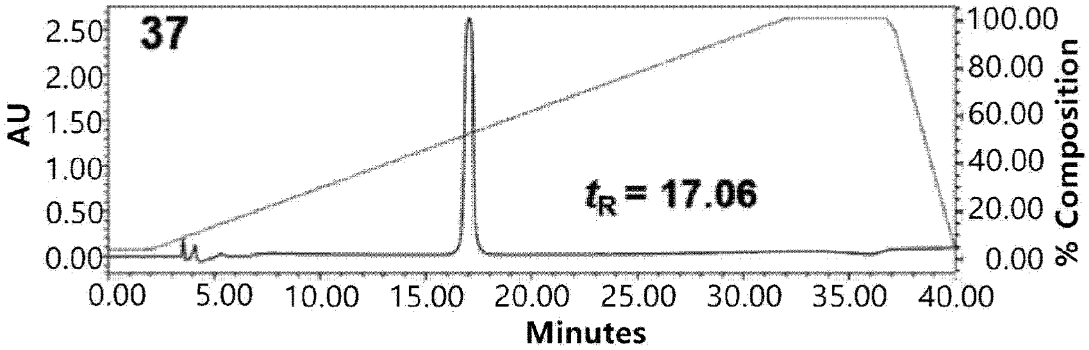


FIG. 46

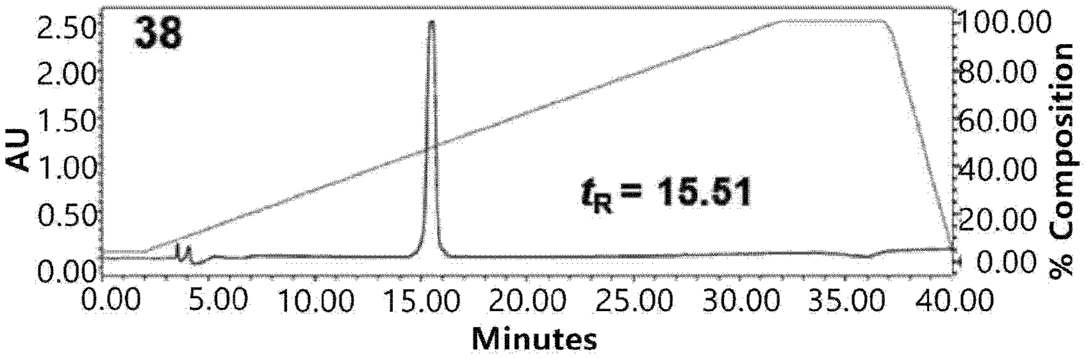


FIG. 47

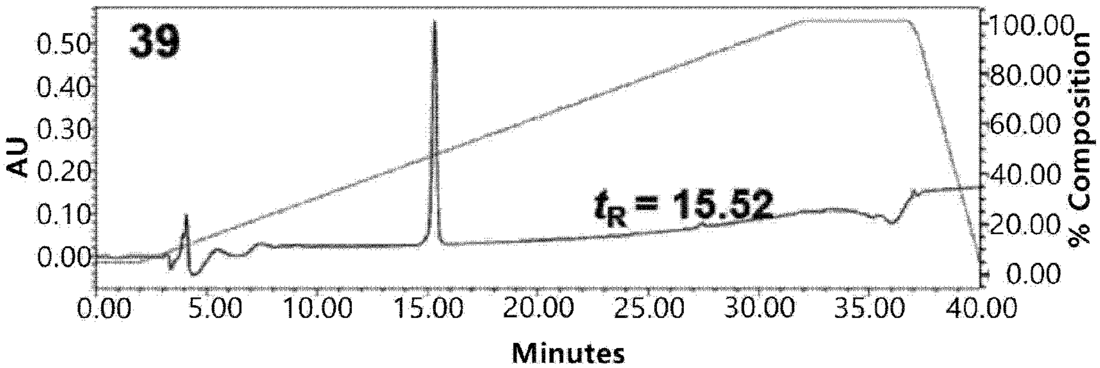

FIG. 48

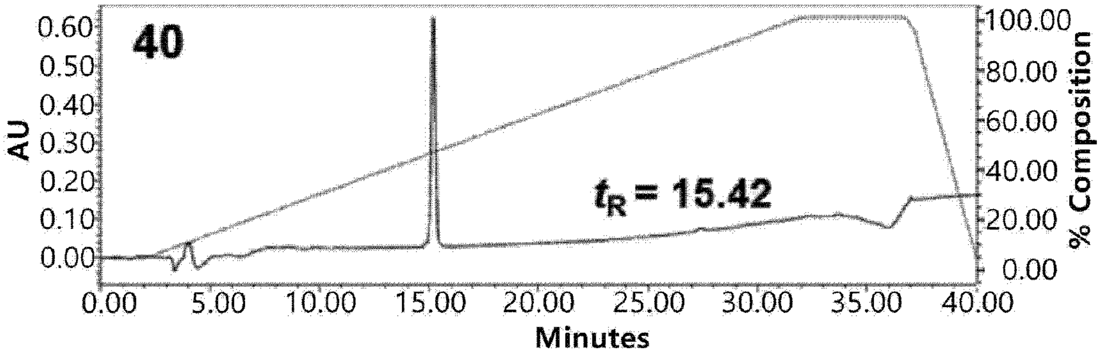


FIG. 49

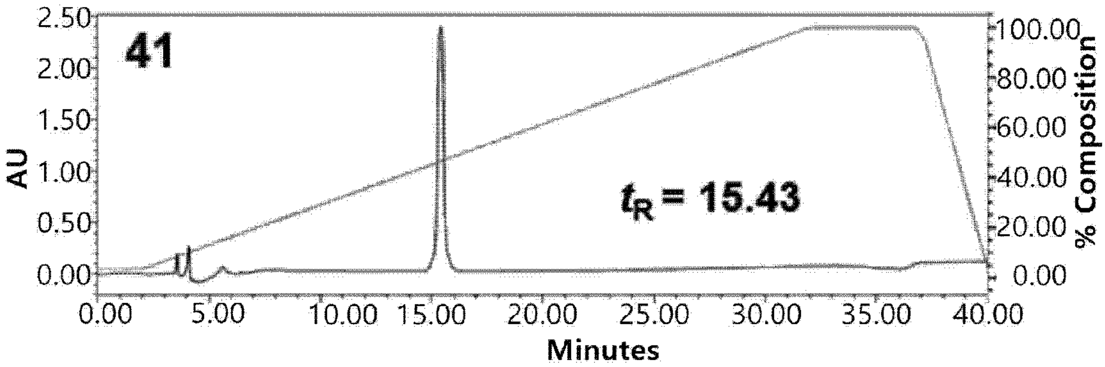


FIG. 50

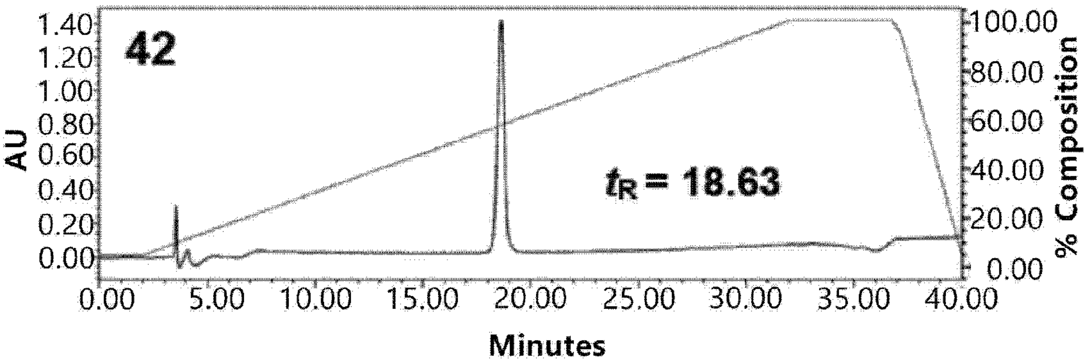


FIG. 51

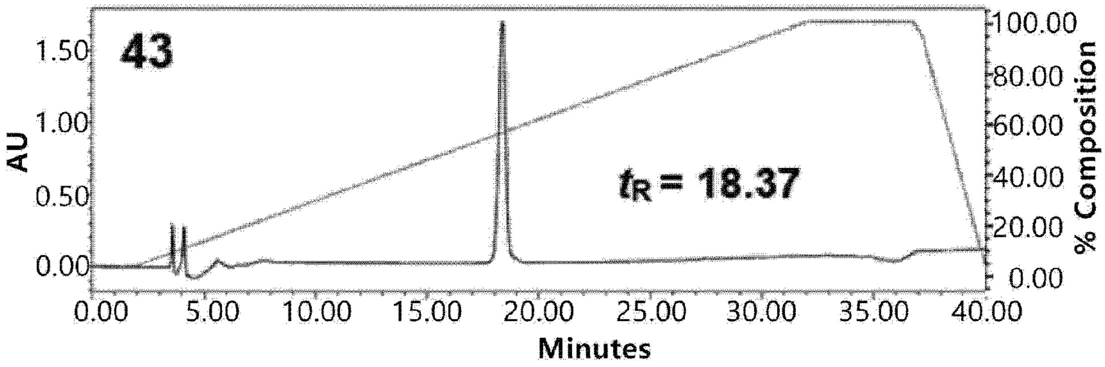


FIG. 52

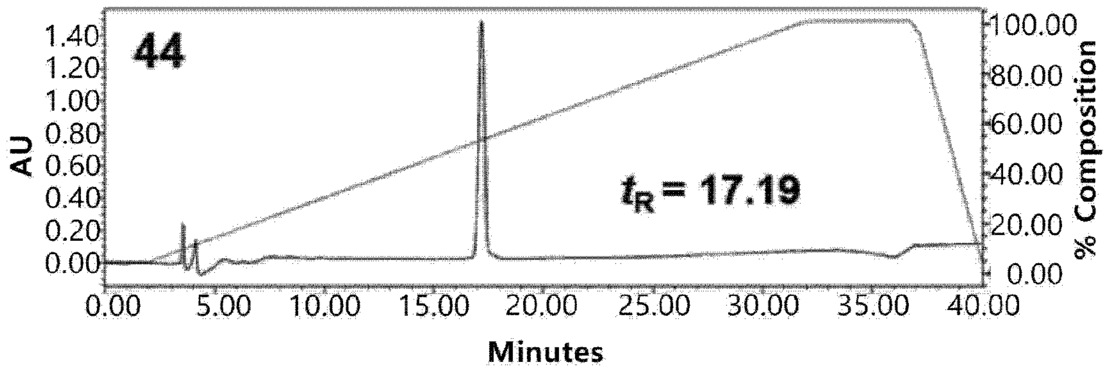


FIG. 53

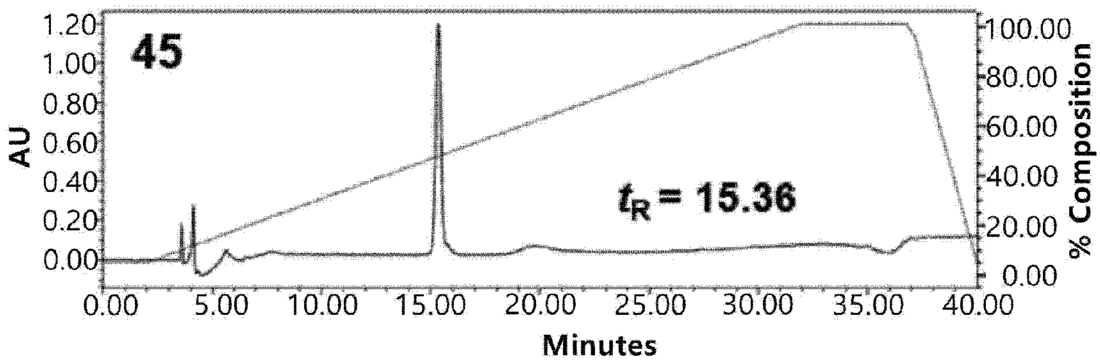


FIG. 54

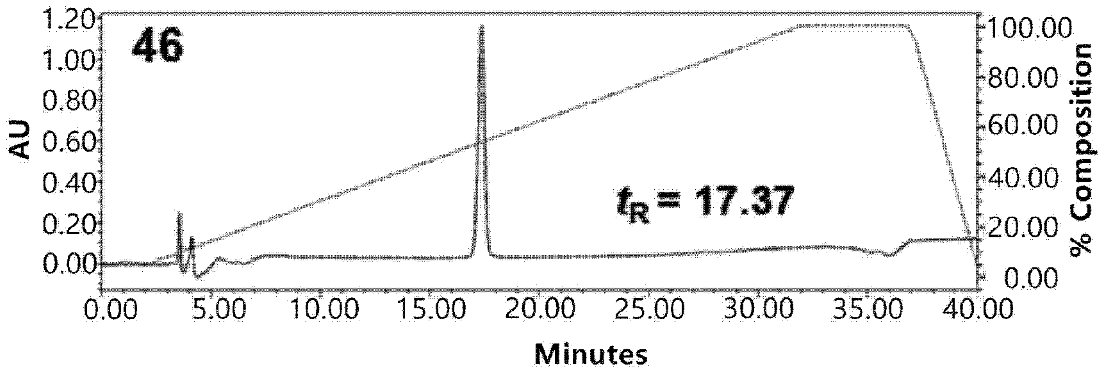

FIG. 55

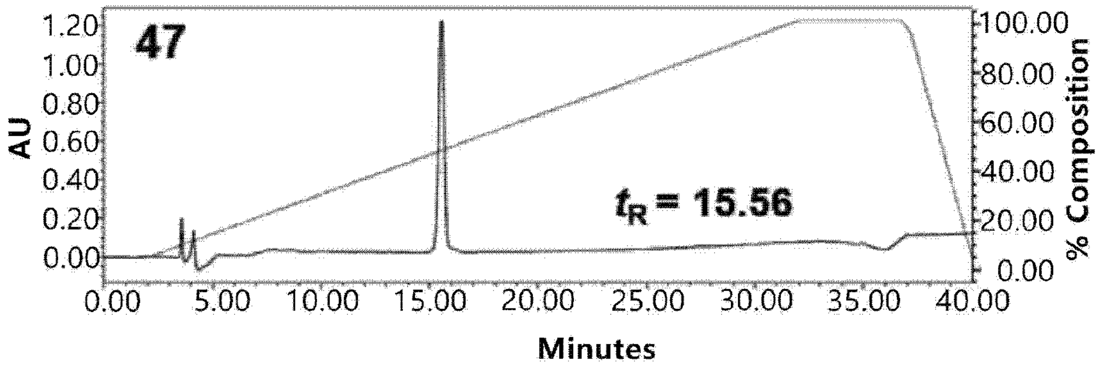


FIG. 56

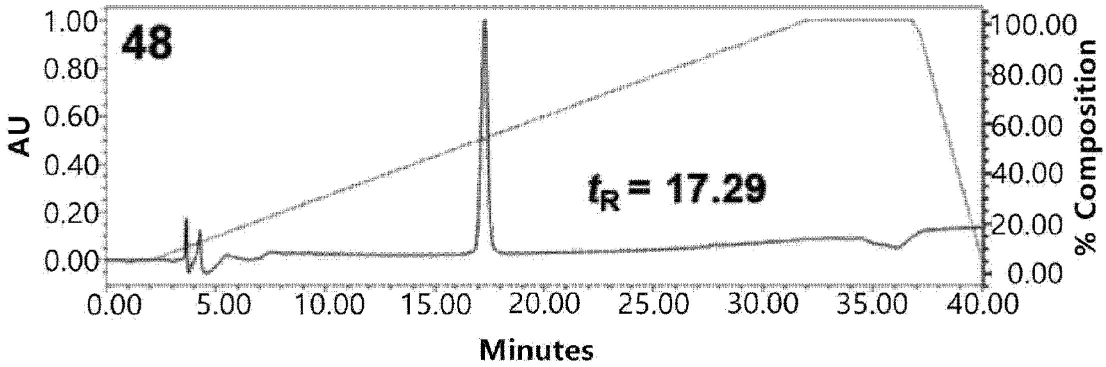


FIG. 57

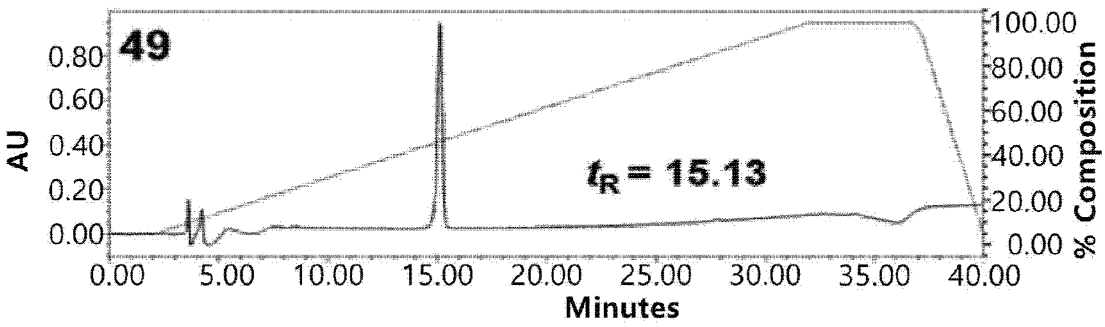


FIG. 58

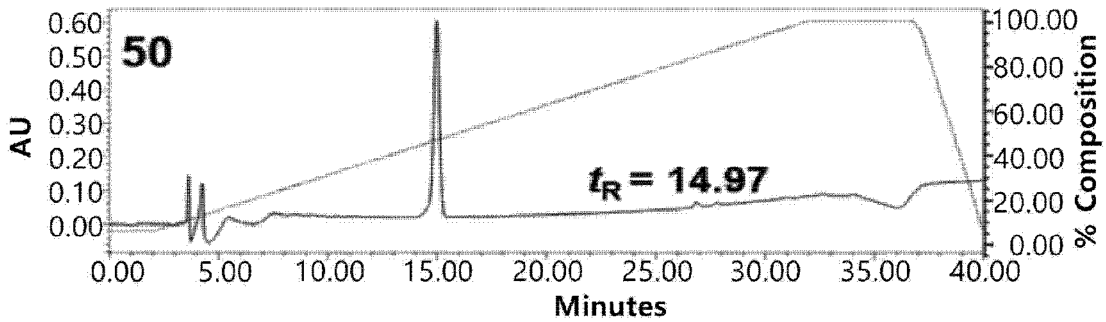


FIG. 59

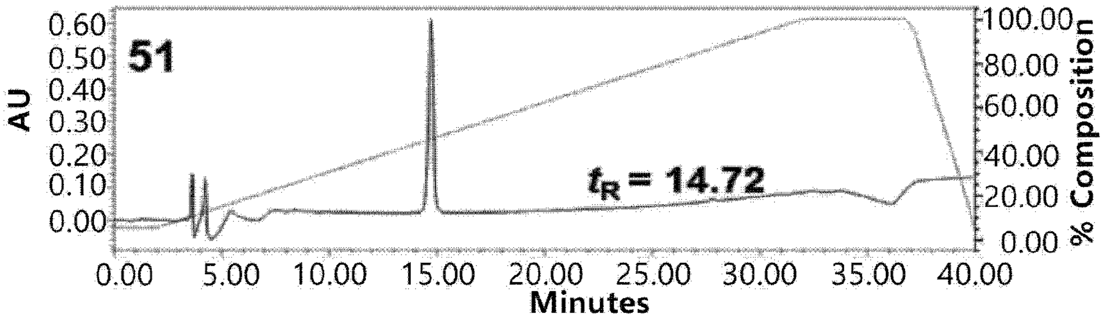
51
AU
0.60
0.50
0.40
0.30
0.20
0.10
0.00
$t_R$ = 14.72
100.00
80.00
60.00
40.00
20.00
0.00
% Composition
0.00 5.00 10.00 15.00 20.00 25.00 30.00 35.00 40.00
Minutes

FIG. 60

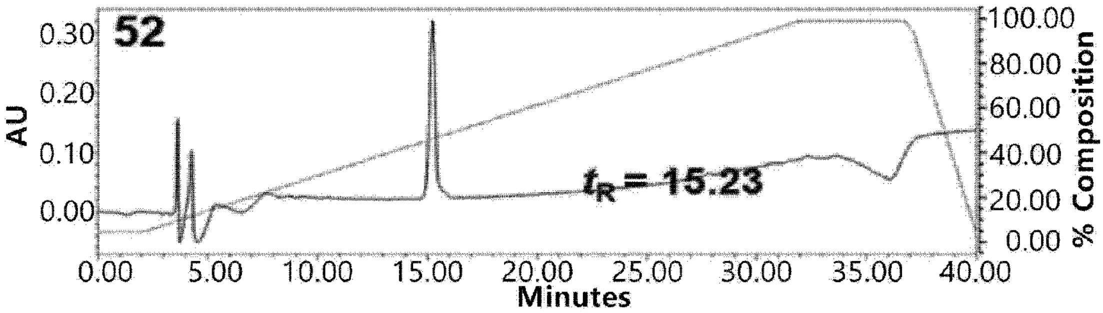
52
AU
0.30
0.20
0.10
0.00
$t_R$ = 15.23
100.00
80.00
60.00
40.00
20.00
0.00
% Composition
0.00 5.00 10.00 15.00 20.00 25.00 30.00 35.00 40.00
Minutes

FIG. 61

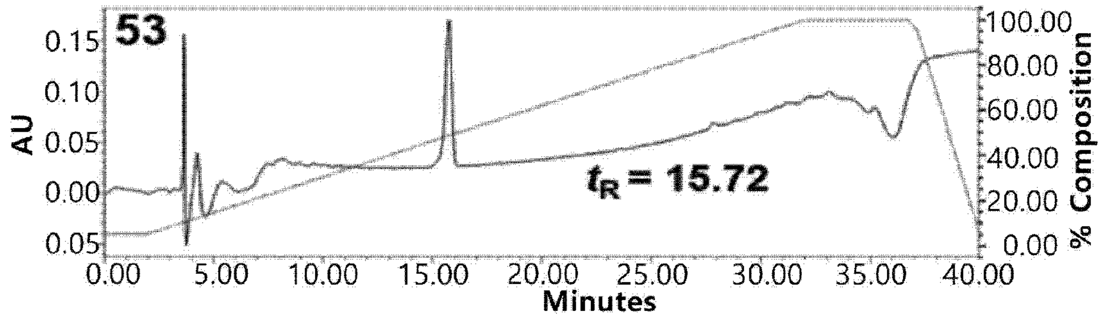


FIG. 62

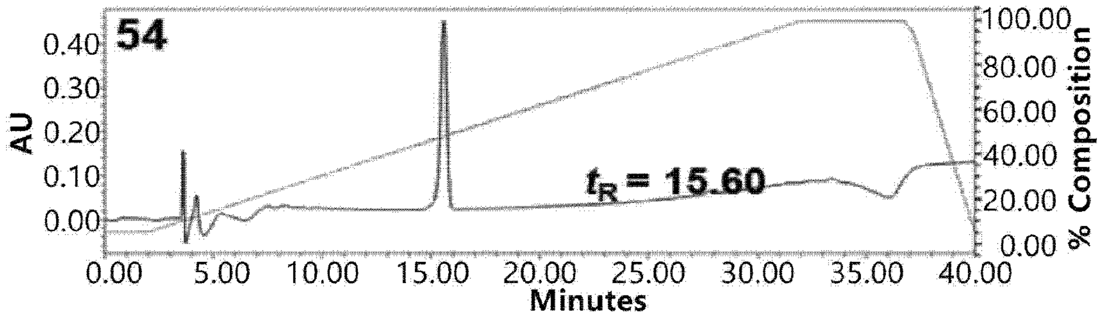


FIG. 63

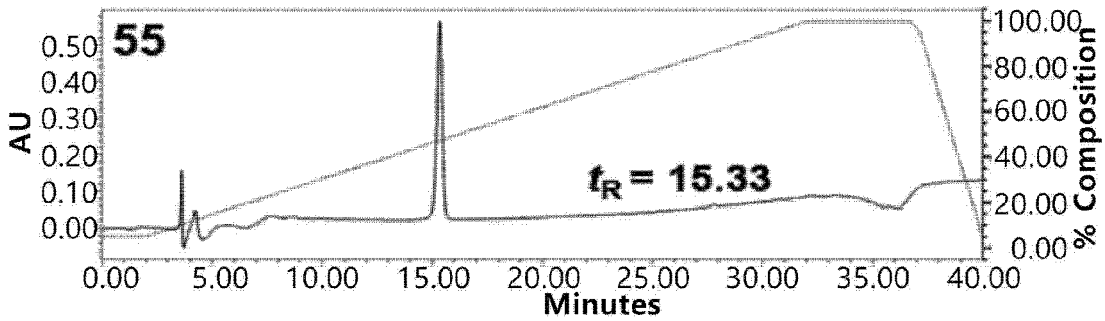


FIG. 64

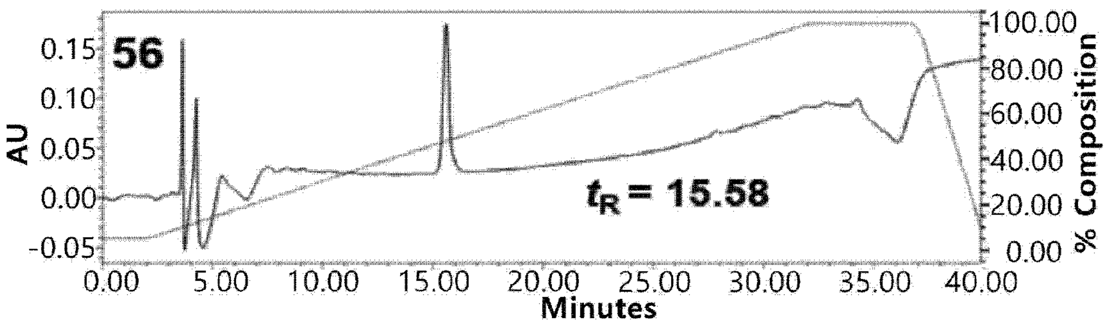

FIG. 65

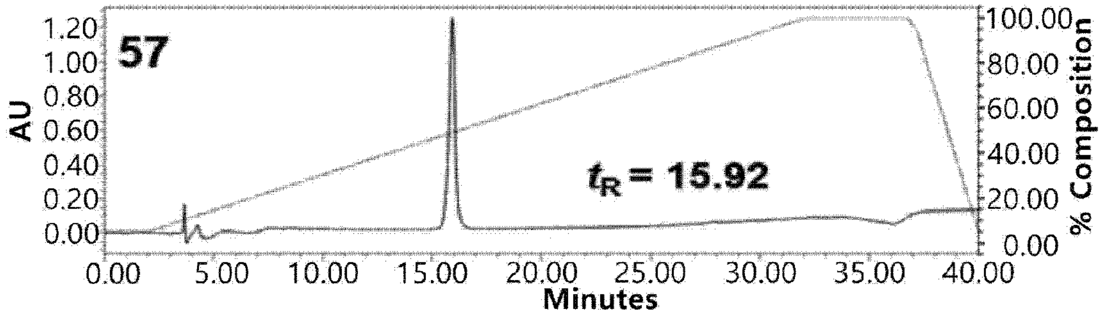


FIG. 66

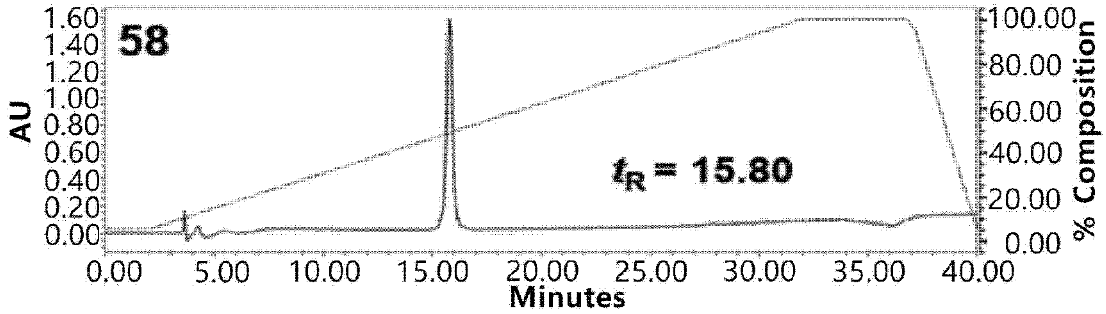


FIG. 67

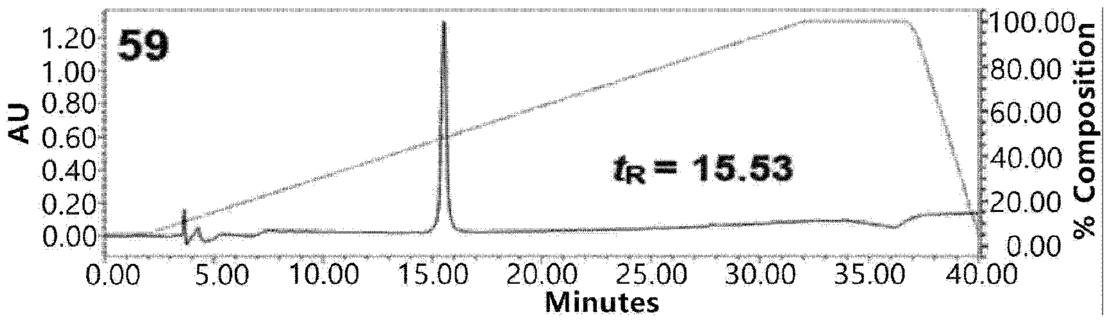


FIG. 68

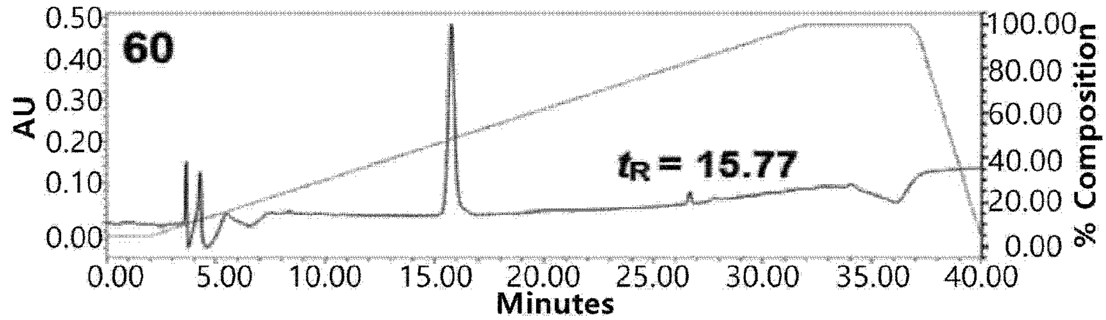

FIG. 69

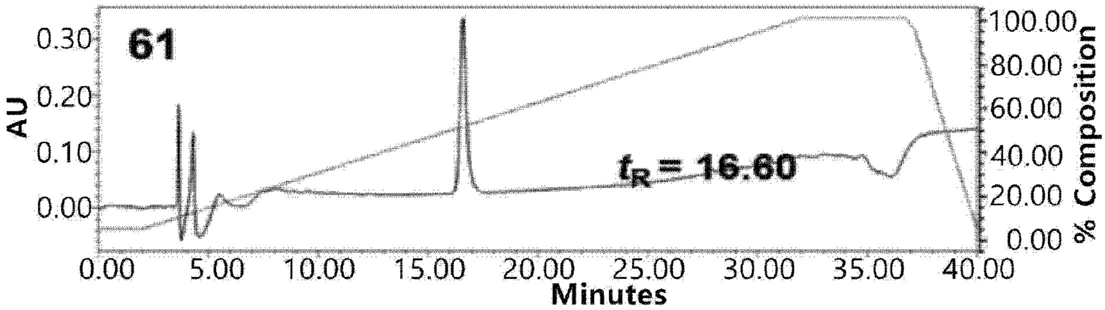


FIG. 70

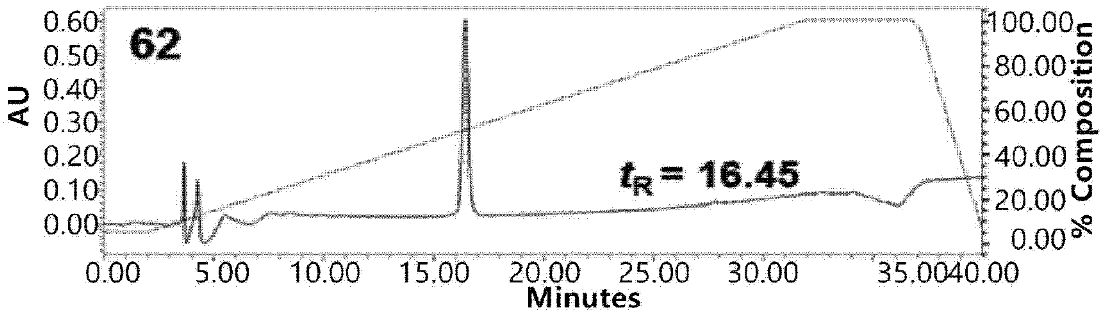


FIG. 71

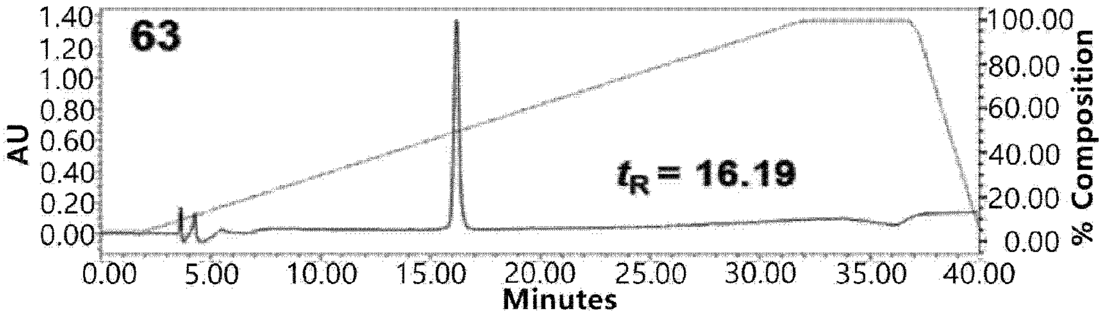


FIG. 72

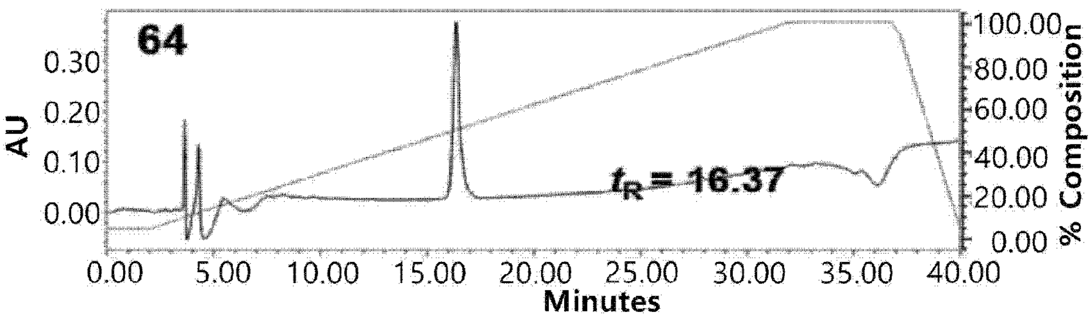

FIG. 73

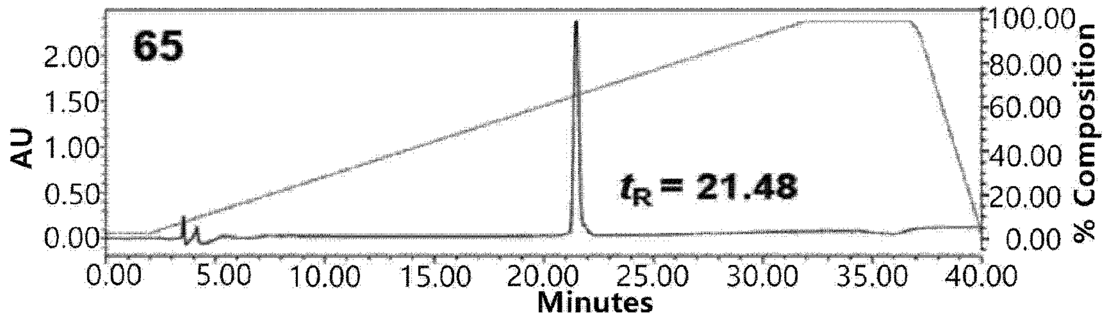
65
AU
2.00
1.50
1.00
0.50
0.00
tR = 21.48
100.00
80.00
60.00
40.00
20.00
0.00
% Composition
0.00
5.00
10.00
15.00
20.00
25.00
30.00
35.00
40.00
Minutes

FIG. 74

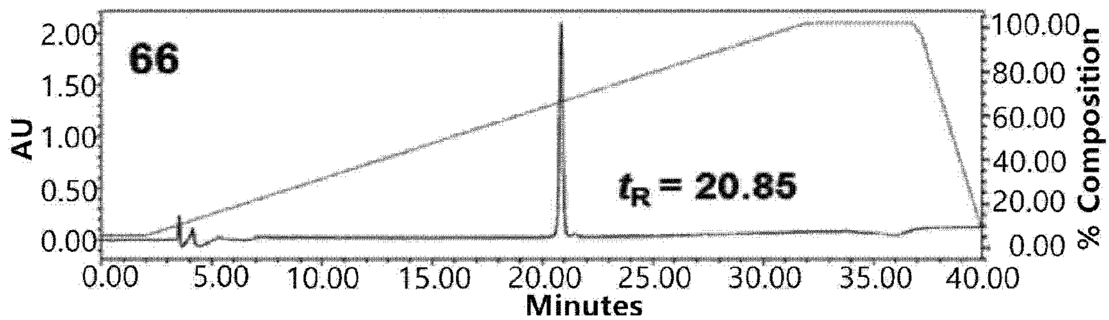
66
AU
2.00
1.50
1.00
0.50
0.00
tR = 20.85
100.00
80.00
60.00
40.00
20.00
0.00
% Composition
0.00
5.00
10.00
15.00
20.00
25.00
30.00
35.00
40.00
Minutes

FIG. 75

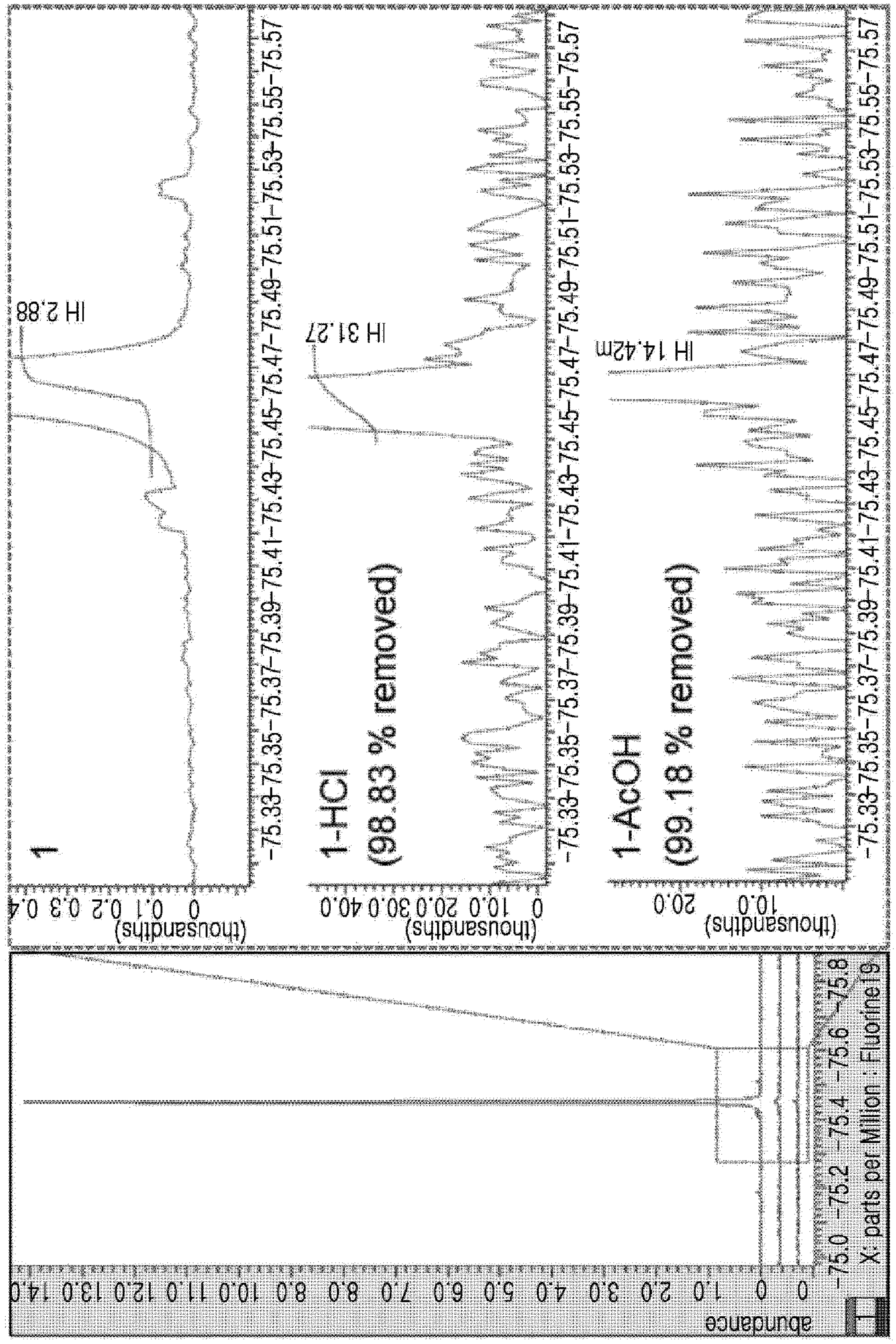
1
1-HCl
(98.83 % removed)
1-AcOH
(99.18 % removed)
(thousandths)
abundance
X: parts per Million : Fluorine19

FIG. 76

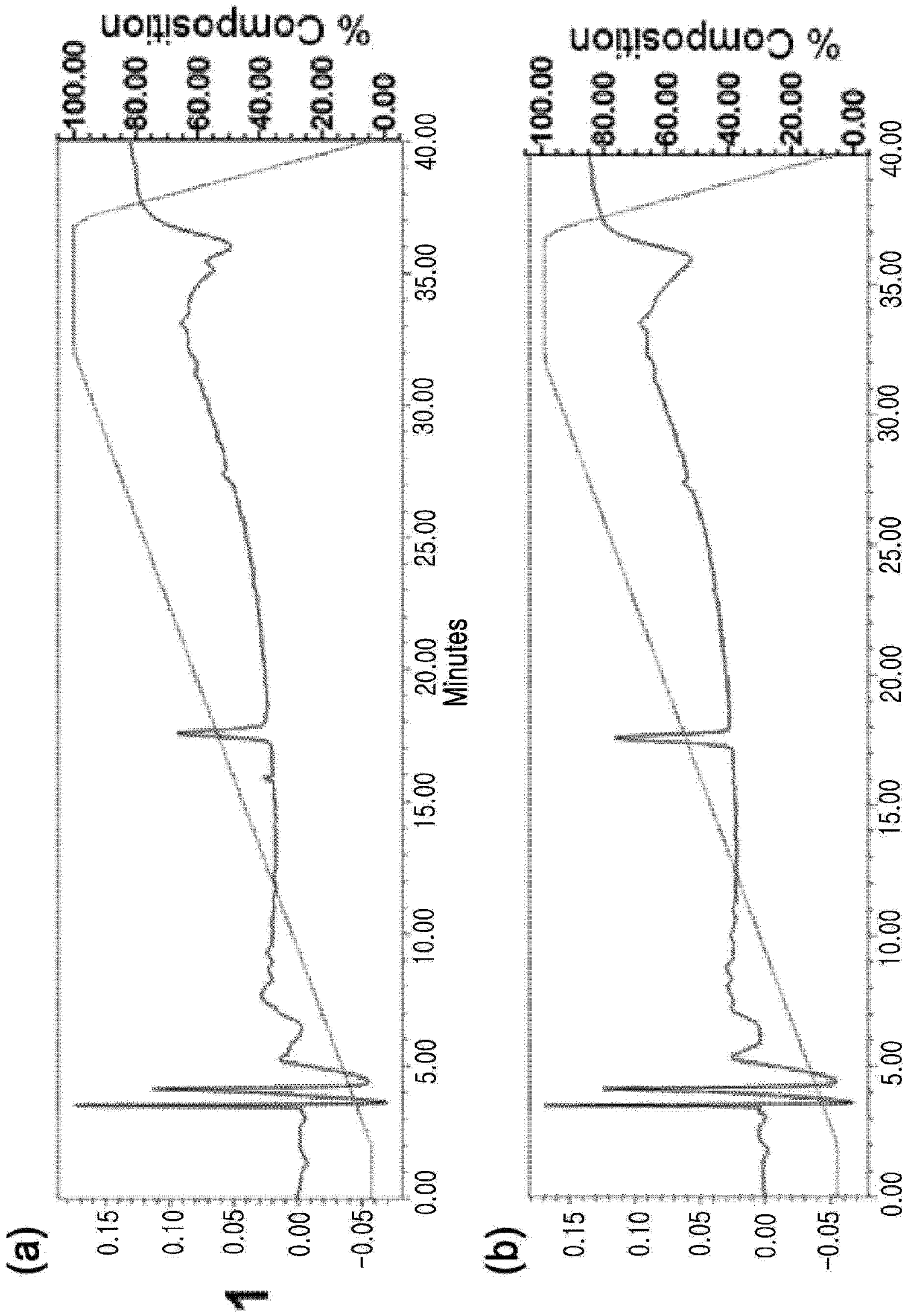
(a)
1
0.15
0.10
0.05
0.00
−0.05
0.00
5.00
10.00
15.00
20.00
25.00
30.00
35.00
40.00
Minutes
100.00
80.00
60.00
40.00
20.00
0.00
% Composition
(b)
0.15
0.10
0.05
0.00
−0.05
0.00
5.00
10.00
15.00
20.00
25.00
30.00
35.00
40.00
Minutes
100.00
80.00
60.00
40.00
20.00
0.00
% Composition

FIG. 77

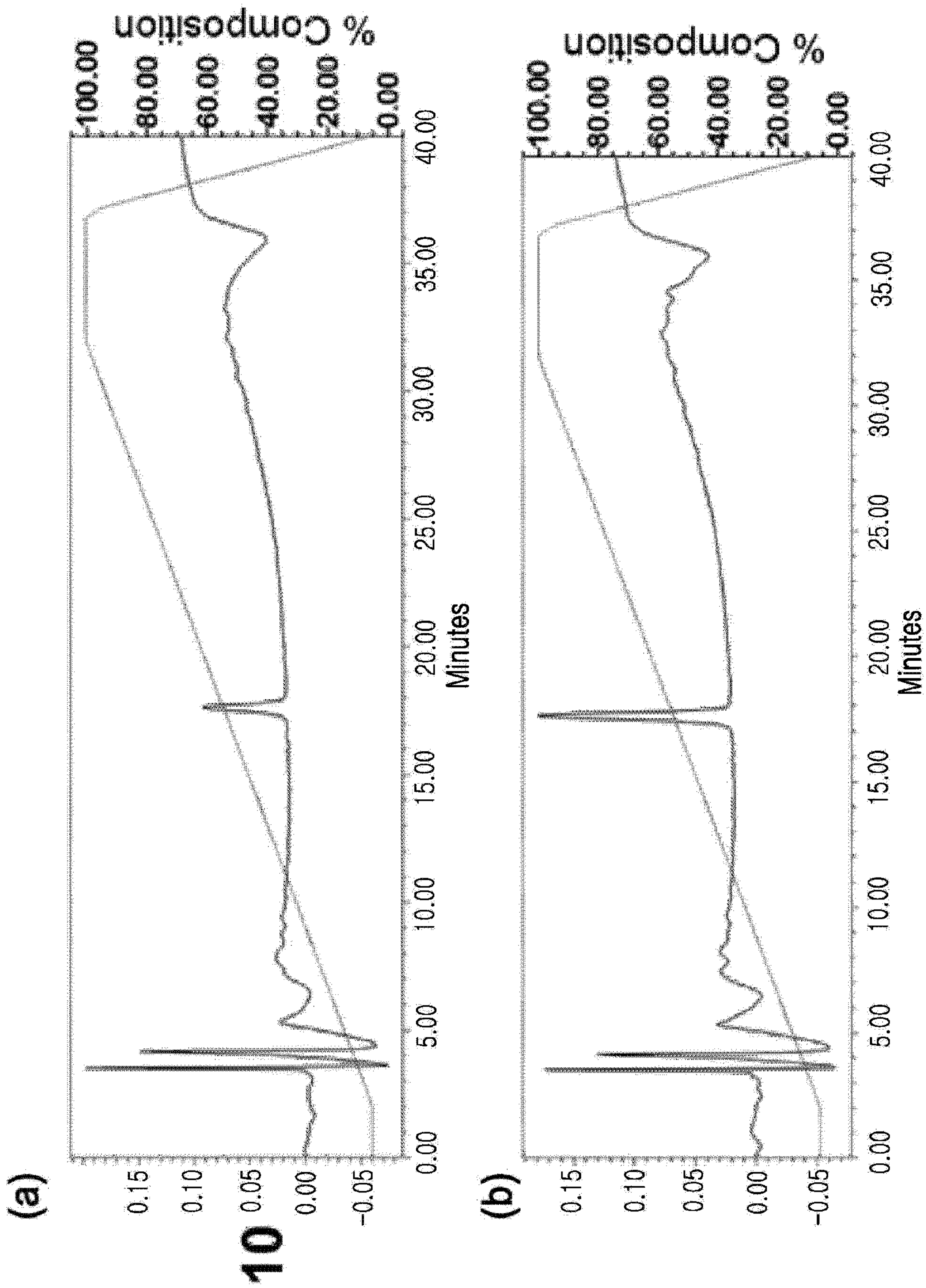

FIG. 78

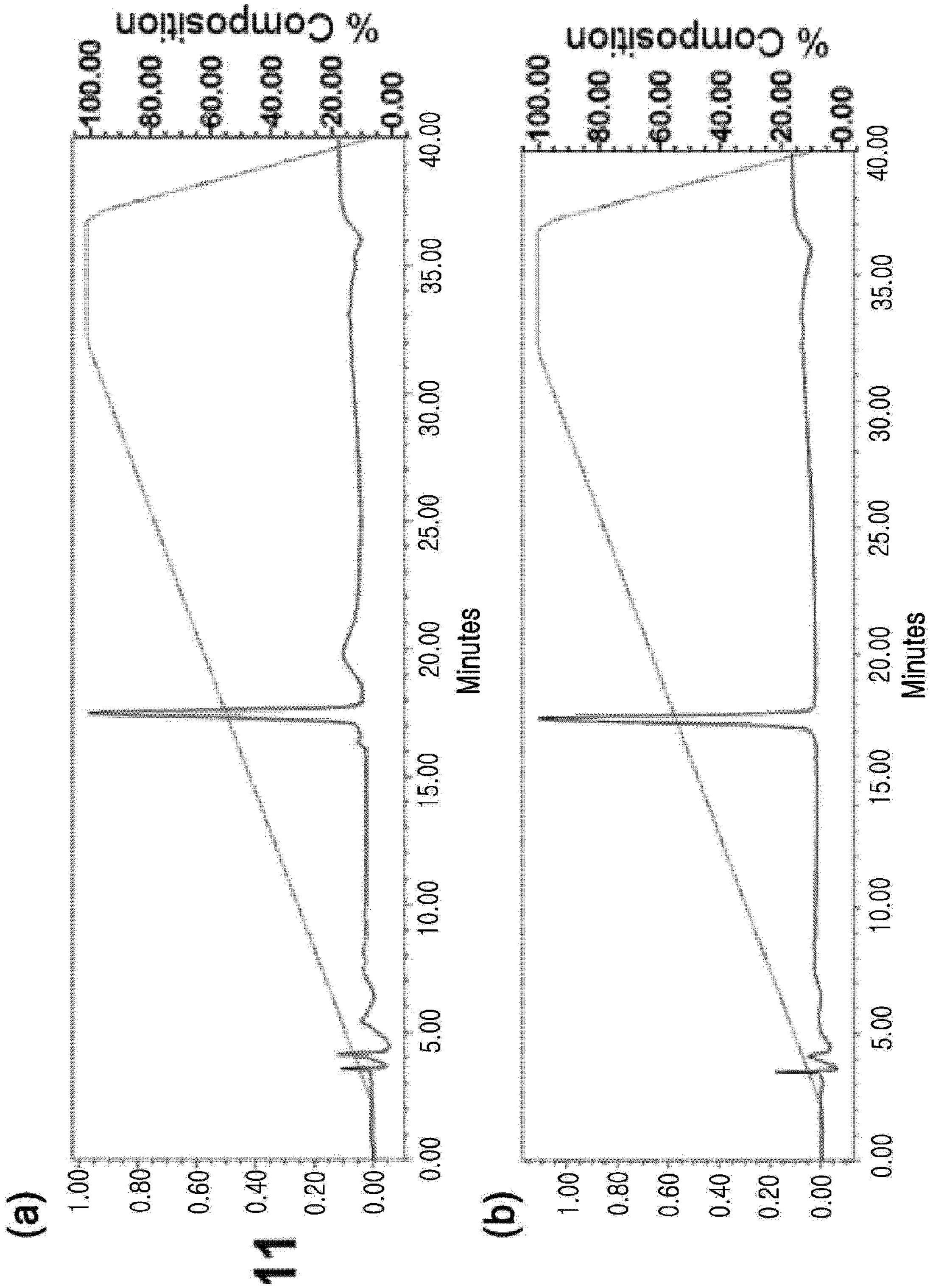

FIG. 79

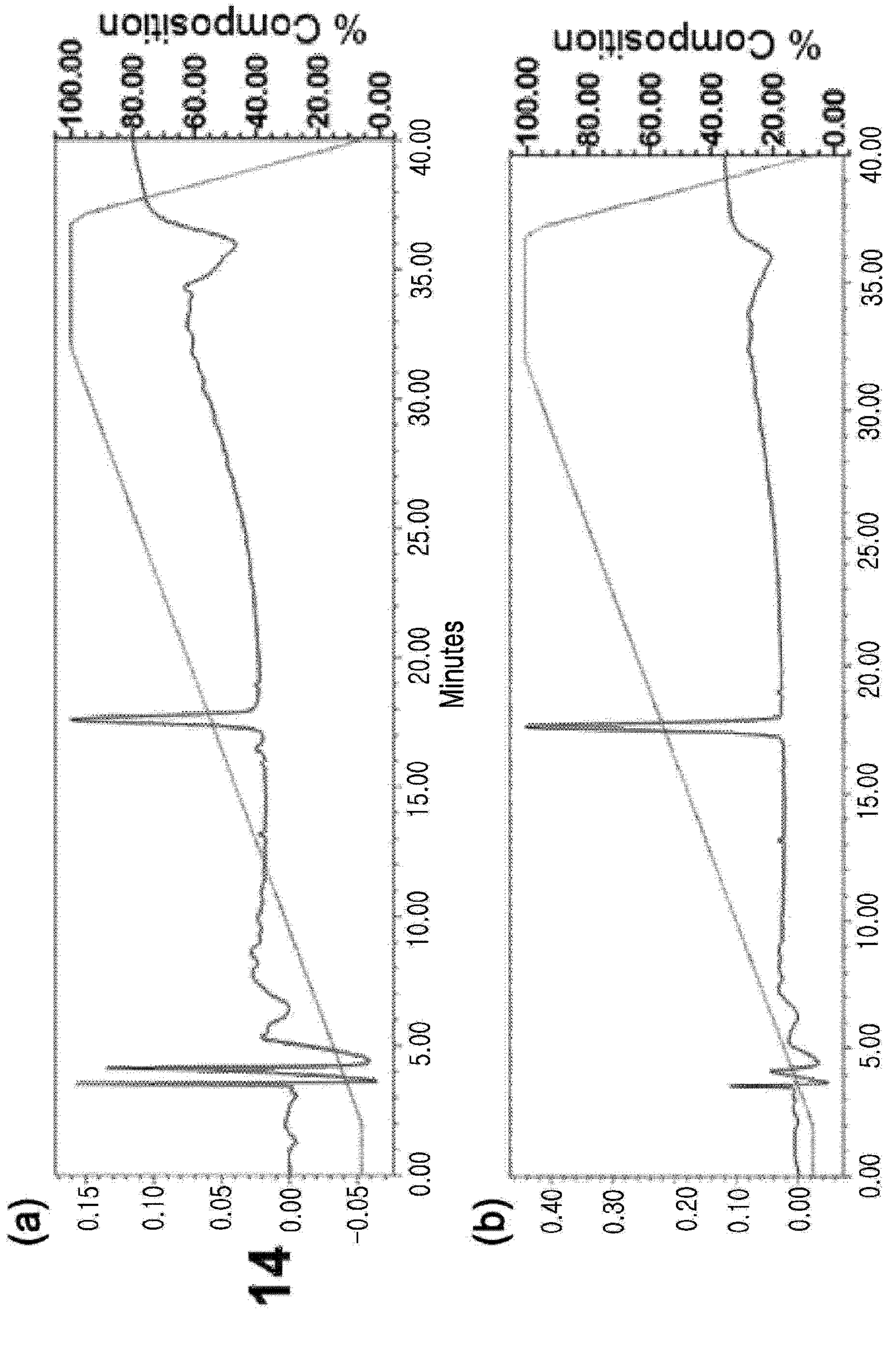

FIG. 80

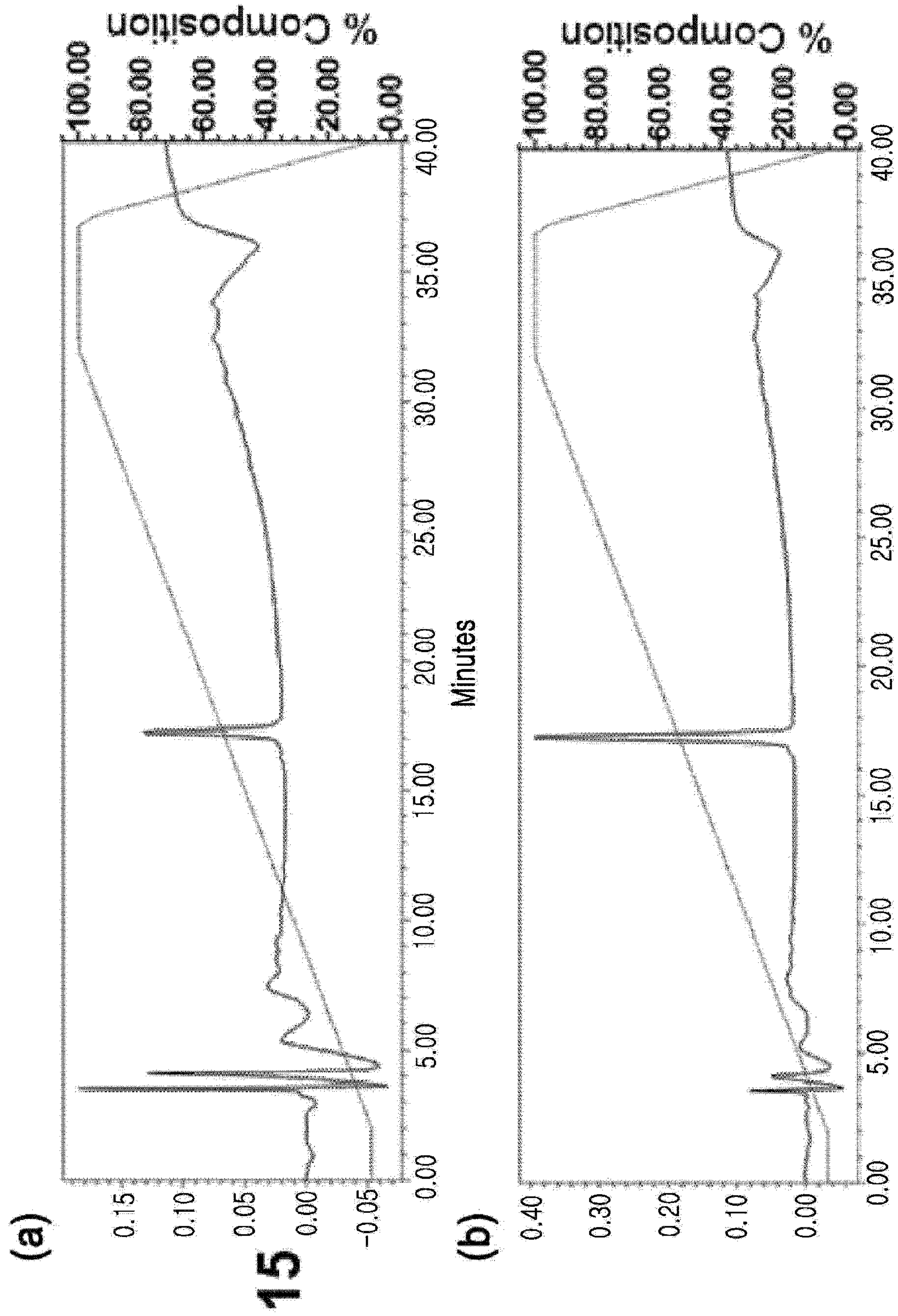
(a)
(b)
Minutes
% Composition
0.00
5.00
10.00
15.00
20.00
25.00
30.00
35.00
40.00
100.00
80.00
60.00
40.00
20.00
0.15
0.10
0.05
-0.05
0.40
0.30
0.20

FIG. 81

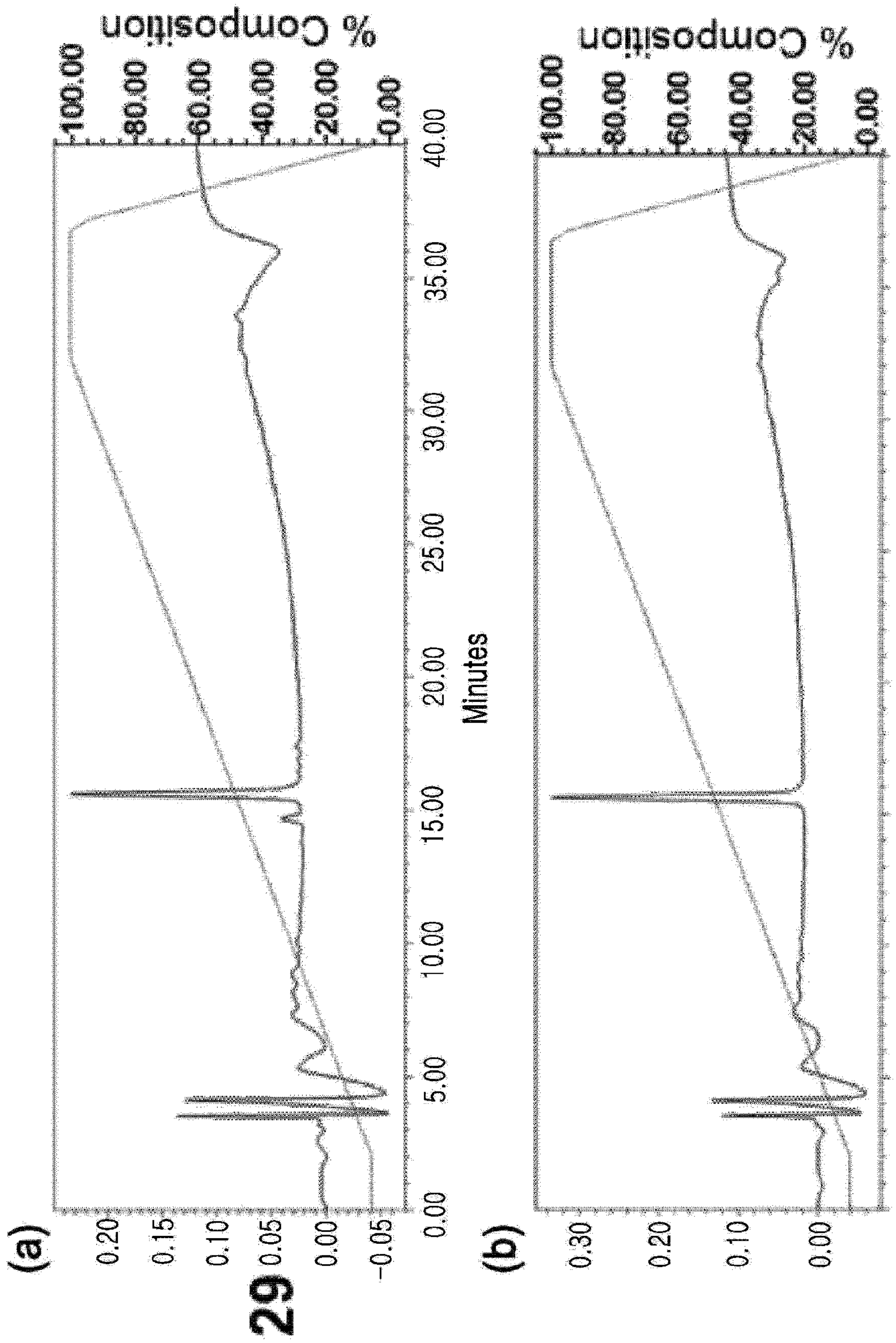
(a)
29
0.20
0.15
0.10
0.05
0.00
-0.05
0.00
5.00
10.00
15.00
20.00
25.00
30.00
35.00
40.00
Minutes
100.00
80.00
60.00
40.00
20.00
0.00
% Composition
(b)
0.30
0.20
0.10
0.00
0.00
5.00
10.00
15.00
20.00
25.00
30.00
35.00
40.00
Minutes
100.00
80.00
60.00
40.00
20.00
0.00
% Composition

FIG. 82

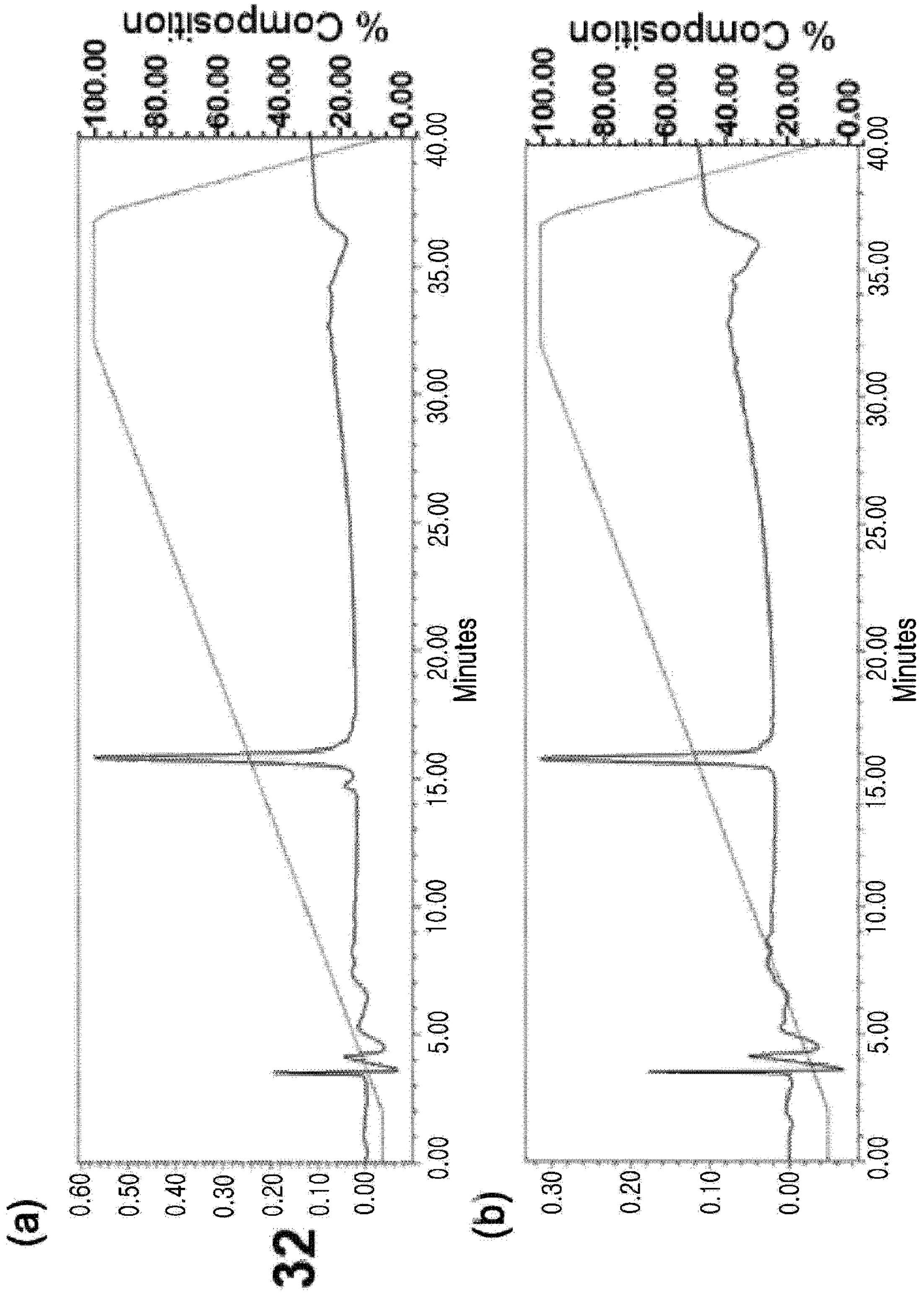
(a)
32
(b)
% Composition
Minutes
0.00
5.00
10.00
15.00
20.00
25.00
30.00
35.00
40.00
0.60
0.50
0.40
0.30
0.20
0.10
0.00
100.00
80.00
60.00
40.00
20.00

FIG. 83

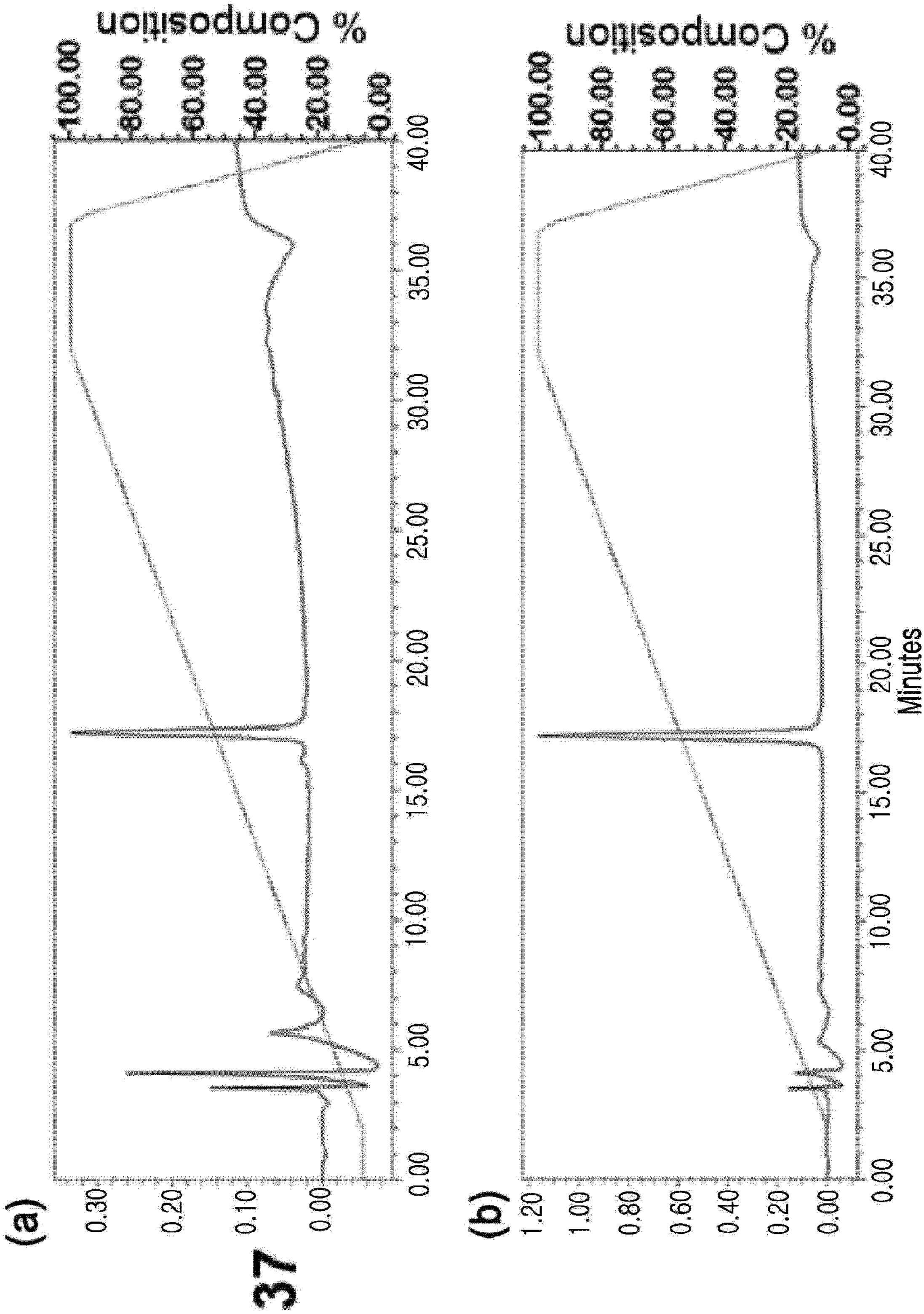

FIG. 84

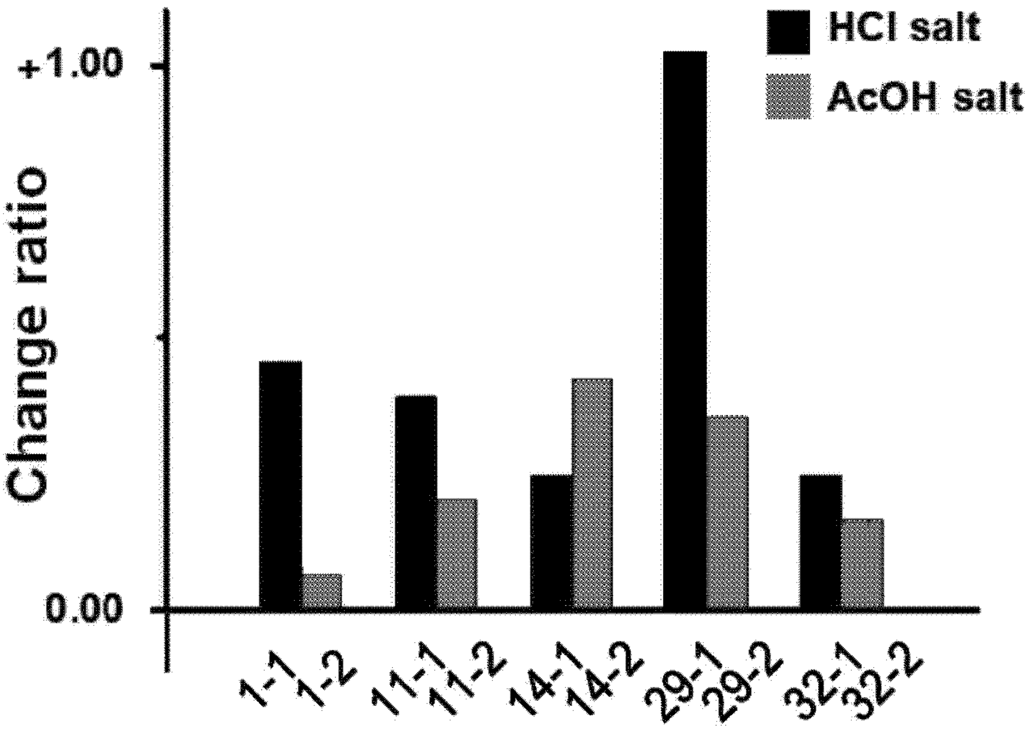
HCl salt
AcOH salt
+1.00
0.00
Change ratio
1-1
1-2
11-1
11-2
14-1
14-2
29-1
29-2
32-1
32-2

FIG. 85

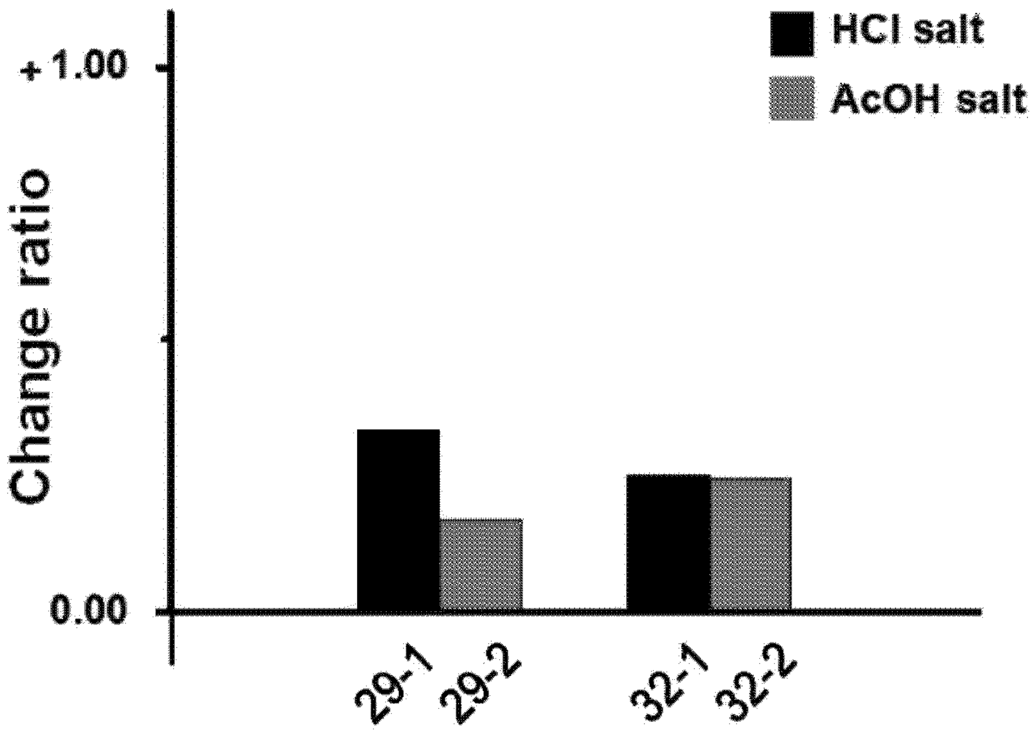
HCl salt
AcOH salt
+1.00
0.00
Change ratio
29-1
29-2
32-1
32-2

FIG. 86

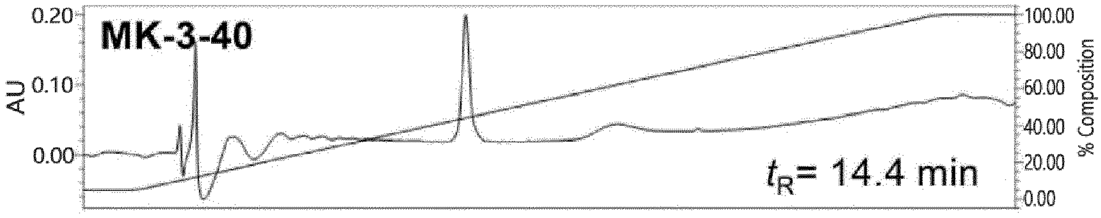


FIG. 87

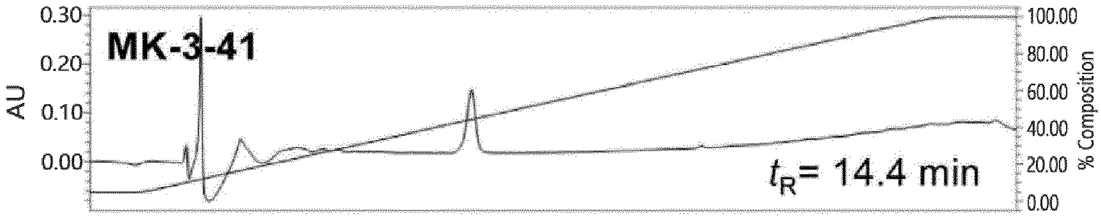


FIG. 88

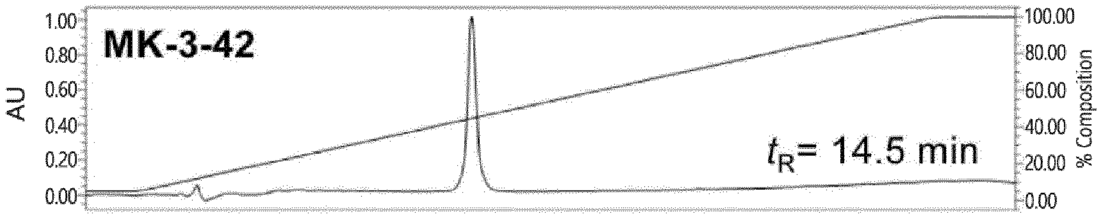


FIG. 89

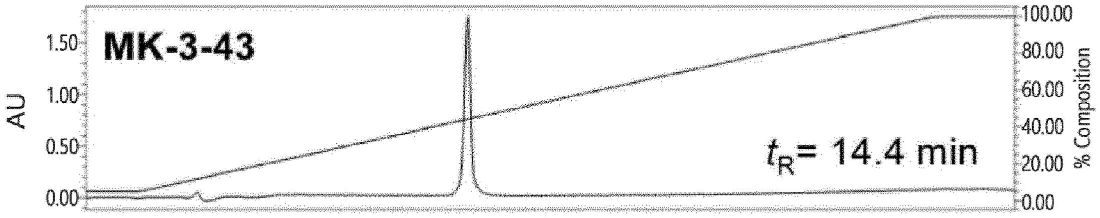


FIG. 90

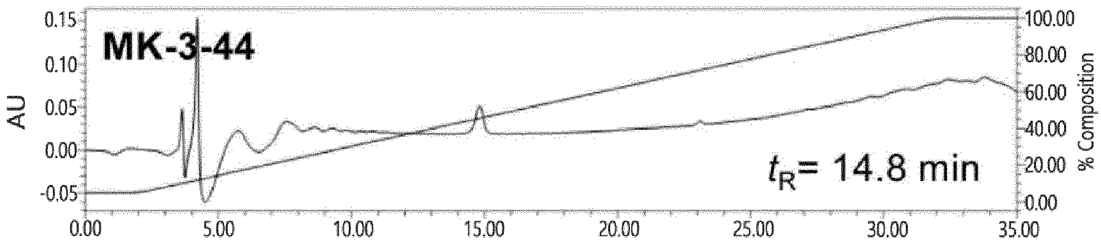

FIG. 91

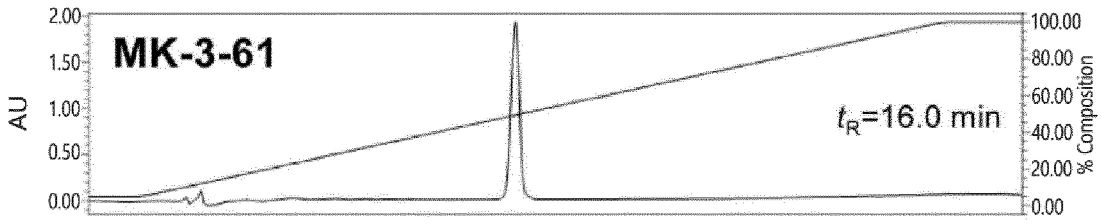


FIG. 92

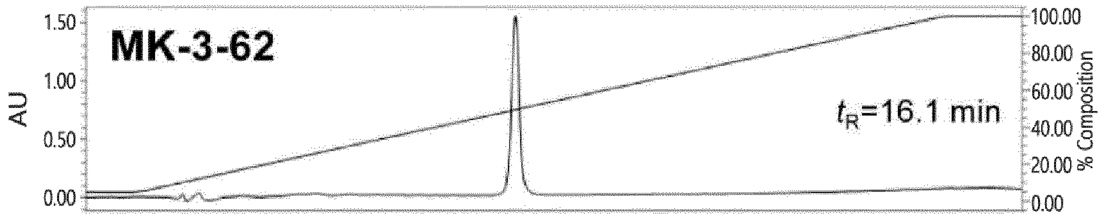


FIG. 93

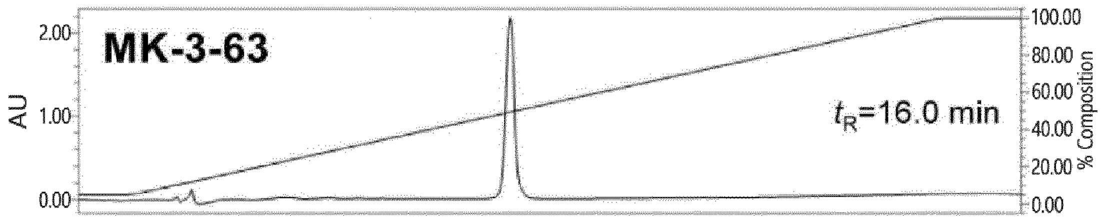


FIG. 94

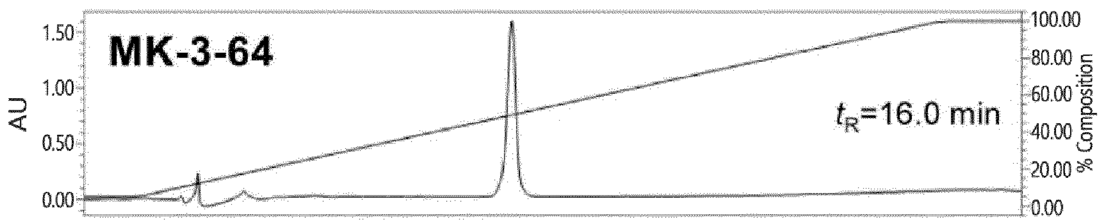


FIG. 95

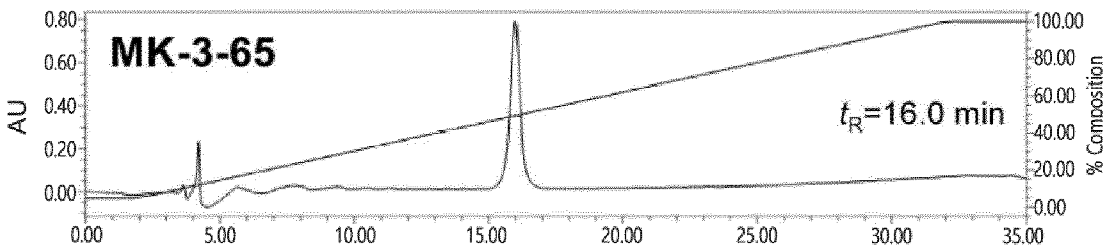

FIG. 96

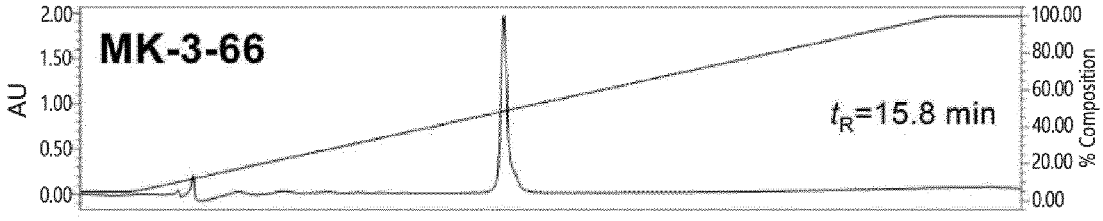


FIG. 97

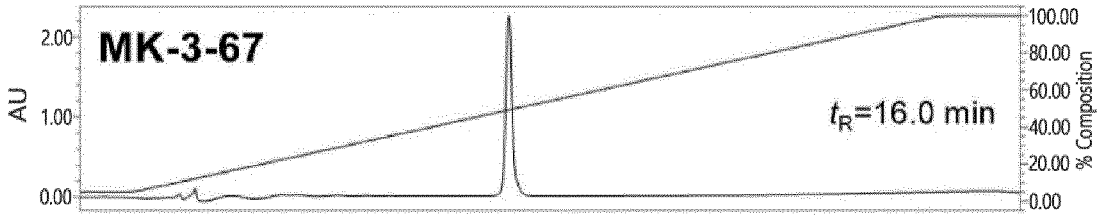


FIG. 98

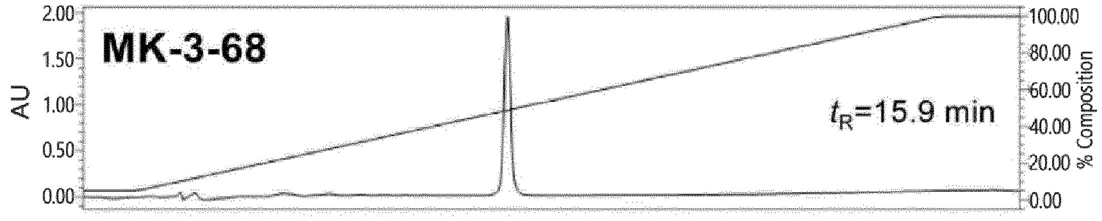


FIG. 99

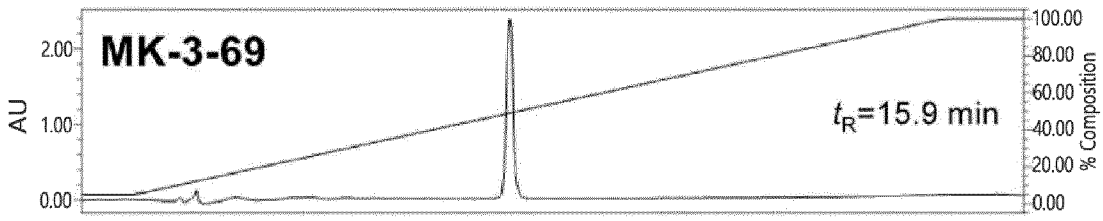


FIG. 100

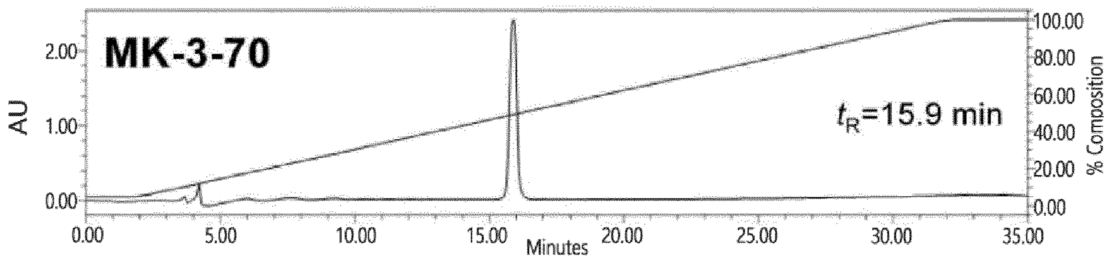

**INTERNATIONAL SEARCH REPORT**

International application No.
**PCT/KR2022/005387**

**A. CLASSIFICATION OF SUBJECT MATTER**

**C07K 7/08**(2006.01)i; **A01N 37/46**(2006.01)i

According to International Patent Classification (IPC) or to both national classification and IPC

**B. FIELDS SEARCHED**

Minimum documentation searched (classification system followed by classification symbols)

C07K 7/08(2006.01); A61K 38/00(2006.01); A61K 38/12(2006.01); A61P 31/04(2006.01); C07K 14/00(2006.01); C07K 7/06(2006.01); C07K 7/64(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 펩토이드(peptoid), 항균(antimicrobial), 스타필로코커스 속(Staphylococcus sp.), 살모넬라 속(Salmonella sp.)

**C. DOCUMENTS CONSIDERED TO BE RELEVANT**

| Category* | Citation of document, with indication, where appropriate, of the relevant passages | Relevant to claim No. |
|---|---|---|
| X | KR 10-2019-0110061 A (GWANGJU INSTITUTE OF SCIENCE AND TECHNOLOGY) 27 September 2019 (2019-09-27)<br>See abstract; claims 1-7; and paragraphs [0001] and [0061]-[0070]. | 1,3-9 |
| Y | | 10-11 |
| A | | 2 |
| Y | Internet Webpage. Lifeprotein. TFA removal service: switch to acetate or HCl salt form of peptide. 12 November 2020. Retrieved from https://web.archive.org/web/20201112012819/https://www.lifetein.com/TFA_Removal_Peptide_Synthesis_Services.html.<br>See pages 1-4. | 10-11 |
| X | LEE, J. et al. Effect of side chain hydrophobicity and cationic charge on antimicrobial activity and cytotoxicity of helical peptoids. Bioorganic & Medicinal Chemistry Letters. 2018, vol. 28, pp. 170-173.<br>See abstract; and table 1. | 1,3 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 July 2022** | **28 July 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2022/005387**

**C. DOCUMENTS CONSIDERED TO BE RELEVANT**

| Category* | Citation of document, with indication, where appropriate, of the relevant passages | Relevant to claim No. |
|---|---|---|
| A | JP 2011-511077 A (NORTHWESTERN UNIVERSITY) 07 April 2011 (2011-04-07)<br>See entire document. | 1-11 |
| A | US 2010-0222255 A1 (KIRSHENBAUM, Kent et al.) 02 September 2010 (2010-09-02)<br>See entire document. | 1-11 |

Form PCT/ISA/210 (second sheet) (July 2019)

**INTERNATIONAL SEARCH REPORT**
**Information on patent family members**

International application No.
**PCT/KR2022/005387**

| Patent document cited in search report | Publication date (day/month/year) | Patent family member(s) | Publication date (day/month/year) |
|---|---|---|---|
| KR 10-2019-0110061 A | 27 September 2019 | KR 10-2210934 B1 | 03 February 2021 |
| | | US 10815275 B2 | 27 October 2020 |
| | | US 2019-0284238 A1 | 19 September 2019 |
| JP 2011-511077 A | 07 April 2011 | CA 2714853 A1 | 27 August 2009 |
| | | EP 2252576 A2 | 24 November 2010 |
| | | US 2010-0036088 A1 | 11 February 2010 |
| | | US 2012-0295838 A1 | 22 November 2012 |
| | | US 2014-0031523 A1 | 30 January 2014 |
| | | US 8445632 B2 | 21 May 2013 |
| | | WO 2009-105167 A2 | 27 August 2009 |
| | | WO 2009-105167 A3 | 30 December 2009 |
| US 2010-0222255 A1 | 02 September 2010 | EP 2401264 A2 | 04 January 2012 |
| | | US 2015-0011465 A1 | 08 January 2015 |
| | | US 2016-0219880 A1 | 04 August 2016 |
| | | US 8828413 B2 | 09 September 2014 |
| | | US 9315548 B2 | 19 April 2016 |
| | | US 9872495 B2 | 23 January 2018 |
| | | WO 2010-098843 A2 | 02 September 2010 |
| | | WO 2010-098843 A3 | 13 January 2011 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Non-patent literature cited in the description

- **ALEXANDER FLEMING.** *Nobel Prize in Physiology or Medicine,* 1945